# EUROPEAN PATENT APPLICATION

(11) **EP 4 079 726 A1**
(43) Date of publication of application: **26.10.2022**
(21) Application number: 20901025.5
(22) Date of filing: 16.12.2020
(51) Int. Cl.: C07D 239/48, A61K 31/505, A61K 31/506, C07F 9/6558, A61P 1/16, A61P 31/06, A61P 31/12, A61P 35/00, A61P 25/16, C07D 401/04

(54) **NOVEL PYRIMIDIN DERIVATIVE AND USE THEREOF**

(30) Priority: 16.12.2019 KR 20190168363
(71) Applicant: Korea Research Institute of Chemical Technology, Daejeon 34114 (KR)
(72) Inventor: KIM, Pil Ho, Daejeon 34114 (KR); KIM, Seong Hwan, Daejeon 34114 (KR)
(74) Representative: Gerauer, Marc Philippé
(86) International application number: PCT/KR2020/018478
(87) International publication number: WO 2021/125803

(57) **Abstract**

The present invention relates to a novel pyrimidin derivative and a use thereof. The pyrimidin derivative has excellent MLK3 inhibitory activity and LRRK2 inhibitory activity and thus can be utilized in a pharmaceutical composition for preventing or treating cancers, virus infectious diseases, Parkinson's disease, nonalcoholic fatty liver disease, and tuberculosis.

## Description

### Technical Field

The present disclosure relates to a novel pyrimidine derivative and a use thereof and, more specifically, to a novel pyrimidine derivative having inhibitory activity against MLK3 and LRRK2 and a pharmaceutical composition comprising same for prevention or treatment of cancers, virus infectious diseases, Parkinson's disease, non-alcoholic steatohepatitis, and tuberculosis.

### Background Art

A protein kinase, functioning as an enzyme to catalyze the transfer of the terminal phosphate of adenosine triphosphate (ATP) to specific residues (tyrosine, serine, threonine) of proteins, is involved in the regulation of activity, growth, and differentiation of cells in response of changes of extracellular mediators and environments.

On the whole, protein kinases are classified into a serine- and/or threonine-specific protein kinase group and a tyrosine-specific protein kinase group according to the substrate to be phosphorylated (Hanks, S. K. et al., FASEB J., 9(8), 576-596, 1995). The serine/threonine-specific protein kinase group includes protein kinase C isoforms (Newton, A. C., J Biol Chem., 270(48), 28495-28498, 1995), cyclin-dependent protein kinases, and cdc2 (Pines, J., Trends Biochem Sci., 18(6), 197, 1993). The tyrosine-specific protein kinase group is divided into: membrane-spanning growth factor receptors including epithelial growth factor receptor (Iwashita, S. et al., Cell Signal., 4(2), 123-132, 1992); cytoplasmic non-receptor kinases including p56tck, p59fYn, and ZAP-70; and C-terminal Src kinase (Chan, A. C. et al., Annu Rev Immunol., 12, 555-592, 1994) .

Inappropriately high protein kinase activity is associated directly or indirectly with many diseases attributed to abnormal cellular actions. For example, mutation, over-expression or failure of appropriate regulatory mechanism of kinases involved in inappropriate enzyme activity; or excessive synthesis or deficiency of factors involved in upstream or downstream signal transduction of cytokines or kinases can cause disease. Therefore, selective inhibition of kinase activity can be a beneficial target for the development of new drugs for the treatment of disease.

MLK3, which is a member of the mixed lineage kinase (MLK) family, is implicated in multiple signaling cascades to regulate MAP kinases (inter alia, JNK) which play critical roles in the proliferation and survival of cancer cells.

In addition, many studied have recently been directed toward the development of compounds inhibitory of the enzymatic activity of MLK3 and advanced to therapies for cancer, virus infectious disease, Parkinson's disease, non-alcoholic steatohepatitis, etc. (Chadee, D. N., Can J Physiol Pharmacol., 91(4), 268-274, 2013; Rattanasinchai, C. et al., Cancers (Basel)., 8(5), 51, 2016; Xu, H. et al., J Virol., 93(18), e00758-19, 2019; Saminathan, P. et al., J Neuroimmune Pharmacol., 14(1), 44-51, 2019; Goodfellow, V. S. et al., J Med Chem., 56(20), 8032-8048, 2013; Jiang, J. X. et al., Liver Int., 34(8), 1131-1132, 2014; Tomita, K. et al., JCI Insight., 2(15), 94488, 2017; Ibrahim, S. H. et al., Liver Int., 34(3), 427-437, 2014; Parkinson Study Group PRECEPT Investigators, Neurology, 69(15), 1480-1490, 2007; Kline, E. M. et al., Exp Neurol., 318, 157-164, 2019).

LRRK2 (leucine-rich repeat kinase-2), which is a member of the leucine-rich repeat kinase family, is composed of 2527 amino acids with high interspecies similarity. Characteristically, it contains both GTPase activity and serine-threonine kinase activity in one protein. Expressed LRRK2 is found in various organs and tissues including the brain, existing in the cytoplasm or cell membrane and mitochondrial outer membrane at the cellular level. Currently, active studies are conducted on in vivo functions of LRRK2. LRRK2 has five functionally important domains which are involved in self-active regulation by autophosphorylation and cell function regulation by protein interaction and enzymatic action. Particularly, it is known that chaperone machinery, cytoskeleton arrangement, protein translational machinery, synaptic vesicle endocytosis, mitogen-activated protein kinases signaling cascades, and ubiquitin/autophageprotein degradation pathways are regulated by LRRK2.

In addition, a number of papers have been reported on the enzymatic activity of LRRK2 (Liu, Z. et al., Biochim Biophys Acta Proteins Proteom., 1865(3), 274-280, 2017; Herbst, S. et al., ACS Infect Dis., 5(6), 809-815, 2019; Hartlova, A. et al., EMBO J., 37(12), e98694, 2018).

With regard to compositions comprising pyrimidine derivatives as an active ingredient for prevention or treatment of cancer, virus infectious diseases, Parkinson's disease, non-alcoholic steatohepatitis, and tuberculosis, reference may be referred to Korean Patent Number 10-0832602, which discloses a novel polycyclic compound associated with the activity of MLK3 enzyme and a use thereof, Korean Patent Number 10-1876514, which discloses a novel pyrimidine compound and a pharmaceutical composition comprising same for prevention or treatment of cancer, and the paper [Haidasz, E. A. et al., Org Lett., 19(7), 1854-1857, 2017], which discloses the synthesis of diazaphenoxazine and diazaphenothiazine derivatives. However, the pyrimidine derivatives of Chemical Formulas 1 and 2 of the present disclosure that have inhibitory activity against MLK3 and LRRK2 and can be used for therapy for cancer, virus infectious diseases, Parkinson's disease, non-alcoholic steatohepatitis, and tuberculosis have not been disclosed anywhere in previous reports.

Leading to the present disclosure, the research has been conducted into pyrimidine derivatives and resulted in the finding that the pyrimidine derivatives are inhibitory of both MLK3 and LRRK2.

### Disclosure of Invention

### Technical Problem

The present disclosure aims to provide novel pyrimidine derivatives and a use thereof and, more specifically, to novel pyrimidine derivatives having inhibitory activity against MLK3 and LRRK2 and a pharmaceutical composition comprising same for prevention and treatment of cancer, virus infectious diseases, Parkinson's disease, non-alcoholic steatohepatitis, and tuberculosis.

### Solution to Problem

The present disclosure relates to compounds represented by the following Chemical Formulas 1 to 10 or pharmaceutically acceptable salts thereof.

The present disclosure relates to a compound represented by the following Chemical Formula 1, an optical isomer thereof, or a pharmaceutically acceptable salt thereof: 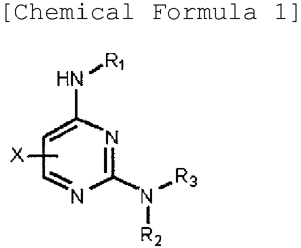 wherein,
R₁ is at least one substituent selected from the group consisting of a substituted or unsubstituted C₄-C₁₂ aryl, a substituted or unsubstituted C₄-C₁₂ heterocycloalkyl, a substituted or unsubstituted C₄-C₁₂ heterobicycloalkyl, a substituted or unsubstituted C₄-C₁₂ cycloalkyl, and a substituted or unsubstituted C₄-C₁₂ heterocycloaryl;
wherein the substituted aryl, heterocycloalkyl, cycloalkyl, or heteroaryl has as a substituent at least one selected from the group consisting of a hydrogen atom, 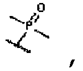 and 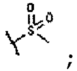
X is at least one selected from the group consisting of a hydrogen atom, halogen, hydroxy, a C₁-C₆ alkyl, and a C₁-C₆ alkoxy;
R₂ is at least one substituent selected from the group consisting of a hydrogen atom, a C₁-C₄ alkyl, and acetyl;
R₃ is one substituent selected from the group consisting of a substituted or unsubstituted C₄-C₁₂ aryl, a substituted or unsubstituted C₄-C₁₂ heterobicycloalkyl, and a substituted or unsubstituted C₄-C₁₂ heterobicycloaryl,
wherein the substituted aryl, heterocycloalkyl, or heteroaryl has as a substituent at least one selected from the group consisting of a hydrogen atom, halogen, cyano, aldehyde, CF₃, SH, trifluoroketone, a C₁-C₆ alkyl, a C₁-C₆ alkoxy, a substituted or unsubstituted C₄-C₁₂ cycloalkyl, a substituted or unsubstituted C₄-C₁₂ heterocycloalkyl, a substituted or unsubstituted C₄-C₁₂ aryl, and a substituted or unsubstituted C₄-C₁₂ heteroaryl,
wherein the substituted cycloalkyl, heterocycloalkyl, aryl, or heteroaryl has as a substituent at least one selected from the group consisting of a hydrogen atom, halogen, NH₂, hydroxy, CF₃, NO₂, methanesulfonate, Boc(tert-butoxycarbonyl), methylketone, aldehyde, a C₁-C₆ hydroxyalkyl, a C₁-C₆ alkyloxy, piperazine, and a C₁-C₆ alkyl.

The present disclosure pertains to compounds represented by the following Chemical Formulas 2 to 9, optical isomers thereof, or pharmaceutically acceptable salts thereof. 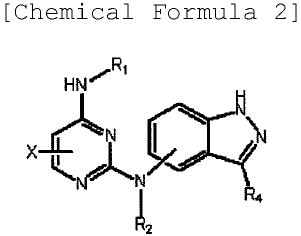 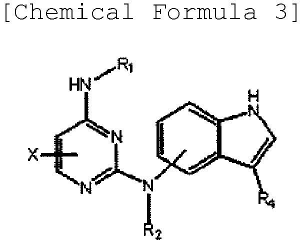 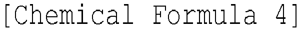 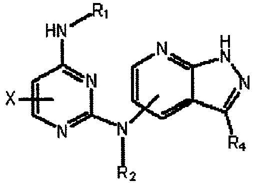 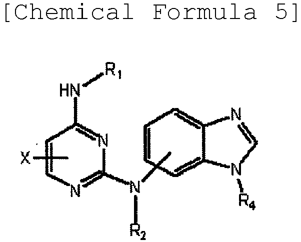 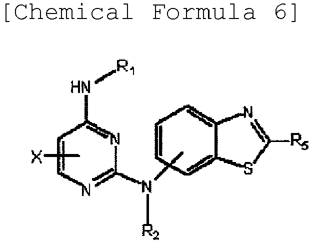 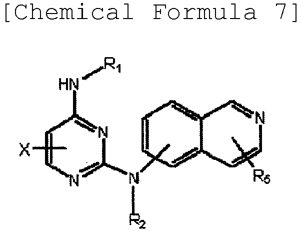 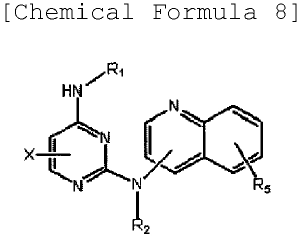 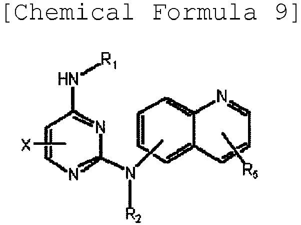 wherein,
R₁ is a substituted or unsubstituted C₄-C₁₂ aryl;
wherein the substituted aryl has as a substituent at least one selected from the group consisting of a hydrogen atom, 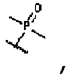 and 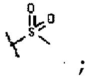
X is at least one selected from the group consisting of a hydrogen atom, halogen, hydroxy, a C₁-C₆ alkyl, and a C₁-C₆ alkoxy;
R₂ is at least one selected from the group consisting of a hydrogen atom, a C₁-C₄ alkyl, and acetyl;
R₄ is at least one selected from the group consisting of a hydrogen atom, halogen, cyano, aldehyde, CF₃, SH, trifluoroketone, C₁-C₆ alkyl, C₁-C₆ alkoxy, a substituted or unsubstituted C₄-C₁₂ cycloalkyl, a substituted or unsubstituted C₄-C₁₂ heterocycloalkyl, a substituted or unsubstituted C₄-C₁₂ aryl, and a substituted or unsubstituted C₄-C₁₂ heteroaryl,
wherein the substituted cycloalkyl, heterocycloalkyl, aryl, or heteroaryl has as a substituent at least one selected from the group consisting of a hydrogen atom, halogen, NH₂, hydroxy, CF₃, NO₂, methanesulfonate, Boc(tert-butoxycarbonyl), methylketone, aldehyde, a C₁-C₆ hydroxyalkyl, a C₁-C₆ alkyloxy, piperazine, and a C₁-C₆ alkyl;
R₅ is selected from a hydrogen atom, halogen, a C₁-C₄ alkyl, a C₁-C₄ alkoxy, hydroxy, a thio C₁-C₄ alkyl, and amino.

In addition, the present disclosure pertains to a compound represented by the following Chemical Formula 10, an optical isomer thereof, or a pharmaceutically acceptable salt thereof:

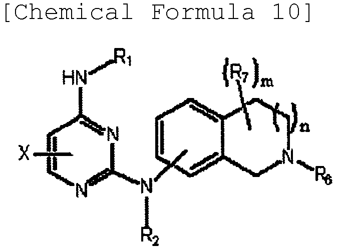

wherein,
R₁ is a substituted or unsubstituted C₄-C₁₂ aryl;
wherein the substituted aryl has as a substituent at least one selected from the group consisting of a hydrogen atom, 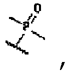 and 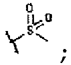
X is at least one substituent selected from the group consisting of a hydrogen atom, halogen, hydroxy, a C₁-C₆ alkyl, and a C₁-C₆ alkoxy;
R₂ is at least one substituent selected from the group consisting of a hydrogen atom, a C₁-C₄ alkyl, and acetyl;
R₆ is at least one substituent selected from the group consisting of a hydrogen atom, a C₁-C₆ alkyl, a halo C₁-C₆ alkylketone, trifluoroketone, acetyl, and Boc(tert-butoxycarbonyl);
R₇ is at least one substituent selected from the group consisting of a hydrogen atom, a C₁-C₆ alkyl, halogen, and hydroxy;
n is an integer of 0 or 1; and
m is an integer of 0, 1, or 2.

Unless otherwise indicated, the terms used herein have the meanings set forth therefor in the following. Any terms that are not defined herein should be understood to have the meaning commonly accepted in the art.

The term "halogen" refers to fluorine (F), chlorine (Cl), bromine (Br), or iodine (I).

The term "alkyl" refers to a monovalent linear or branched hydrocarbon radical. Examples include methyl, ethyl, propyl, n-butyl, iso-butyl, tert-butyl, and 1-methylpropyl.

The term "alkoxy" refers to a linear or branched, saturated alkyl radical singularly bonded to oxygen. Examples include methoxy, ethoxy, propoxy, n-butoxy, tert-butoxy, 1-methylpropoxy, etc.

The term "cycloalkyl" refers to a monovalent saturated hydrocarbon radical in a cyclic form. Examples include cyclopropyl, cyclobutyl, cyclopentyl, cyclohexyl, cycloheptyl, cyclooctyl, etc.

The term "heterocycloalkyl" or "heteroaryl" refers to a ring-shaped hydrocarbon radical bearing at least one heteroatom, such as N, O, or S, as a ring member. According to numbers and kinds of the heteroatoms and numbers of carbon atoms within the ring, there are various radicals including indazole, indole, tetrahydroisoquinoline, quinoline, isoquinoline, pyrrolidinyl, piperidinyl, piperazinyl, morpholinyl, tetrahydrofuranyl, tetrahydropyranyl, pyrrolyl, furanyl, pyridinyl, pyrimidinyl, pyran, etc.

The term "aryl" refers to an aromatic substituent having at least one ring with a pi-system of electrons delocalized therein, as exemplified by phenyl, benzyl, etc.

Moreover, each of the compounds of the present disclosure may have at least one chiral carbon atom and may be in a racemic mixture or an optically active form. All of the compounds and diastereomers fall within the scope of the present disclosure.

As used herein, the term "pharmaceutically acceptable salt" refers to a salt or complex of Chemical Formula 1 to 10, which retains a desired biological activity. Examples of the salt include, but are not limited to, acid addition salts formed with inorganic acids (e.g., hydrochloride, hydrobromide, sulfuric acid, phosphoric acid, nitric acid, etc.), and salts formed with organic acids such as acetic acid, oxalic acid, tartartic acid, succinic acid, malic acid, fumaric acid, maleic acid, ascorbic acid, benzoic acid, tannic acid, pamoic acid, alginic acid, polyglutamic acid, naphthalene sulfonic acid, naphthalene disulfonic acid, and poly-galacturonic acid. The compound may be administered in a form of a pharmaceutically acceptable quaternary salt known to those skilled in the art, as exemplified by, in particular, chloride, bromide, iodide, -O-alkyl, toluenesulfonate, methylsulfonate, sulfonate, phosphate, or carboxylates (e.g., benzoates, succinates, acetates, glycorates, maleates, malates, fumarates, citrates, tartrates, ascorbates, cinnamoates, mandeloate and diphenylacetate). The compounds of Chemical Formulas 1 to 10 according to the present disclosure may be provided in any form, including all salts, hydrates, solvates, and prodrugs that can be prepared by methods known in the art, as well as in the form of pharmaceutically acceptable salts thereof.

The acid addition salt according to the present disclosure may be prepared in a typical manner. By way of example, the derivatives of Chemical Formulas 1 to 10 are dissolved in an organic solvent such as methanol, ethanol, acetone, dichloromethane, acetonitrile, etc., to which organic acid or inorganic acid is added to induce precipitation. Then, the precipitate is filtered and dried to give the salt. Alternatively, the solvent and the excessive acid are distillated under a reduced pressure and dried, followed by crystallizing the precipitate in an organic solvent to give the salt.

A pharmaceutically acceptable metal salt can be prepared by using a base. Alkali metal or alkaline earth metal salt is obtained by, for example, dissolving the compound in excessive alkali metal hydroxide or alkaline earth metal hydroxide solution, filtering non-soluble compound salt, evaporating the filtrate, and drying same. In this regard, the metal salt is preferably prepared in the pharmaceutically suitable form of sodium, potassium, or calcium salt. In addition, the corresponding silver salt is prepared by reacting an alkali metal or alkaline earth metal salt with proper silver salt (ex; silver nitrate).

In another aspect, the present disclosure pertains to a composition comprising at least one of the compounds represented by Chemical Formulas 1 to 10 as an active ingredient for prevention or treatment of cancers, Parkinson's disease, virus infectious diseases, and tuberculosis. Each of the compounds represented by Chemical Formulas 1 to 10 has inhibitory activity against both MLK3 and LRRK2.

The cancer may be selected from the group consisting of lung cancer, liver cancer, stomach cancer, colorectal cancer, bladder cancer, prostate cancer, breast cancer, ovarian cancer, cervical cancer, thyroid cancer, melanoma, blood cancer, colon cancer, non-small cell lung cancer, pancreatic cancer, skin cell, head and neck cancer, small bowel cancer, rectal cancer, endometrial cancer, vaginal cancer, testis cancer, esophageal cancer, bile duct cancer, lymph gland cancer, gall bladder cancer, endocrine gland cancer, adrenal cancer, lymphoma, multiple myeloma, thymoma, mesothelioma, kidney cancer, brain cancer, tumors of central nervous system, brainstem glioma, and pituitary adenoma, but with no particular limitations thereto.

The virus infectious disease may be a disease caused by the infection of at least one virus selected from the group consisting of ZIKA virus, acquired human immunodeficiency virus (HIV), influenza virus, influenza A virus subtype H1N1, avian influenza virus, rhinovirus, adenovirus, coronavirus, parainfluenza virus, respiratory syncytial virus, herpesvirus (HSV), rotavirus, and hepatitis virus, but with no particular limitations thereto.

Together with a pharmaceutically acceptable carrier typically used, the pharmaceutical composition according to the present disclosure may be formulated into proper dosage forms. As used herein, the term "pharmaceutically acceptable" means physiologically acceptable and, when administered to human beings or animals, generally does not cause allergic responses, such as gastrointestinal disorder and dizziness, or similar reactions thereto. In addition, the composition may be formulated into oral dosage forms, topical agents, suppositories, and sterile injections, such as powders, granules, tablets, capsules, suspensions, emulsions, syrups, aerosols, etc.

Examples of a carrier, an excipient, or a diluent available in the composition include lactose, dextrose, sucrose, mannitol, xylitol, erythritol, maltitol, starch, Arabic gum, alginate, gelatin, calcium phosphate, calcium silicate, cellulose, methyl cellulose, microcrystalline cellulose, polyvinyl pyrrolidone, water, paraoxybenzoate methyl, paraoxybenzoate propyl, talc, magnesium stearate, and mineral oil, but are not limited thereto. For a formulation, a diluent or excipient such as a filler, a stabilizer, a binder, a disintegrant, a surfactant, etc. is typically used. Solid formulations for oral administration include tablets, pills, pulvis, granules, capsules, and so on. Such solid formulations are prepared by mixing the compound of the present disclosure with one or more excipients, for example, starch, microcrystalline cellulose, sucrose or lactose, low-substituted hydroxypropyl cellulose, hypromellose, etc. In addition to the simple excipients, lubricants, such as magnesium stearate, talc, etc., can be used. Liquid formulations for oral administration are suspensions, solutions, emulsions, and syrups, and may contain various excipients, for example, wetting agents, sweeteners, aromatics, and preservatives in addition to generally used simple diluents such as water and liquid paraffin. Formulations for parenteral administration are sterilized aqueous solutions, non-aqueous solvents, suspensions, emulsions, lyophilized preparations and suppositories. As non-aqueous solvents or solvents for suspensions, propylene glycol, polyethylene glycol, vegetable oil like olive oil, injectable ester like ethylolate, etc. may be employed. As a suppository base, witepsol, macrogol, tween 61, cacao butter, lauric butter, glycerol, gelatin, or the like may be used. For a formulation for parenteral administration, each of the pyrimidine derivation compounds of Chemical Formulas 1 to 10 or a pharmaceutically acceptable salt thereof may be sterilized and mixed in water, together with an adjuvant such as a preservative, a stabilizer, a hydrating agent or an emulsifying accelerator, a salt for controlling osmotic pressure, a buffer and the like, and other therapeutically useful substances, to give a solution or suspension, which is then prepared into the unit dosage form such as an ampoule or a vial.

A pharmaceutical composition comprising the compound of each of Chemical Formulas 1 to 10 disclosed herein as an active ingredient may be administered into mice, livestock, humans, etc. via various routes.

All modes of administration may be considered, and for example, it can be administered by oral, rectal or intravenous, intramuscular, subcutaneous, endometrial or cerebrovascular injection. The dose may vary depending on the age, sex, weight of the subject to be treated, the specific disease or pathological condition to be treated, the severity of the disease or pathological condition, the duration of administration, the route of administration, the absorption, distribution and excretion rate of drug, the types of other drugs used, the judgment of prescriber, and the like. Dose determination based on such factors is within the standards of those skilled in the art, and the doses generally range from 0.01 mg/kg/day to approximately 2000 mg/kg/day. A more preferred dose is 1 mg/kg/day to 500 mg/kg/day. The composition may be administered once a day or in several divided doses. The dosage does not limit the scope of the present invention in any way.

In addition, the pharmaceutical composition of the present disclosure may be used alone or in combination with surgical operation, hormone therapy, chemotherapy, and a biological response regulator, to prevent or treat cancer, virus infectious disease, Parkinson's disease, non-alcoholic steatohepatitis, and tuberculosis.

### Advantageous Effects of Invention

The present disclosure pertains to a novel pyrimidine derivative and a use thereof. With excellent inhibitory activity against MLK3 and LRRK2, the pyrimidine derivative can find advantageous applications in a composition for prevention or treatment of cancer, virus infectious disease, Parkinson's disease, non-alcoholic steatohepatitis, and tuberculosis.

### Best Mode for Carrying out the Invention

Below, a better understanding of the present disclosure may be obtained through the following examples which are set forth to illustrate, but are not to be construed as limiting the present disclosure.

### <EXAMPLE 1. Synthesis and Physicochemical Characterization of Pyrimidine Derivative>

Synthesis procedures and physicochemical properties of compounds 1 to 119 according to the present disclosure are as follows.

### Compound 1. 5-Chloro-N²-(3,5-dimethoxyphenyl)-N⁴-(2-(isopropylsulfonyl)phenyl)pyrimidine-2,4-diamine

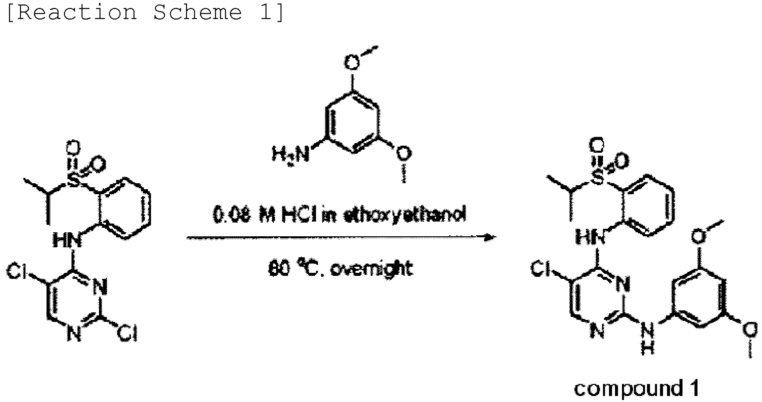

To 0.08 M ethoxyethanol (1 ml) were added 2,5-dichloro-N-[2-[(1-methylethyl)sulfonyl] phenyl] pyrimidine-4-amine (30 mg, 0.087 mmol) and then 3,5-dimethoxyaniline (15 mg, 0.095 mmol). The mixture was heated overnight at 80°C. The reaction was terminated by adding water, followed by extraction with DCM (2×25 ml). Subsequently, the organic layer was dried over Na₂SO₄ and the solvent was removed by evaporation in a vacuum. The crude mixture was subjected to purification by preparative thin layer chromatography using EA:MC (1/4) as an eluent to afford Compound 1 of the present disclosure (15 mg, 0.032 mmol, 38 %, ivory solid).

¹H NMR (300 MHz, CDCl₃) δ 9.64(s, 1H), 8.63(dd, J1=9.0 Hz, J2=0.0 Hz, 1H), 8.18(s, 1H), 7.93(dd, J1=7.5 Hz, J2=3.0 Hz, 1H), 7.66(t, J=7.5 Hz, 1H), 7.30(t, J=15 Hz, 1H), 6.96(s, 1H), 6.72(d, J=6.0 Hz, 1H), 6.24-6.20(m, 1H), 3.77(s, 6H), 3.32-3.19(m, 1H), 1.34(s, 3H), 1.32(s, 3H).

LC/MS (ESI) m/z 463.5 [M+H]⁺.

### Compound 2. 5-Chloro-N²-(3,5-dichlorophenyl)-N⁴-(2-(isopropylsulfonyl)phenyl)pyrimidine-2,4-diamine

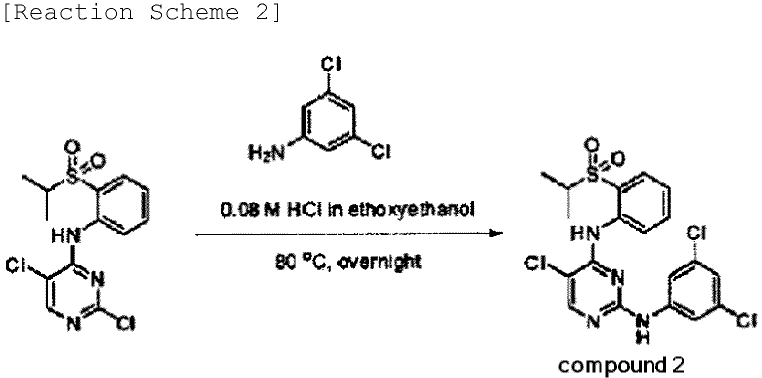

In a mixture of ethoxyethanol and 0.08 M HCl (1 ml) was dissolved 3,5-dichloroaniline (15 mg, 0.095 mmol), and the aminopyrimidine 2,5-dichloro-N-[2-[(1-methylethyl)sulfonyl] phenyl] pyrimidine-4-amine (30 mg, 0.087 mmol) was added thereto before heating overnight at 80°C. The reaction was terminated by adding water, followed by extraction with DCM (2×25 ml). Subsequently, the organic layer was dried over Na₂SO₄ and the solvent was removed by evaporation in a vacuum. The crude mixture was subjected to purification by preparative thin layer chromatography using EA:MC (1/4) as an eluent to afford Compound 2 of the present disclosure (14 mg, 0.030 mmol, 34 %, light pink solid).

¹H NMR (300 MHz, CDCl₃) δ 9.65(s, 1H), 8.47 (dd, J1=9.0 Hz, J2=0.0 Hz, 1H), 8.20(s, 1H), 7.96(dd, J1=9.0 Hz, J2 =0.0 Hz, 1H), 7.76(t, J=15 Hz, 1H), 7.51(d, J= 1.8 Hz, 1H) 7.32(t, J=18 Hz, 1H), 7.06(s, 1H), 7.05-7.00(m, 1H), 3.32-3.20(m, 1H), 1.35(s, 3H), 1.32(s, 3H).

LC/MS (ESI) m/z 471.3 [M+H]⁺.

### Compound 3. 5-Chloro-N²-(3,5-dimethylphenyl)-N⁴-(2-(isopropylsulfonyl)phenyl)pyrimidine-2,4-diamine

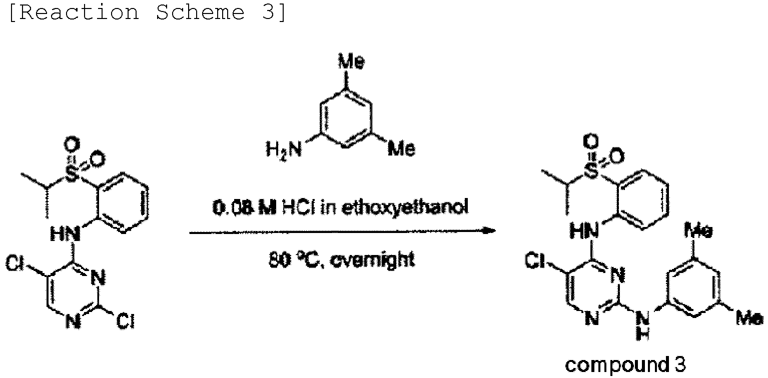

To 0.08M ethoxyethanol (1 ml) were added the aminopyrimidine 2,5-dichloro-N-[2-[(1-methylethyl)sulfonyl] phenyl] pyrimidine-4-amine (30 mg, 0.087 mmol) and then 3,5-dimethylaniline (0.022 ml, 0.17 mmol). The mixture was heated overnight at 80°C. The reaction was terminated by adding water, followed by extraction with DCM (2×25 ml). Subsequently, the organic layer was dried over Na₂SO₄ and the solvent was removed by evaporation in a vacuum. The crude mixture was subjected to purification by preparative thin layer chromatography using EA:MC (1/4) as an eluent to afford Compound 3 of the present disclosure (17 mg, 0.039 mmol, 45 %, beige solid).

¹H NMR (300 MHz, CDCl₃) δ 10.69(s, 1H), 10.58(s, 1H), 8.45(dd, J1=9.0 Hz, J2=0.9 Hz, 1H), 8.00(dd, J1=9.0 Hz, J2=9.3 Hz, 1H), 7.95(s, 1H), 7.61(t, J=15 Hz, 1H), 7.46(t, J=15 Hz, 1H), 7.17-7.13(m, 2H), 6.90(s, 1H), 3.30-3.17(m, 1H), 2.29(s, 6H), 1.36(s, 3H), 1.34(s, 3H).

LC/MS (ESI) m/z 346.3 [M+H]⁺.

### Compound 4. N²-(4-Bromo-3,5-difluorophenyl)-5-chloro-N⁴-(2-(isopropylsulfonyl)phenyl)pyrimidine-2,4-diamine

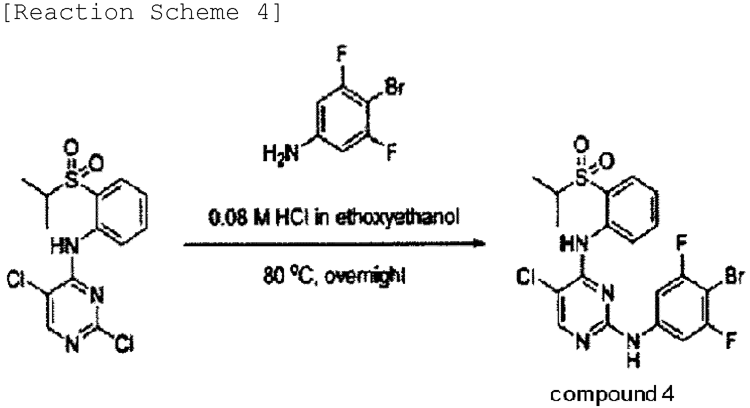

In a mixture of ethoxyethanol and 0.08 M HCl (1 ml) was dissolved 3,5-dichloroaniline (40 mg, 0.19 mmol), and the aminopyrimidine 2,5-dichloro-N-[2-[(1-methylethyl)sulfonyl] phenyl] pyrimidine-4-amine (30 mg, 0.087 mmol) was added thereto before heating overnight at 80°C. The reaction mixture was cooled to room temperature, and the resulting precipitate was filtered. The solid filtrate was washed with EtOH and dried to afford Compound 4 of the present disclosure (15 mg, 0.029 mmol, 33 %, beige solid).

¹H NMR (300 MHz, DMSO-d6) δ 10.09(s, 1H), 9.44(s, 1H), 8.40(s, 1H), 8.39-8.33(m, 1H), 7.90(dd, J1=14 Hz, J2=8.4 Hz, 1H), 7.82(t, J=15 Hz, 1H), 7.58-7.45(m, 3H), 3.53-3.41(m, 1H), 1.16(s, 3H), 1.14(s, 3H).

LC/MS (ESI) m/z 516.8 [M+H]⁺.

### Compound 5. N²-(3,5-bis(trifluoromethyl)phenyl)-5-chloro-N⁴-(2-(isopropylsulfonyl)phenyl)pyrimidine-2,4-diamine

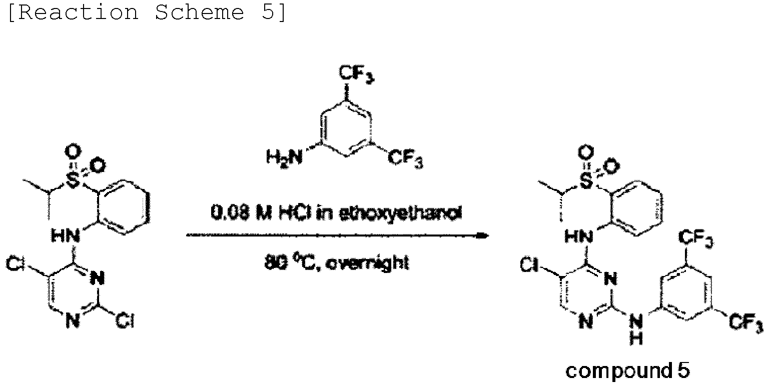

In a mixture of ethoxyethanol and 0.08 M HCl (1 ml) was dissolved 3,5-bis(trifluoromethyl)aniline (0.030 ml, 0.19 mmol), and the aminopyrimidine 2,5-dichloro-N-[2-[(1-methylethyl)sulfonyl] phenyl] pyrimidine-4-amine (30 mg, 0.087 mmol) was added thereto before heating overnight at 80°C. The reaction was terminated by adding water, followed by extraction with DCM (2×25 ml). Subsequently, the organic layer was dried over Na₂SO₄ and the solvent was removed by evaporation in a vacuum. The crude mixture was subjected to purification by preparative thin layer chromatography using MC (100%) as an eluent to afford Compound 5 of the present disclosure (25 mg, 0.046 mmol, 53 %, white solid).

¹H NMR (300 MHz, CDCl₃) δ 9.63(s, 1H), 8.36(d, J=8.4 Hz, 1H), 8.252(s, 1H), 8.05(m, 2H), 7.97(dd, J1=9.0 Hz, J2=1.8 Hz, 1H), 7.64(t, J= 15 Hz, 1H), 7.512(s, 1H), 7.33 (t, J=16 Hz, 1H), 7.246(s, 1H), 3.32-3.21(m, 1H), 1.34(s, 3H), 1.32(s, 3H).

LC/MS (ESI) m/z 538.9 [M+H]⁺.

### Compound 6. 5-Chloro-N²-(3,5-difluorophenyl)-N⁴-(2-(isopropylsulfonyl)phenyl)pyrimidine-2,4-diamine

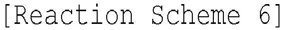

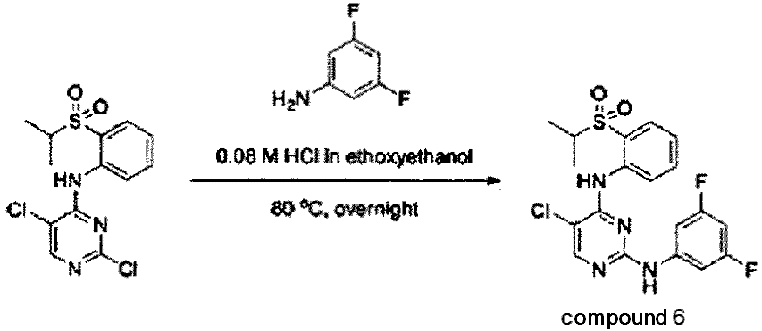

In a mixture of ethoxyethanoland and 0.08 M HCl (1 ml) was dissolved 3,5-difluoroaniline (25 mg, 0.19 mmol), and the aminopyrimidine 2,5-dichloro-N-[2-[(1-methylethyl)sulfonyl] phenyl] pyrimidine-4-amine (30 mg, 0.087 mmol) was added thereto before heating overnight at 80°C. The reaction mixture was cooled to room temperature, and the resulting precipitate was filtered. The solid filtrate was washed with EtOH and dried to afford Compound 6 of the present disclosure (32 mg, 0.073 mmol, 84 %, light pink solid).

¹H NMR (300 MHz, CDCl₃) δ 9.64(s, 1H), 8.49(dd, J1=9.0 Hz, J2=0.9 Hz, 1H), 8.209(s, 1H), 7.95(dd, J1=9.0 Hz, J2=1.5 Hz, 1H), 7.73(t, J=15 Hz, 1H), 7.33(t, J= 18 Hz, 1H), 7.24-7.13 (m, 1H), 7.09(s, 1H), 6.50(t, J=14 Hz, 1H), 3.31-3.20(m, 1H), 1.35(s, 3H), 1.32(s, 3H).

LC/MS (ESI) m/z 438.9 [M+H]⁺.

### Compound 7. (2-((5-Chloro-2-((5,5,8,8-tetramethyl-5,6,7,8-tetrahydronaphthalen-2-yl)amino)pyrimidin-4-yl)amino)phenyl)dimethylphosphine oxide

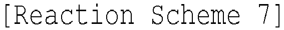

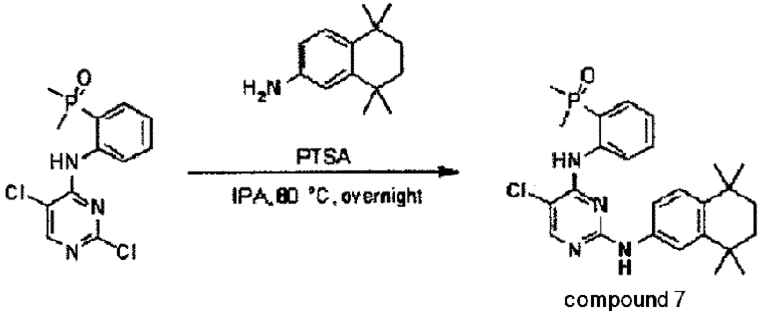

To s solution of 5,5,8,8-tetramethyl-5,6,7,8-tetrahydronaphthlane-2-amine (39 mg, 0.19 mmol) in IPA (0.6 mL) were added 2,5-dichloro-N-(2-(dimethylphosphoryl)phenyl)pyrimidine-4-amine (50 mg, 0.16 mmol) and then PTSA (30 mg, 0.16 mmol). The reaction mixture was heated overnight at 80°C. The crude mixture was subjected to purification by silica gel column chromatography using EA:MeOH (9.5/0.5) to afford Compound 7 of the present disclosure as a beige solid (43 mg, 0.089 mmol, 56%).

LC/MS (ESI) m/z 483.7 [M+H]⁺

¹H NMR (300 MHz, CDCl₃) δ 10.90 (s, 1H), 8.64 (dd, J = 9.0, 3.0 Hz, 1H), 8.11 (s, 1H), 7.58 (dd, J = 9.0, 3.0 Hz, 1H), 7.46 (t, J = 9.0 Hz, 1H), 7.33-7.26 (m, 2H), 7.20-7.09 (m, 2H), 1.87 (s, 3H), 1.83 (s, 3H), 1.70 (s, 4H), 1.30 (s, 6H), 1.26 (s, 6H)

### Compound 8. (2-((5-Chloro-2-(quinolin-5-ylamino)pyrimidin-4-yl)amino)phenyl)dimethylphosphine oxide

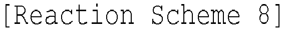

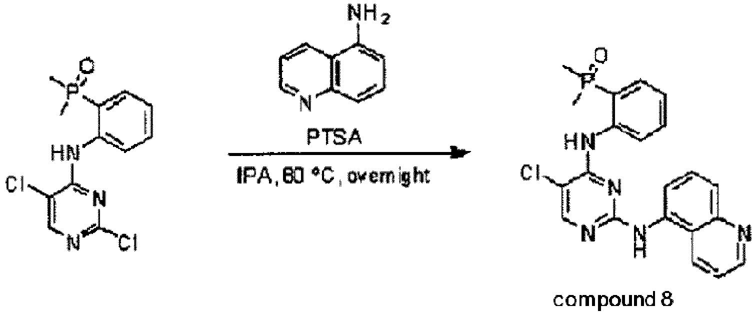

To a solution of 1H-indole-5-amine (25 mg, 0.19 mmol) in IPA (0.6 mL) were added 2,5-dichloro-N-(2-(dimethylphosphoryl)phenyl)pyrimidine-4-amine (50 mg, 0.16 mmol) and then PTSA (30 mg, 0.16 mmol). The reaction mixture was heated overnight at 80°C. The crude mixture was subjected to purification by silica gel column chromatography using EA:MeOH (9.5/0.5) to afford Compound 8 of the present disclosure as a solid (9.5 mg, 0.022 mmol, 14 %).

LC/MS (ESI) m/z 424.5 [M+H]⁺

¹H NMR (300 MHz, CDCl₃) δ 11.12 (s, 1H), 8.96 (dd, J = 6.0, 4.0 Hz, 1H), 8.47-8.38 (m, 1H), 8.15-8.06 (m, 2H), 8.04 (d, J = 9.0 Hz, 1H), 7.89-7.83 (m, 1H), 7.79-7.71 (m, 1H), 7.51 (bs, 1H), 7.43 (dd, J = 9.0, 3.0 Hz, 1H), 7.24-7.14 (m, 1H), 7.07-6.95 (m, 2H), 1.84 (s, 3H), 1.79 (s, 3H)

### Compound 9. (2-((2-(5-Amino-3,4-dihydroisoquinolin-2(1H)-yl)-5-chloropyrimidin-4-yl)amino)phenyl)dimethylphosphine oxide

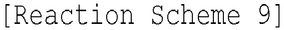

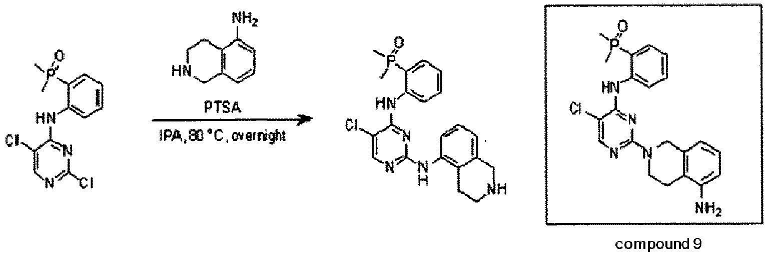

To a solution of 1,2,3,4-tetrahydroisoquinoline-5-amine (28 mg, 0.19 mmol) in IPA (0.6 mL) were added 2,5-dichloro-N-(2-(dimethylphosphoryl)phenyl)pyrimidine-4-amine (50 mg, 0.16 mmol) and then PTSA (30 mg, 0.16 mmol) . The reaction mixture was heated overnight at 80°C. The crude mixture was subjected to purification by silica gel column chromatography using EA:MeOH (9.5/0.5) to afford Compound 9 of the present disclosure as a solid (14 mg, 0.033 mmol, 21 %).

LC/MS (ESI) m/z 428.6 [M+H]⁺

¹H NMR (300 MHz, CDCl₃) δ 10.79 (s, 1H), 8.77-8.62 (m, 1H), 8.08 (s, 1H), 7.57 (t, J = 6.0 Hz, 1H), 7.35-7.26 (m, 1H), 7.17-7.09 (m, 1H), 7.05 (t, J = 9.0 Hz, 1H), 6.67 (d, J = 9.0 Hz, 1H), 6.60 (d, J = 6.0 Hz, 1H), 4.88 (s, 2H), 4.11 (t, J = 6.0 Hz, 2H), 3.78-3.52 (m, 2H), 2.68 (t, J = 6 Hz, 2H), 1.88 (s, 3H), 1.83 (s, 3H)

### Compound 10. (2-((5-Chloro-2-((2-methylpyridin-4-yl)amino)pyrimidin-4-yl)amino)phenyl)dimethylphosphine oxide

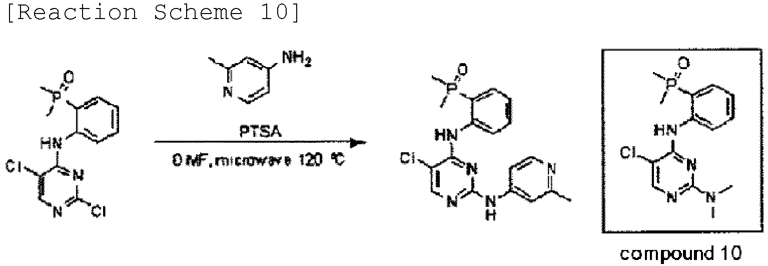

To a solution of 2-methyl pyridine-4-amine (26 mg, 0.24 mmol) in DMF (0.5 mL) were added 2,5-dichloro-N-(2-(dimethylphosphoryl)phenyl)pyrimidine-4-amine (50 mg, 0.16 mmol) and then PTSA (46 mg, 0.24 mmol) . The reaction mixture was heated at 120°C for 30 minutes in a microwave reactor. The reaction mixture was poured to H₂O and subjected to extraction with EA (2×25 mL). The organic layer thus pooled was dried over Na₂SO₄ and the solvent was removed by evaporation in a vacuum. The crude mixture was purified by using silica gel column chromatography using EA:MeOH (9.5/0.5) to afford Compound 10.

LC/MS (ESI) m/z 325.4 [M+H]⁺

¹H NMR (300 MHz, CDCl₃) δ 10.81 (s, 1H), 8.82-8.75 (m, 1H), 8.05 (s, 1H), 7.54-7.46 (m, 1H), 7.33-7.24 (m, 1H), 7.13-7.05 (m, 1H), 1.87 (s, 3H), 1.83 (s, 3H)

### Compound 11. (2-((2-(Benzo[d] thiazol-6-ylamino)-5-chloropyrimidin-4-yl)amino)phenyl)dimethylphosphine oxide

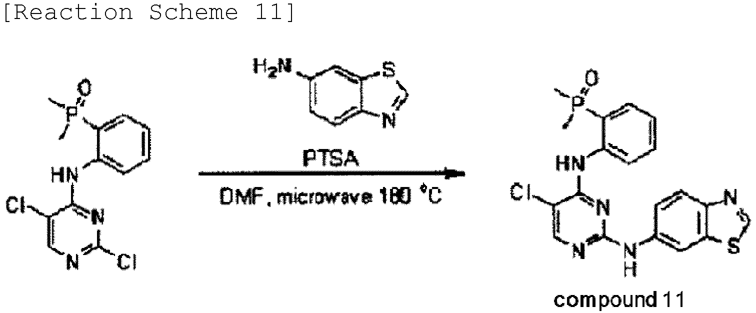

To a solution of 6-aminobenzothiazole (36 mg, 0.24 mmol) in DMF (0.5 mL) was added 2,5-dichloro-N-(2-(dimethylphosphoryl)phenyl)pyrimidine-4-amine (50 mg, 0.16 mmol) . Subsequently, PTSA (46 mg, 0.24 mmol) was added thereto. The reaction mixture was heated at 180 °C for 30 minutes in a microwave reactor. The reaction mixture was poured to H₂O and subjected to extraction with EA (2×25 mL). The organic layer thus pooled was dried over Na₂SO₄ and the solvent was removed by evaporation in a vacuum. The crude mixture was purified by using silica gel column chromatography using EA:MeOH (9.5/0.5) to afford Compound 11 as a yellow solid (26 mg, 0.060 mmol, 38 %).

LC/MS (ESI) m/z 430.4 [M+H]⁺

¹H NMR (300 MHz, CDCl₃) δ 10.97 (s, 1H), 8.89 (s, 1H), 8.62-8.54 (m, 2H), 8.17 (s, 1H), 8.05 (d, J = 8.7 Hz, 1H), 7.57-7.49 (m, 1H), 7.41(dd, J = 8.7, 2.1 Hz, 1H), 7.38-7.29 (m, 2H), 7.23-7.15 (m, 1H), 1.90 (s, 3H), 1.85 (s, 3H)

### Compound 12. (2-((5-Chloro-2-((4-fluorobenzyl)amino)pyrimidin-4-yl)amino)phenyl)dimethylphosphine oxide

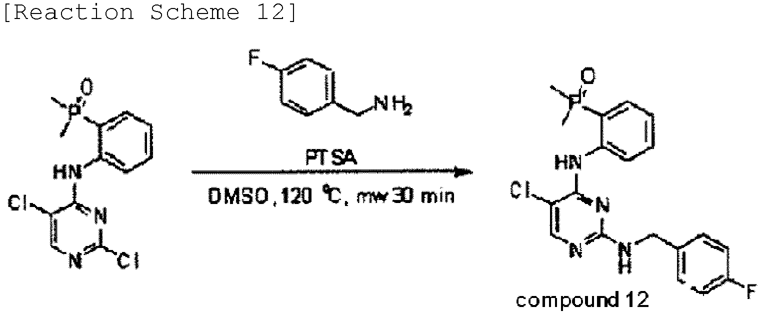

To a solution of (4-fluorophenyl)methanamine (0.28 mL, 0.19 mmol) in DMSO (0.5 mL) were added 2,5-dichloro-N-(2-(dimethylphosphoryl)phenyl)pyrimidine-4-amine (50 mg, 0.16 mmol) and then PTSA (9.1 mg, 0.048 mmol). The reaction mixture was heated 130°C for 1.5 hours in a microwave reactor. The reaction was quenched with water, followed by extraction with EtOAc (2×25 mL). The organic layer thus pooled was dried over MgSO₄ and the solvent was removed by evaporation in a vacuum. The crude mixture was subjected to purification by silica gel column chromatography using EA:MeOH (9.5/0.5) to afford compound 12 as a solid.

LC/MS (ESI) m/z 405.4 [M+H]⁺, 407.4 [M+H]⁺

¹H NMR (300 MHz, Chloroform-d) δ 10.87 (s, 1H), 8.55 - 8.42 (m, 1H), 8.03 (s, 1H), 7.41 - 7.30 (m, 3H), 7.28 - 7.21 (m, 1H), 7.11 - 6.98 (m, 3H), 5.39 - 5.33 (m, 1H), 4.58 (d, J = 6.0 Hz, 2H), 1.86 (s, 3H), 1.82 (s, 3H).

### Compound 13. (2-((5-Chloro-2-(cyclopentylamino)pyrimidin-4-yl)amino)phenyl)dimethylphosphine oxide

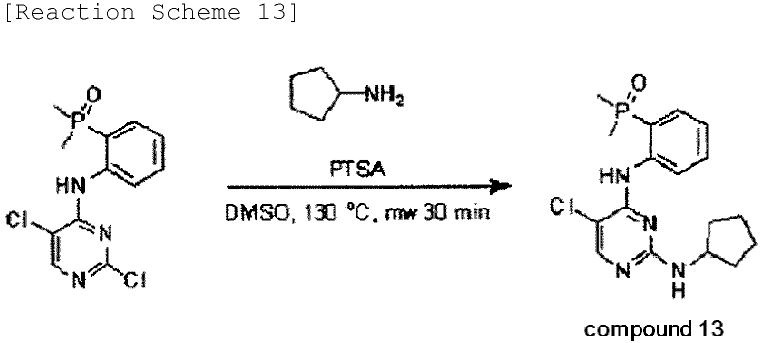

To a solution of cyclopentylamine (0.019 mL, 0.19 mmol) in DMSO (0.5 mL) were added 2,5-dichloro-N-(2-(dimethylphosphoryl)phenyl)pyrimidine-4-amine (50 mg, 0.16 mmol) and then PTSA (9.1 mg, 0.048 mmol). The reaction mixture was heated 130°C for 30 minutes in a microwave reactor. The reaction was quenched with water, followed by extraction with EtOAc (2×25 mL). The organic layer thus pooled was dried over Na₂So₄ and the solvent was removed by evaporation in a vacuum. The crude mixture was subjected to purification by silica gel column chromatography using DCM:MeOH (9.5/0.5) to afford compound 13 (45 mg, 0.12 mmol, 78 %) as a beige solid.

LC/MS (ESI) m/z [M+H]⁺, [M+H]⁺

¹H NMR (300 MHz, Chloroform-d) δ 10.83 (s, 1H), 8.86 - 8.58 (m, 1H), 8.00 (s, 1H), 7.51 (td, J = 8.7, 7.2 Hz, 1H), 7.33 - 7.22 (m, 1H), 7.10 (tdd, J = 7.4, 2.4, 1.1 Hz, 1H), 4.94 (d, J = 7.1 Hz, 1H), 4.22 (h, J = 6.5 Hz, 1H), 2.13 - 1.98 (m, 2H), 1.80 - 1.59 (m, 4H), 1.58 - 1.45 (m, 2H).

### Compound 14. (2-((2-(Benzylamino)-5-chloropyrimidin-4-yl)amino)phenyl)dimethylphosphine oxide

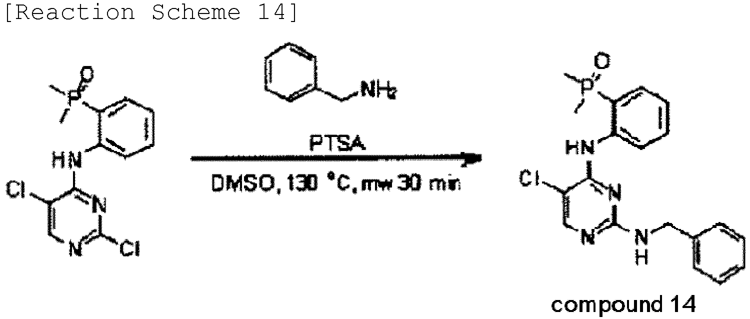

To a solution of benzylamine (0.021 mL, 0.19 mmol) in DMSO (0.4 mL) were added 2,5-dichloro-N-(2-(dimethylphosphoryl)phenyl)pyrimidine-4-amine (50 mg, 0.16 mmol) and then PTSA (9.1 mg, 0.048 mmol). The reaction mixture was heated 130°C for 30 minutes in a microwave reactor. The reaction was quenched with water, followed by extraction with EtOAc (2×25 mL). The organic layer thus pooled was dried over Na₂So₄ and the solvent was removed by evaporation in a vacuum. The crude mixture was subjected to purification by silica gel column chromatography using DCM:MeOH (9.5/0.5) to afford Compound 14 as an ivory solid (27 mg, 0.070 mmol, 44 %).

LC/MS (ESI) m/z [M+H]⁺, [M+H]⁺

¹H NMR (300 MHz, Chloroform-d) δ 10.85 (s, 1H), 8.47 (s, 1H), 8.02 (s, 1H), 7.37 (d, J = 4.4 Hz, 4H), 7.36 - 7.18 (m, 3H), 7.07 (tdd, 1H), 5.40 (s, 1H), 4.62 (d, J = 5.9 Hz, 2H), 1.86 (s, 3H), 1.81 (s, 3H).

### Compound 15. (2-((5-Chloro-2-(pyridin-4-ylamino)pyrimidin-4-yl)amino)phenyl)dimethylphosphine oxide

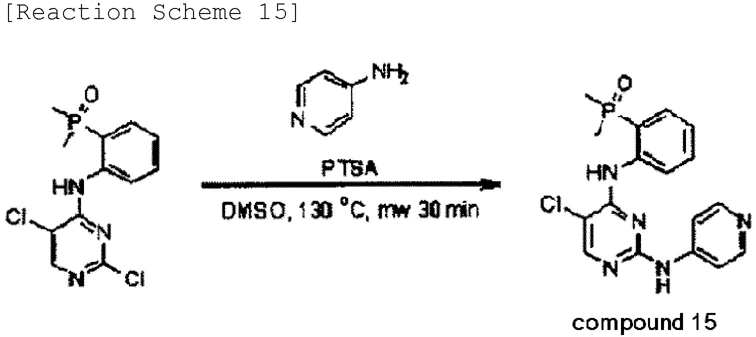

To a solution of 4-aminopyridine (18 mg, 0.19 mmol) in DMSO (0.3 mL) were added 2,5-dichloro-N-(2-(dimethylphosphoryl)phenyl)pyrimidine-4-amine (50 mg, 0.16 mmol) and then PTSA (9.1 mg, 0.048 mmol). The reaction mixture was heated 130°C for 30 minutes in a microwave reactor. The reaction was quenched with water, followed by extraction with EtOAc (2×25 mL). The organic layer thus pooled was dried over Na₂So₄ and the solvent was removed by evaporation in a vacuum. The crude mixture was subjected to purification by silica gel column chromatography using DCM:MeOH (9.5/0.5) to afford Compound 15 as an ivory solid (4.7 mg, 0.013 mmol, 7.8%) .

LC/MS (ESI) m/z [M+H]⁺, [M+H]⁺

¹H NMR (300 MHz, Chloroform-d) δ 11.57 (s, 1H), 8.69 (dd, J = 8.6, 4.4 Hz, 1H), 8.25 (s, 1H), 7.62 (t, J = 8.0 Hz, 1H), 7.28 (s, 4H), 7.21 (dt, J = 8.5, 4.2 Hz, 1H), 1.89 (s, 3H), 1.85 (s, 3H).

### Compound 16. (2-((5-Chloro-2-(cyclohexylamino)pyrimidin-4-yl)amino)phenyl)dimethylphosphine oxide

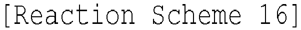

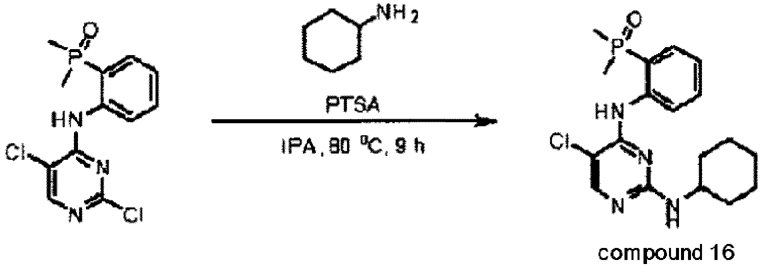

To a solution of 2,5-dichloro-N-(2-(dimethylphosphoryl)phenyl)pyrimidine-4-amine (51 mg, 0.16 mmol) in IPA (1 mL) were added cyclohexylamine (0.66 ml, 0.57 mmol) and then PTSA (30 mg, 0.16 mmol). The reaction mixture was heated overnight at 90°C. The crude mixture was subjected to purification by silica gel column chromatography using EA:Hex (9.5/0.5), followed by fractionation to afford Compound 16 as a white solid (46 mg, 0.12 mmol, 76 %).

LC/MS (ESI) m/z 379.5 [M+H]⁺, [M-H] -

¹H NMR (300 MHz, CDCl₃) δ 11.09 (s, 1H), 8.73 (s, 1H), 7.99 (s, 1H), 7.69-7.47 (m, 2H), 7.16-7.10 (m, 1H), 7.00 (s, 1H), 3.58 (s, 1H), 2.51 (p, J = 3.9, 1.8 Hz, 6H), 1.90 (d, J = 7.8 Hz, 2H), 1.83 (s, 1H), 1.81 (s, 2H), 1.79 (s, 1H), 1.72 (s, 1H), 1.61 (d, J = 12.6 Hz, 1H), 1.28 (s, 1H), 1.24 (s, 1H)

### Compound 17. (2-((5-Chloro-2-((2-methylbenzo[d] thiazol-6-yl)amino)pyrimidin-4-yl)amino)phenyl)dimethylphosphine oxide

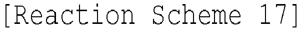

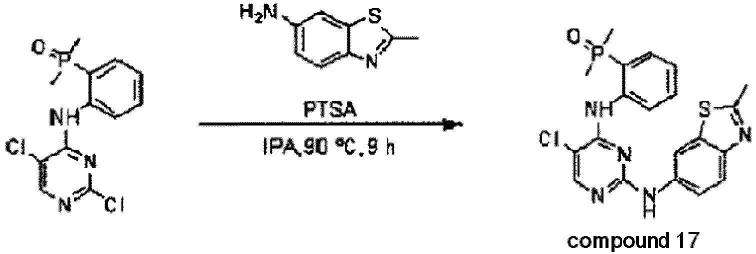

To a solution of 2,5-dichloro-N-(2-(dimethylphosphoryl)phenyl)pyrimidine-4-amine (51 mg, 0.16 mmol) in IPA (1 mL) were added 2-methylbenzo[d] thiazol-6-amine (39mg, 0.24 mmol) and then PTSA (46 mg, 0.24 mmol). The reaction mixture was heated overnight at 90°C. The crude mixture was subjected to purification by silica gel column chromatography using EA:MeOH (9.5/0.5) to afford compound 17 as a yellow solid (1.7 mg, 0.0038 mmol, 2.4 %).

LC/MS (ESI) m/z [M+H]⁺, [M-H] -

¹H NMR (300 MHz, CDCl₃) δ 10.97 (s, 1H), 8.58 (q, J = 8.7, 4.5 Hz, 1H), 8.43 (d, J = 2.1 Hz, 1H), 8.14 (s, 1H), 7.86 (d, J = 8.7 Hz, 1H), 7.56-7.51 (m, J = 7.5 Hz, 1H), 7.46 (s, 1H), 7.36 (dd, J = 8.7, 2.1 Hz, 1H), 7.33-7.30 (m, J = 7.8, 1.8 Hz, 1H), 7.23-7.18 (m, 1H), 2.85 (s, 3H), 1.90 (s, 3H), 1.85 (s, 3H)

### Compound 18. (2-((5-Chloro-2-(quinolin-6-ylamino)pyrimidin-4-yl)amino)phenyl)dimethylphosphine oxide

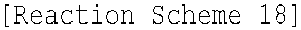

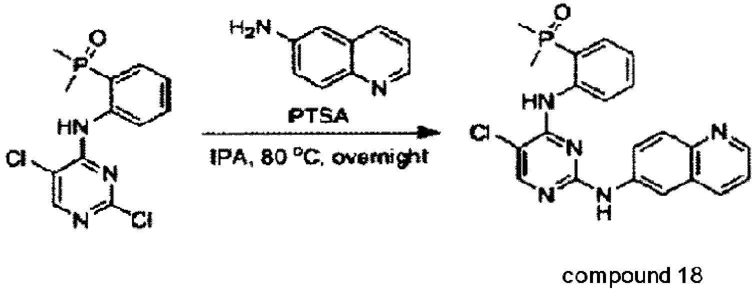

To a solution of quinoline-6-amino (28 mg, 0.19 mmol) in IPA (0.6 mL) were added 2,5-dichloro-N-(2-(dimethylphosphoryl)phenyl)pyrimidine-4-amine (50 mg, 0.16 mmol) and then PTSA·H₂O (36 mg, 0.19 mmol) . The reaction mixture was heated overnight at 80°C. TLC indicated the formation of a new spot. The reaction was quenched with sodium bicarbonate. After extraction with DCM (20 mL×2), the organic layer was dried over sodium sulfate and the solvent was removed by evaporation in a vacuum. The crude mixture was subjected to purification by MPLC using 5% MeOH:DCM as an eluent to afford Compound 18 as a softy solid (51 mg, 0.120 mmol, 75 %).

LCMS: 424.2 [M+H]⁺

¹H NMR (400 MHz, Chloroform-d) δ 11.03 (s, 1H), 8.82 (s, 1H), 8.69-8.62 (m, 1H), 8.29 (s, 1H), 8.20 (s, 1H), 8.04 (dd, J = 14.6, 8.7 Hz, 2H), 7.73 (d, J = 9.1 Hz, 1H), 7.49 (t, J = 7.9 Hz, 1H), 7.44-7.30 (m, 3H), 7.21 (t, J = 7.7 Hz, 1H), 1.90 (s, 3H), 1.87 (s, 3H).

### Compound 19. (2-((5-Chloro-2-(quinolin-3-ylamino)pyrimidin-4-yl)amino)phenyl)dimethylphosphine oxide

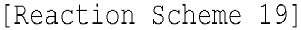

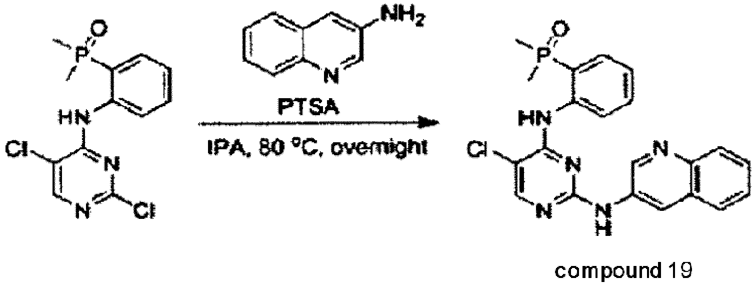

To a solution of quinolin-3-amine (28 mg, 0.19 mmol) in IPA (0.6 mL) were added 2,5-dichloro-N-(2-(dimethylphosphoryl)phenyl)pyrimidine-4-amine (50 mg, 0.16 mmol) and then PTSA (46 mg, 0.19 mmol) . The reaction mixture was heated overnight at 80°C. TLC indicated the formation of a new spot. The reaction was quenched with sodium bicarbonate, followed by extraction with DCM (20 mL×2). The organic layer was dried over sodium sulfate and the solvent was removed by evaporation in a vacuum. The crude mixture was subjected to purification by MPLC using 5% MeOH:DCM as an eluent to afford Compound 19 as a softy solid (60.0 mg, 0.141 mmol, 88%).

¹H NMR (400 MHz, Chloroform-d) δ 11.03 (s, 1H), 8.80 (s, 1H), 8.74 (s, 1H), 8.61-8.54 (m, 1H), 8.19 (s, 1H), 8.04 (d, J = 8.4 Hz, 1H), 7.68 (d, J = 8.2 Hz, 1H), 7.59 (t, J = 7.6 Hz, 1H), 7.49 (q, J = 9.0, 8.5 Hz, 2H), 7.37-7.31 (m, 1H), 7.31-7.28 (m, 1H), 7.19 (t, J = 7.7 Hz, 1H), 1.88 (s, 3H), 1.85 (s, 3H).

LCMS: 424.2 [M+H]⁺

### Compound 20. (2-((5-Chloro-2-(quinolin-8-ylamino)pyrimidin-4-yl)amino)phenyl)dimethylphosphine oxide

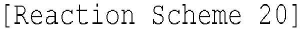

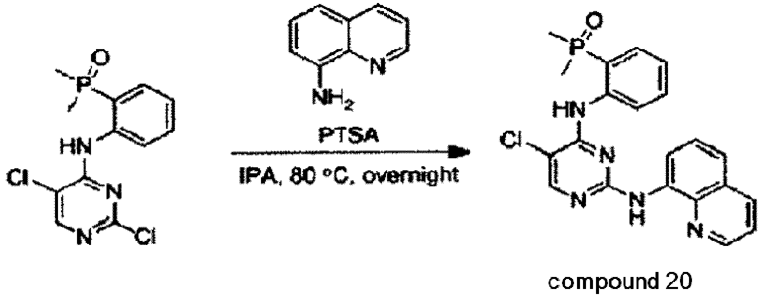

To a solution of quinolin-3-amine (28 mg, 0.19 mmol) in IPA (0.6 mL) were added 2,5-dichloro-N-(2-(dimethylphosphoryl)phenyl)pyrimidine-4-amine (50 mg, 0.16 mmol) and then PTSA (46 mg, 0.19 mmol) . The reaction mixture was heated overnight at 80°C. TLC indicated the formation of a new spot. The reaction was quenched with sodium bicarbonate, followed by extraction with DCM (20 mL×2). The organic layer was dried over sodium sulfate and the solvent was removed by evaporation in a vacuum. The crude mixture was subjected to purification by MPLC using 5% MeOH:DCM as an eluent to afford Compound 20 as a softy solid (55.0 mg, 0.141 mmol, 88 %).

LCMS 424.6 [M+H]⁺

¹H NMR (400 MHz, Chloroform-d) δ 10.86 (s, 1H), 9.64 (s, 1H), 8.83 (s, 1H), 8.78-8.67 (m, 2H), 8.24 (s, 1H), 8.15 (d, J = 8.2 Hz, 1H), 7.63 (t, J = 8.1 Hz, 1H), 7.47 (q, J = 11.2, 9.6 Hz, 2H), 7.39 (d, J = 8.2 Hz, 1H), 7.33 (dd, J = 14.1, 7.6 Hz, 1H), 7.19 (t, J = 7.8 Hz, 1H), 1.88 (s, 3H), 1.84 (s, 3H).

### Compound 21. (2-((5-Chloro-2-(isoquinolin-5-ylamino)pyrimidin-4-yl)amino)phenyl)dimethylphosphine oxide

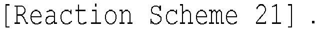

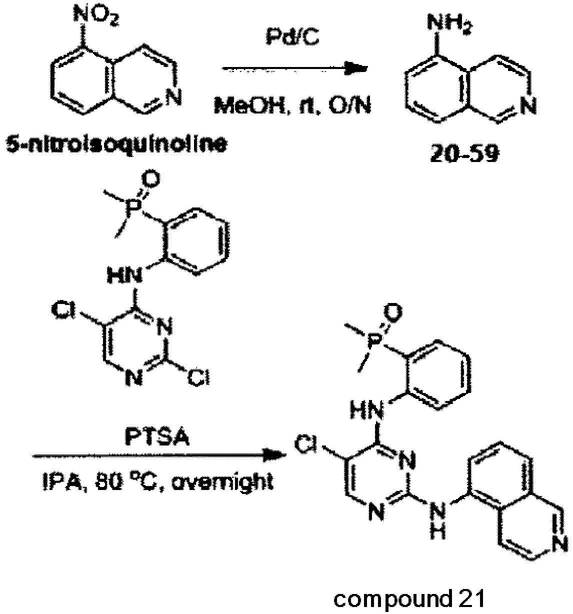

### 1) Synthesis of isoquinolin-5-amine

To a solution of 2,5-dichloro-N-(2-(dimethylphosphoryl)phenyl)pyrimidine-4-amine (100 mg, 0.413 mmol) in MeOH 10 mL was added Pd/C (10 mg, 10 mol%) which was then shaken overnight in a Parr shaker. After completion of the reaction as monitored by TLC, the catalyst was filtered out, and the solvent was evaporated to afford 20-59 as a purple solid (400 mg, 2.7 mmol, 96 %).

¹H NMR (500 MHz, Chloroform-d) δ 9.18 (s, 1H), 8.49 (d, J = 6.0 Hz, 1H), 7.58 (d, J = 6.0 Hz, 1H), 7.41 (d, J = 4.6 Hz, 2H), 6.96 (q, J = 4.4 Hz, 1H), 4.22 (s, 2H).

### 2) (2-((5-Chloro-2-(isoquinolin-5-ylamino)pyrimidin-4-yl)amino)phenyl)dimethylphosphine oxide

To a solution of 20-59 (25.0 mg, 0.17 mmol) in IPA (0.6 mL) were added 2,5-dichloro-N-(2-(dimethylphosphoryl)phenyl)pyrimidine-4-amine (50 mg, 0.16 mmol) and then PTSA (46 mg, 0.19 mmol) . The reaction mixture was heated overnight at 80°C. TLC indicated the formation of a new spot. The reaction was quenched with sodium bicarbonate, followed by extraction with DCM (20 mL×2). The organic layer was dried over sodium sulfate and the solvent was removed by evaporation in a vacuum. The crude mixture was subjected to purification by MPLC using 5% MeOH:DCM as an eluent to afford Compound 21 as a softy solid (25 g, 0.050 mmol, 31 %).

LCMS 424.0 [M+H]⁺

¹H NMR (400 MHz, Chloroform-d) δ 10.86 (s, 1H), 9.64 (s, 1H), 8.83 (s, 1H), 8.78-8.67 (m, 2H), 8.24 (s, 1H), 8.15 (d, J = 8.2 Hz, 1H), 7.63 (t, J = 8.1 Hz, 1H), 7.47 (q, J = 11.2, 9.6 Hz, 2H), 7.39 (d, J = 8.2 Hz, 1H), 7.33 (dd, J = 14.1, 7.6 Hz, 1H), 7.19 (t, J = 7.8 Hz, 1H), 1.88 (s, 3H), 1.84 (s, 3H).

### Compound 22. (2-((5-Chloro-2-((2,3-hydro-1H-inden-2-yl)amino)pyrimidin-4-yl)amino)phenyl)dimethylphosphine oxide

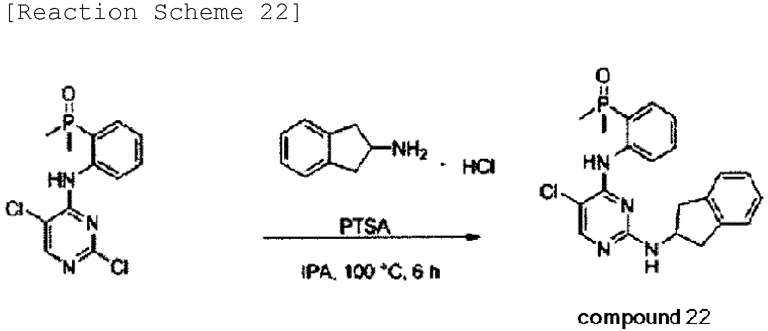

To a 5-mL vial were added 2,5-dichloro-N-(2-(dimethylphosphoryl)phenyl)pyrimidine-4-amine (50.0 mg, 0.158 mmol) and then 2-aminoindane hydrochloride (22.1 mg, 0.166 mmol), PTSA (48.1 mg, 0.506 mmol), and IPA (0.5 mL). The reaction mixture was heated at 100°C for 6 hours. The reaction was quenched with sodium bicarbonate, followed by extraction with DCM (50 mL×2) and water. The organic layer was dried over sodium sulfate and the solvent was evaporated in a vacuum. The mixture was subjected to purification by MPLC using 4 % to 5 % MeOH in MC (gradient) to afford Compound 22 as a pale pink solid (8 mg, 0.0194 mmol, 12 %).

¹H NMR (300 MHz, CDCl₃) δ 10.95 (s, 1H), 10.58 (s, 1H), 8.73 (dd, J = 8.6, 4.5 Hz, 1H), 8.56 (dd, J = 8.6, 4.6 Hz, 1H), 8.12 (d, J = 2.3 Hz, 1H), 7.62-7.53 (m, 1H), 7.46 (td, J = 7.9, 7.1, 1.4 Hz, 1H), 7.27-7.17 (m, 4H), 7.16-7.09 (m, 1H), 7.09-6.99 (m, 1H), 3.99-3.83 (m, 1H), 3.24 (dd, J = 15.8, 6.8 Hz, 2H), 2.80 (dd, J = 15.8, 5.2 Hz, 2H), 1.87-1.85 (m, 3H), 1.84-1.82 (m, 3H)

### Compound 23. (2-((5-Chloro-2-((2-mercaptobenzo[d] thiazol-6-yl)amino)pyrimidin-4-yl)amino)phenyl)dimethylphosphine oxide

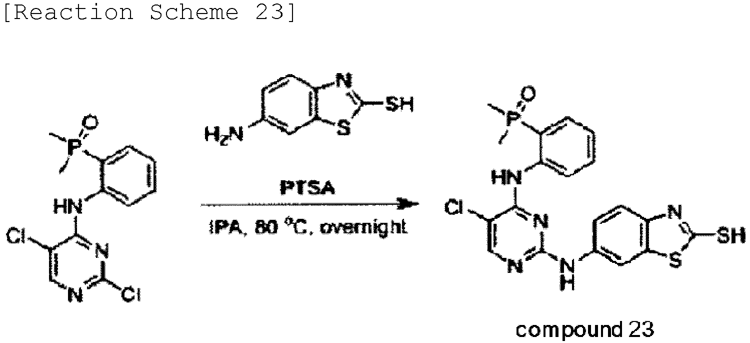

To a solution of 6-aminobenzo[d] thiazole-2-thiol (31 mg, 0.17 mmol) in IPA (0.6 mL) were added 2,5-dichloro-N-(2-(dimethylphosphoryl)phenyl)pyrimidine-4-amine (50 mg, 0.16 mmol) and then PTSA (46 mg, 0.19 mmol) . The reaction mixture was heated overnight at 80°C. TLC indicated the formation of a new spot. The reaction was quenched with sodium bicarbonate, followed by extraction with DCM (20 mL×2). The organic layer was dried over sodium sulfate and the solvent was removed by evaporation in a vacuum. The crude mixture was subjected to purification by MPLC using 5% MeOH:DCM as an eluent to afford Compound 23 as a downy solid (21 mg, 0.045 mmol, 28 %).

LCMS: 462.1 [M+H]⁺

¹H NMR (400 MHz, DMSO-d6) δ 13.64 (s, 1H), 11.06 (s, 1H), 9.66 (s, 1H), 8.45 (s, 1H), 8.22 (s, 1H), 8.12 (s, 1H), 7.64 (dd, J = 13.7, 7.7 Hz, 1H), 7.54 (t, J = 8.0 Hz, 1H), 7.46 (d, J = 8.8 Hz, 1H), 7.36-7.13 (m, 2H), 1.80 (s, 3H), 1.76 (s, 3H).

### Compound 24. (2-((5-Chloro-2-isopropoxypyrimidin-4-yl)amino)phenyl)dimethylphosphine oxide

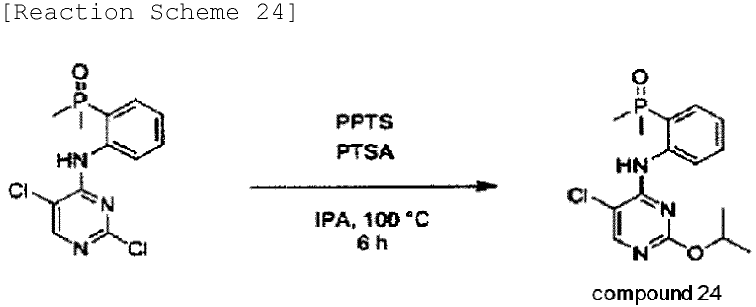

To a 5-mL vial were added 2,5-dichloro-N-(2-(dimethylphosphoryl)phenyl)pyrimidine-4-amine (100 mg, 0.316 mmol) and then *2-aminoindane hydrochloride (37.9 mg, 0.285 mmol), PPTS (37.9 mg, 0.285 mmol), PTSA (96.3 mg, 0.506 mmol), and IPA (1 mL). The reaction mixture was heated 100°C for 6 hours. The reaction was quenched with sodium bicarbonate, followed by extraction with DCM (30 mL×2) and water. The organic layer was dried over sodium sulfate and the solvent was evaporated in a vacuum. The mixture was subjected to purification by MPLC using 5 % MeOH in MC to afford 24 (22 mg, 0.0533 mmol, 17 %, white solid).

¹H NMR (400 MHz, CDCl₃) δ 11.04 (s, 1H), 8.66 (d, J = 9.1 Hz, 1H), 8.15 (d, J = 2.3 Hz, 1H), 7.54 (t, J = 8.1 Hz, 1H), 7.29 (d, J = 6.5 Hz, 1H), 7.12 (t, J = 7.8 Hz, 1H), 5.27-5.10 (m, 1H), 1.83 (s, 3H), 1.81 (s, 3H), 1.39 (q, J = 2.5 Hz, 6H).

### Compound 25. (2-((5-Chloro-2-((6-ethoxybenzo[d] thiazol-2-yl)amino)pyrimidin-4-yl)amino)phenyl)dimethylphosphine oxide

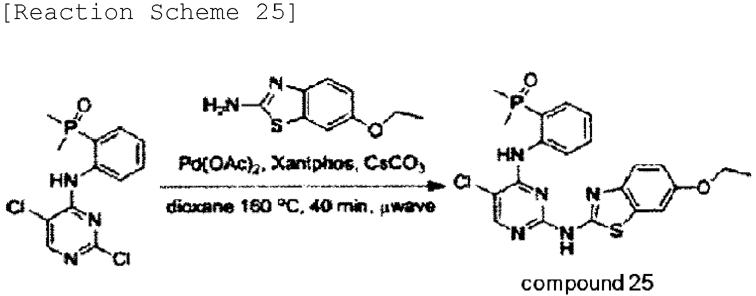

To a solution of 2-amino-6-ethoxybenzothiazole (37 mg, 0.19 mmol) in dioxane (0.6 mL) were added 2,5-dichloro-N-(2-(dimethylphosphoryl)phenyl)pyrimidine-4-amine (50 mg, 0.16 mmol) and then Pd(OAc)₂ (0.71 mg, 0.0032 mmol), xantphos (2.8 mg, 0.0048 mmol), and cesium carbonate (104 mg, 0.32 mmol). The reaction mixture was heated at 140°C for 40 minutes with microwaves. The resulting mixture was filtered on a celite bed and washed with EA, and the solvent was evaporated in a vacuum. The residue was subjected to purification by MPLC using 5 % MeOH:MC to afford Compound 25 as a yellow solid (42.0 mg, 0.103 mmol 55 %).

LCMS: 474.2 [M+H]⁺

¹H NMR (400 MHz, DMSO-d6) δ 11.72 (s, 1H), 11.49 (s, 1H), 8.89 (s, 1H), 8.37 (s, 1H), 7.70-7.55 (m, 2H), 7.55 (d, J = 8.8 Hz, 1H), 7.44 (d, J = 2.5 Hz, 1H), 7.25 (t, J = 7.6 Hz, 1H), 6.98 (dd, J = 8.8, 2.6 Hz, 1H), 4.06 (q, J = 6.9 Hz, 2H), 1.80 (d, J = 13.6 Hz, 6H), 1.35 (t, J = 7.0 Hz, 3H)

### Compound 26. (2-((5-Chloro-2-((4-methoxybenzo[d] thiazol-2-yl)amino)pyrimidin-4-yl)amino)phenyl)dimethylphosphine oxide

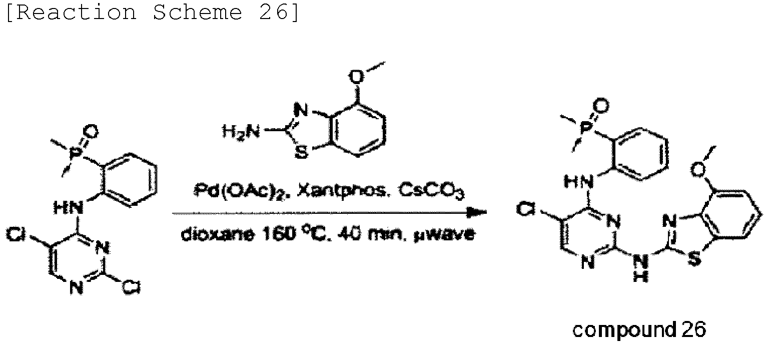

To a solution of 4-methoxybenzo[d] thiazol-2-amine (34 mg, 0.19 mmol) in dioxane (0.6 mL) were added 2,5-dichloro-N-(2-(dimethylphosphoryl)phenyl)pyrimidine-4-amine (50 mg, 0.16 mmol) and then Pd(OAc)₂ (0.71 mg, 0.0032 mmol), xantphos (2.8 mg, 0.0048 mmol), and cesium carbonate (104 mg, 0.32 mmol). The reaction mixture was heated at 140°C for 40 minutes with microwaves. The resulting mixture was filtered on a celite bed and washed with EA, and the solvent was evaporated in a vacuum. The residue was subjected to purification by MPLC using 5 % MeOH:MC to afford Compound 26 as a yellow solid (45.0 mg, 0.097 mmol 61 %).

¹H NMR (300 MHz, Chloroform-d) δ 11.08 (s, 1H), 8.75 (s, 1H), 8.57 (dd, J = 8.5, 4.4 Hz, 1H), 8.28 (s, 1H), 7.59-7.50 (m, 1H), 7.38-7.27 (m, 2H), 7.24-7.15 (m, 2H), 6.87 (dd, J = 8.1, 1.0 Hz, 1H), 4.02 (s, 3H), 1.88 (s, 3H), 1.83 (s, 3H).

### Compound 27. (2-((5-Chloro-2-((4-methylbenzo[d] thiazol-2-yl)amino)pyrimidin-4-yl)amino)phenyl)dimethylphosphine oxide

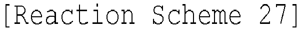

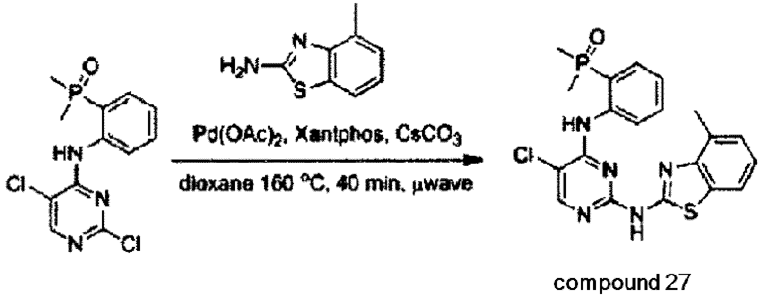

To a solution of 4-methylbenzo[d] thiazol-2-amine (31 mg, 0.19 mmol) in dioxane (0.6 mL) were added 2,5-dichloro-N-(2-(dimethylphosphoryl)phenyl)pyrimidine-4-amine (50 mg, 0.16 mmol) and then Pd(OAc)₂ (0.71 mg, 0.0032 mmol), xantphos (2.8 mg, 0.0048 mmol), and cesium carbonate (104 mg, 0.32 mmol). The reaction mixture was heated at 140°C for 40 minutes with microwaves. The resulting mixture was filtered on a celite bed and washed with EA, and the solvent was evaporated in a vacuum. The residue was subjected to purification by MPLC using 5 % MeOH:MC to afford Compound 27 as a yellow solid (40.0 mg, 0.0901 mmol, 56 %).

LCMS: 444.0 [M+H]⁺

¹H NMR (300 MHz, Chloroform-d) δ 11.13 (s, 1H), 9.26 (s, 1H), 8.61 (dd, J = 8.5, 4ff.4 Hz, 1H), 8.38 (s, 1H), 7.60 (d, J = 7.7 Hz, 1H), 7.60-7.49 (m, 1H), 7.34 (ddd, J = 14.0, 7.7, 1.6 Hz, 1H), 7.26-7.20 (m, 2H), 7.19-7.12 (m, 1H), 2.68 (s, 3H), 1.90 (s, 3H), 1.86 (s, 3H) .

### Compound 28. (2-((5-Chloro-2-((6-nitrobenzo[d] thiazol-2-yl)amino)pyrimidin-4-yl)amino)phenyl)dimethylphosphine oxide

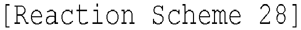

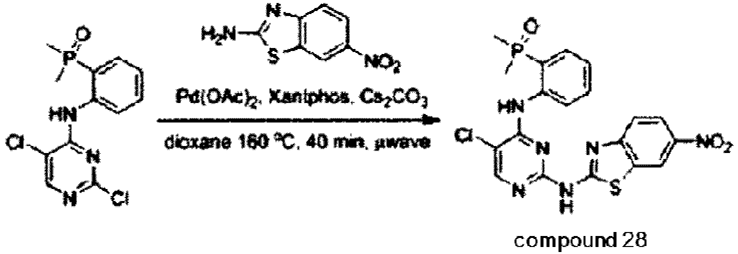

To a solution of 2-amino-6-nitrobenzothiazole (37 mg, 0.19 mmol) in dioxane (0.6 mL) were added 2,5-dichloro-N-(2-(dimethylphosphoryl)phenyl)pyrimidine-4-amine (50 mg, 0.16 mmol) and then Pd(OAc)₂ (0.71 mg, 0.0032 mmol), xantphos (2.8 mg, 0.0048 mmol), and cesium carbonate (104 mg, 0.32 mmol). The reaction mixture was heated at 140°C for 40 minutes with microwaves. The resulting mixture was filtered on a celite bed and washed with EA, and the solvent was evaporated in a vacuum. The residue was subjected to purification by MPLC using 5 % MeOH:MC to afford Compound 28 as a yellow solid (22.0 mg, 0.0463 mmol 28 %).

¹H NMR (300 MHz, DMSO-d6) δ 12.45 (s, 1H), 11.65 (s, 1H), 8.92 (d, J = 2.4 Hz, 1H), 8.46 (s, 1H), 8.25 (dd, J = 8.9, 2.5 Hz, 1H), 7.79 (d, J = 9.0 Hz, 1H), 7.71-7.52 (m, 3H), 7.26 (t, J = 7.4 Hz, 1H), 1.83 (s, 3H), 1.79 (s, 3H).

### Compound 29. (2-((5-Chloro-2-((6-chlorobenzo[d] thiazol-2-yl)amino)pyrimidin-4-yl)amino)phenyl)dimethylphosphine oxide

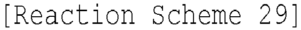

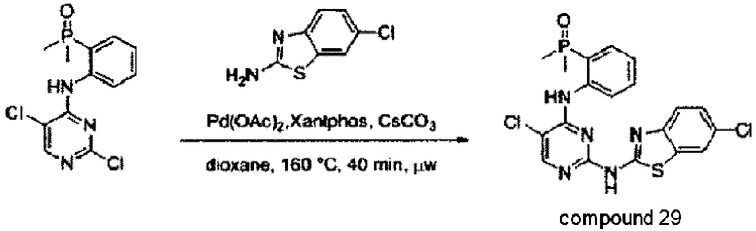

To a solution of 2-amino-6-chloro-benzothiazole (35 mg, 0.19 mmol) in dioxane (1mL) were added 2,5-dichloro-N-(2-(dimethylphosphoryl)phenyl)pyrimidine-4-amine (50 mg, 0.16 mmol) and then Pd(OAc)₂ (4.0 mg, 0.016 mmol), xantphos (9.2 mg, 0.016 mmol), and cesium carbonate (104 mg, 0.32 mmol). The reaction mixture was heated 160°C for 40 minutes with microwaves. The resulting mixture was filtered on a celite bed and washed with MeOH, and the solvent was evaporated in a vacuum. The residue was subjected to purification by MPLC using 5 % MeOH in MC to afford Compound 29 as a bright green solid (9.4 mg, 12 %).

LC/MS: 464.4[M + H+]

¹H NMR (400 MHz, CDCl₃) δ 11.07 (s, 1H), 9.55 (s, 1H), 8.49 (dd, J = 8.4, 4.3 Hz, 1H), 8.31 (s, 1H), 7.75-7.62 (m, 2H), 7.49 (t, J = 7.9 Hz, 1H), 7.37-7.28 (m, 2H), 7.23-7.17 (m, 1H), 1.90 (s, 3H), 1.87 (s, 3H)

### Compound 30. (((5-Chloropyrimidin-2,4-diyl)bis(azanediyl))bis(2,1-phenylene))bis(dimethylphosphine oxide)

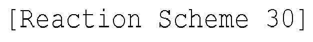

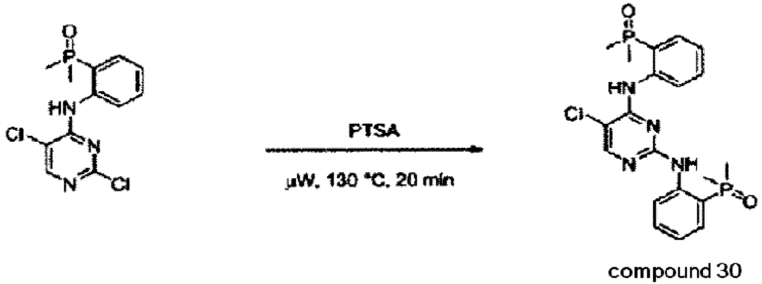

To a 5-mL vial were added 2,5-dichloro-N-(2-(dimethylphosphoryl)phenyl)pyrimidine-4-amine (10.0 mg, 0.0316 mmol) and then PTSA (10.8 mg, 0.0569 mmol) and IPA (0.5 mL). The reaction mixture was heated 130°C for 20 minutes with microwaves. The resulting mixture was subjected to purification by MPLC using 5 % MeOH in MC to afford Compound 30.

LC/MS: 448.82 [M+H+]

¹H NMR (400 MHz, CDCl₃) δ 10.96 (s, 1H), 10.60 (s, 1H), 8.75 (dd, J = 8.5, 4.5 Hz, 1H), 8.64-8.49 (m, 1H), 8.16 (s, 1H), 7.57 (ddt, J = 8.7, 7.3, 1.5 Hz, 1H), 7.47 (ddt, J = 8.6, 7.3, 1.4 Hz, 1H), 7.33-7.29 (m, 1H), 7.28-7.20 (m, 2H), 7.12 (tdd, J = 7.5, 2.4, 1.1 Hz, 1H), 7.03 (tdd, J = 7.6, 2.4, 1.1 Hz, 1H), 1.85 (dd, J = 13.1, 4.7 Hz, 12H).

### Compound 31. (2-((5-Chloro-2-((6-(methylsulfonyl)benzo[d] thiazol-2-yl)amino)pyrimidin-4-yl)amino)phenyl)dimethylphosphine oxide

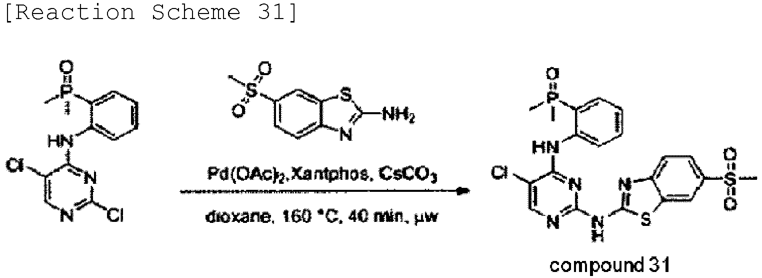

To a solution of 2-amino-6-(methylsulfonyl)benzothiazole (43 mg, 0.19 mmol) in dioxane (1mL) were added 2,5-dichloro-N-(2-(dimethylphosphoryl)phenyl)pyrimidine-4-amine (50 mg, 0.16 mmol) and then Pd(OAc)₂ (4.0 mg, 0.016 mmol), xantphos (9.2 mg, 0.016 mmol), and cesium carbonate (104 mg, 0.32 mmol). The reaction mixture was heated 160°C for 40 minutes with microwaves. The resulting mixture was filtered through a celite filter and washed with MeOH, and the solvent was evaporated in a vacuum. The residue was subjected to purification by MPLC using 5 % MeOH in MC to afford Compound 31 as a yellow solid (39.1 mg, 0.0708 mmol, 49 %).

LC/MS: 508.3[M+H+]

¹H NMR (400 MHz, CDCl₃) δ 11.15 (s, 1H), 11.08 (s, 1H), 8.41 (s, 1H), 8.38 - 8.33 (m, 1H), 8.29 (d, J = 1.9 Hz, 1H), 7.97 (d, J = 8.5 Hz, 1H), 7.79 (dd, J = 8.5, 1.9 Hz, 1H), 7.35 (ddd, J = 13.9, 7.6, 1.5 Hz, 2H), 7.19 (d, J = 7.7 Hz, 1H), 3.10 (s, 3H), 1.90 (s, 3H), 1.87 (s, 3H)

### Compound 32. (2-((5-Chloro-2-((6-(trifluoromethyl)benzo[d] thiazol-2-yl)amino)pyrimidin-4-yl)amino)phenyl)dimethylphosphine oxide

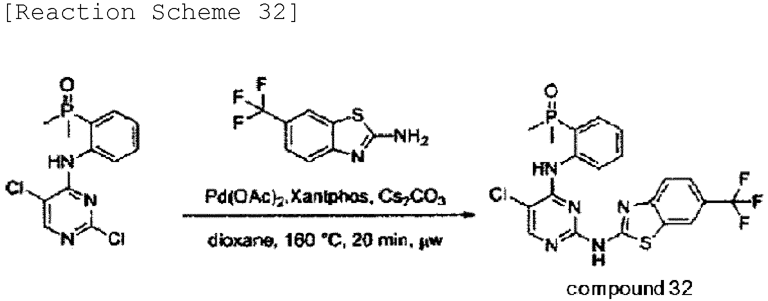

To a solution of 2-amino-6-(trifluoromethyl)benzothiazole (41 mg, 0.19 mmol) in dioxane (1 mL) were added 2,5-dichloro-N-(2-(dimethylphosphoryl)phenyl)pyrimidine-4-amine (50 mg, 0.16 mmol) and then Pd(OAc)₂ (4.0 mg, 0.016 mmol), xantphos (9.2 mg, 0.016 mmol), and cesium carbonate (104 mg, 0.32 mmol). The reaction mixture was heated at 160°C for 20 minutes with microwaves. The resulting mixture was filtered through a celite filter and washed with MeOH, and the solvent was evaporated in a vacuum. The residue was subjected to purification by MPLC using 3 % MeOH in MC to afford Compound 32 as a bright brown solid (14.3 mg, 0.0452 mmol, 18%).

LC/MS: 498.5[M+H+]

¹H NMR (400 MHz, CDCl₃) δ 11.08 (s, 1H), 10.91 (s, 1H), 8.42 (dd, J = 8.5, 4.3 Hz, 1H), 8.38 (s, 1H), 7.96 (d, J = 1.8 Hz, 1H), 7.92 (d, J = 8.5 Hz, 1H), 7.52 (dd, J = 8.5, 1.9 Hz, 1H), 7.44 - 7.28 (m, 3H), 7.17 (t, J = 6.9 Hz, 1H), 1.89 (s, 3H), 1.85 (s, 3H).

### Compound 33. (2-((2-(Benzo[d] thiazol-2-ylamino)-5-chloropyrimidin-4-yl)amino)phenyl)dimethylphosphine oxide

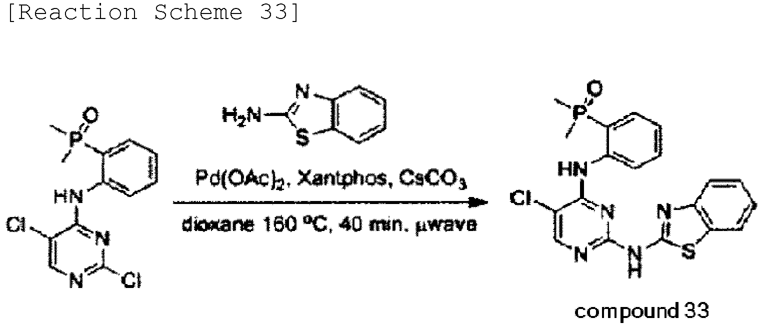

To a solution of benzo[d] thiazol-2-amine (38 mg, 0.19 mmol) in dioxane (0.6 mL) were added 2,5-dichloro-N-(2-(dimethylphosphoryl)phenyl)pyrimidine-4-amine (50 mg, 0.16 mmol) and then Pd(OAc)₂ (0.71 mg, 0.0032 mmol), xantphos (2.8 mg, 0.0048 mmol), and cesium carbonate (104 mg, 0.32 mmol). The reaction mixture was heated at 140°C for 40 minutes with microwaves. The resulting mixture was filtered on a celite bed and washed with EA, and the solvent was evaporated in a vacuum. The residue was subjected to purification by MPLC using 5 % MeOH:MC to afford Compound 33 as a yellow solid (17 mg, 0.039 mmol 24 %).

LCMS: 413.1 [M+H]⁺

¹H NMR (300 MHz, Chloroform-d) δ 11.50 (s, 1H), 9.42-9.31 (m, 1H), 8.68 (s, 1H), 8.32 (s, 1H), 7.72-7.57 (m, 2H), 7.45 (d, J = 8.0 Hz, 1H), 7.31 (t, J = 7.7 Hz, 2H), 7.19 (dd, J = 12.8, 6.7 Hz, 2H), 1.88 (s, 3H), 1.84 (s, 3H).

### Compound 34. (2-((5-Chloro-2-((6-fluorobenzo[d] thiazol-2-yl)amino)pyrimidin-4-yl)amino)phenyl)dimethylphosphine oxide

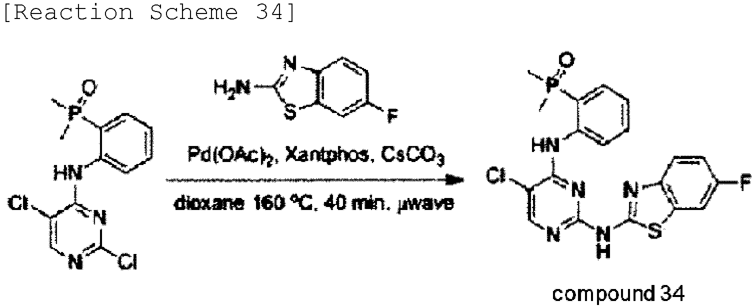

To a solution of 6-fluorobenzo[d] thiazol-2-amine (32 mg, 0.19 mmol) in dioxane (0.6 mL) were added 2,5-dichloro-N-(2-(dimethylphosphoryl)phenyl)pyrimidine-4-amine (50 mg, 0.16 mmol) and then Pd(OAc)₂ (0.71 mg, 0.0032 mmol), xantphos (2.8 mg, 0.0048 mmol), and cesium carbonate (104 mg, 0.32 mmol). The reaction mixture was heated at 140°C for 40 minutes with microwaves. The resulting mixture was filtered on a celite bed and washed with EA, and the solvent was evaporated in a vacuum. The residue was subjected to purification by MPLC using 5 % MeOH:MC to afford Compound 34 as a yellow solid (31.0 mg, 0.0692 mmol 43 %).

¹H NMR (400 MHz, DMSO-d6) δ 11.91 (s, 1H), 11.52 (s, 0H), 8.87 (s, 1H), 8.39 (s, 1H), 7.78 (dd, J = 8.6, 2.7 Hz, 1H), 7.69-7.61 (m, 2H), 7.58 (t, J = 7.9 Hz, 1H), 7.30-7.19 (m, 2H), 1.82 (s, 3H), 1.78 (s, 3H).

### Compound 35. (2-((5-chloro-2-((6-methylbenzo[d] thiazol-2-yl)amino)pyrimidin-4-yl)amino)phenyl)dimethylphosphine oxide

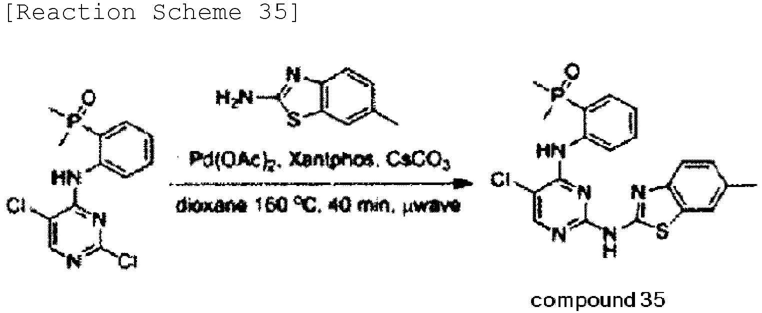

To a solution of 6-methylbenzo[d] thiazol-2-amine (32 mg, 0.19 mmol) in dioxane (0.6 mL) were added 2,5-dichloro-N-(2-(dimethylphosphoryl)phenyl)pyrimidine-4-amine (50 mg, 0.16 mmol) and then Pd(OAc)₂ (0.71 mg, 0.0032 mmol), xantphos (2.8 mg, 0.0048 mmol), and cesium carbonate (104 mg, 0.32 mmol). The reaction mixture was heated at 140°C for 40 minutes with microwaves. The resulting mixture was filtered on a celite bed and washed with EA, and the solvent was evaporated in a vacuum. The residue was subjected to purification by MPLC using 5 % MeOH:MC to afford Compound 35 as a yellow solid (46.0 mg, 0.103 mmol 64 %).

¹H NMR (400 MHz, DMSO-d6) δ 11.79 (s, 1H), 11.46 (s, 1H), 8.85 (s, 1H), 8.38 (s, 1H), 7.70-7.61 (m, 2H), 7.58 (t, 1H), 7.54 (d, J = 8.2 Hz, 1H), 7.26 (t, J = 7.2 Hz, 1H), 7.20 (dd, J = 8.4, 1.8 Hz, 1H), 2.40 (s, 3H), 1.81 (s, 3H), 1.78 (s, 3H).

### Compound 36. (2-((5-Chloro-2-((6-methoxybenzo[d] thiazol-2-yl)amino)pyrimidin-4-yl)amino)phenyl)dimethylphosphine oxide

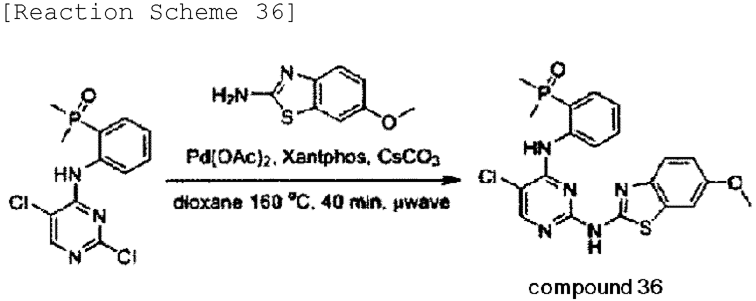

To a solution of 6-methoxybenzo[d] thiazol-2-amine (33 mg, 0.19 mmol) in dioxane (0.6 mL) were added 2,5-dichloro-N-(2-(dimethylphosphoryl)phenyl)pyrimidine-4-amine (50 mg, 0.16 mmol) and then Pd(OAc)₂ (0.71 mg, 0.0032 mmol), xantphos (2.8 mg, 0.0048 mmol), and cesium carbonate (104 mg, 0.32 mmol). The reaction mixture was heated at 140°C for 40 minutes with microwaves. The resulting mixture was filtered on a celite bed and washed with EA, and the solvent was evaporated in a vacuum. The residue was subjected to purification by MPLC using 5 % MeOH:MC to afford Compound 36 as a yellow solid (46.0 mg, 0.103 mmol 64 %).

¹H NMR (400 MHz, Chloroform-d) δ 11.05 (s, 1H), 9.36 (s, 1H), 8.55 (dd, J = 8.4, 4.4 Hz, 1H), 8.29 (s, 1H), 7.69 (d, J = 8.8 Hz, 1H), 7.55-7.46 (m, 1H), 7.36-7.29 (m, 1H), 7.22 (d, J = 2.5 Hz, 1H), 7.21-7.15 (m, 1H), 6.98 (dd, J = 8.8, 2.6 Hz, 1H), 3.86 (s, 3H), 1.87 (s, 3H), 1.84 (s, 3H).

### Compound 37. 5-Fluoro-N², N²-dimethyl-N⁴-(1-(methylsulfonyl)piperidin-4-yl)pyrimidine-2,4-diamine

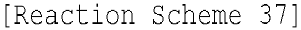

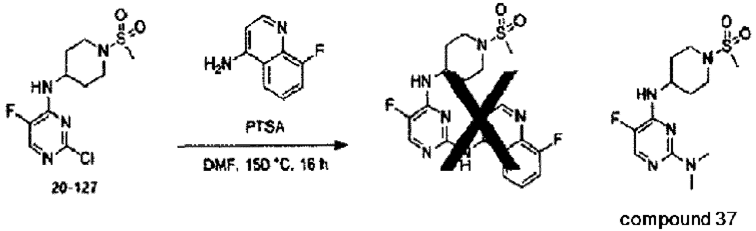

To a solution of 20-127 (50.0 mg, 0.162 mmol) in IPA (1 mL) were added 5-amino-1H-indazole (22.6 mg, 0.170 mmol) and then PTSA (46.2 mg, 0.243 mmol). The reaction mixture was heated at 90°C for 6 hours. The solid was filtered and alkalinized with sodium bicarbonate, followed by extraction with DCM. The reaction mixture was subjected to purification by silica gel column chromatography using 5 % MeOH:MC to afford Compound 37 as a yellow solid (26.5 mg, 0.0835 mmol, 52 %).

LC/MS: 318.52 [M + H+]

¹H NMR (400 MHz, CDCl₃) δ 7.76 (d, J = 3.4 Hz, 1H), 4.66 (d, J = 7.3 Hz, 1H), 4.06 (tdt, J = 11.0, 7.8, 4.1 Hz, 1H), 3.86-3.73 (m, 2H), 3.09 (s, 6H), 2.91 (ddd, J = 12.3, 11.1, 2.8 Hz, 2H), 2.82 (s, 3H), 2.26-2.14 (m, 2H), 1.66 (dtd, J = 12.9, 10.8, 4.1 Hz, 2H).

### Compound 38. 5-Fluoro-N²-(8-fluoroquinolin-4-yl)-N⁴-(1-(methylsulfonyl)piperidin-4-yl)pyrimidine-2,4-diamine

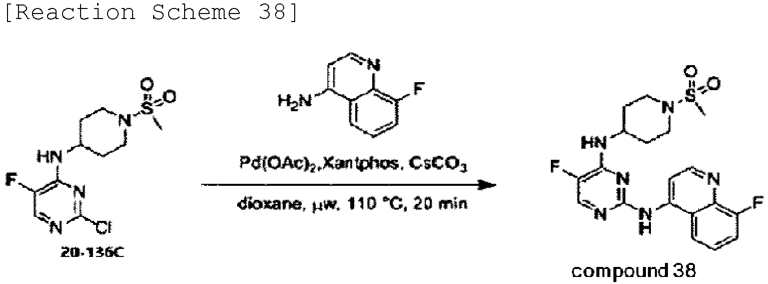

To a solution of 20-136C (50.0 mg, 0.162 mmol) in dioxane (1 mL) were added 8-fluoroquinoline-4-amine (30.8 mg, 0.194 mmol) and then Pd(OAc)₂ (3.64 mg, 0.0162 mmol), xantphos (9.2 mg, 0.0162 mmol), and cesium carbonate (105 mg, 0.324 mmol). The reaction mixture was heated at 110°C for 20 minutes with microwaves. The reaction mixture was filtered through a celite filter and the filtrate was washed with MeOH. After the solvent was evaporated in a vacuum, the residue was subjected to purification by MPLC using 80 % EA(in hex) to afford Compound 38 as a white solid (52.4 mg, 0.121 mmol, 74 %) .

LC/MS: 435.51 [M + H+]

¹H NMR (400 MHz, CDCl₃) δ 8.81 (d, J = 5.2 Hz, 1H), 8.48 (d, J = 5.2 Hz, 1H), 7.94 (d, J = 2.9 Hz, 1H), 7.75 (dt, J = 8.4, 1.2 Hz, 1H), 7.70 (s, 1H), 7.49 (td, J = 8.1, 5.2 Hz, 1H), 7.42 (ddd, J = 10.2, 7.8, 1.3 Hz, 1H), 5.11-5.00 (m, 1H), 4.21-4.14 (m, 1H), 3.87 (dd, J = 10.1, 6.3 Hz, 2H), 2.94 (td, J = 12.0, 2.6 Hz, 2H), 2.86 (s, 3H), 2.24 (dd, J = 13.0, 3.8 Hz, 2H), 1.79-1.70 (m, 2H) .

### Compound 39. (2-((5-Chloro-2-((3,4-dimethylisoxazol-5-yl)amino)pyrimidin-4-yl)amino)phenyl)dimethylphosphine oxide

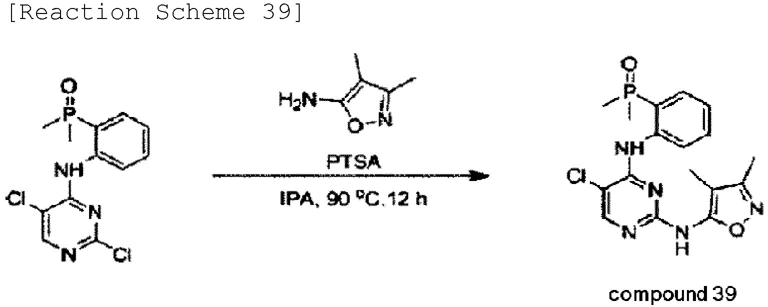

To a solution of (2-((2,5-dichloropyrimidin-4-yl)amino)phenyl)dimethylphosphine oxide (316 mg, 1.00 mmol) in IPA (3 mL) was added 3,4-dimethylisooxazol-5-amine (168 mg, 1.50 mmol). The reaction mixture was stirred at 90°C for 12 hours and then cooled to room temperature before concentration to remove IPA. The reaction mixture was added with water and alkalinized with sodium bicarbonate, followed by extraction with DCM. The organic layer was dried over sodium sulfate and the solvent was evaporated in a vacuum. The residue was subjected to purification by silica gel column chromatography using 5 % MeOH in DCM to afford Compound 39 as a white solid (220 mg, 0.0696 mmol, 56 %).

LC/MS 392.30[M+H+]

¹H NMR (400 MHz, CDCl₃) δ 11.25 (s, 1H), 8.69-8.59 (m, 1H), 8.20 (s, 1H), 7.60-7.58 (m, 1H), 7.34-7.28 (m, 1H), 7.17-7.14 (m, 1H), 2.47 (s, 3H), 1.87 (s, 3H), 1.86-1.85 (s, 3H), 1.82 (s, 3H).

### Compound 40. (2-((5-Chloro-2-((3,4-dimethylisoxazol-5-yl)amino)pyrimidin-4-yl)amino)phenyl)dimethylphosphine oxide hydrochloride

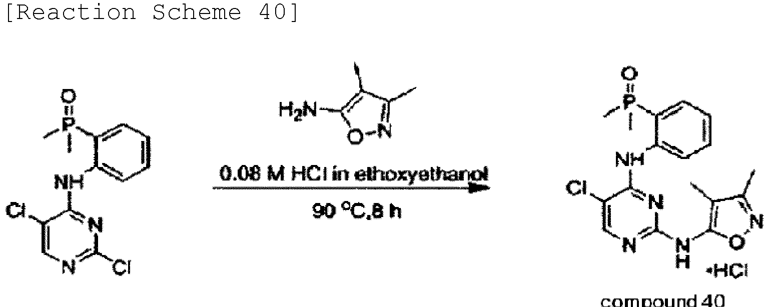

To a solution of (2-((2,5-dichloropyrimidin-4-yl)amino)phenyl)dimethylphosphine oxide (500 mg, 1.58 mmol) in ethoxyethanol containing 0.08 M HCl was added 3,4-dimethylisooxazol-5-amine (195 mg, 1.74 mmol). The reaction mixture was stirred at 90°C for 8 hours and then cooled to 0°C using a cold bath. The solid thus formed was filtered and washed with 2-ethoxyethanol (3 mL). Dryness in a high vacuum afforded Compound 40 as a white solid (380 mg, 0.970 mmol, 61 %).

LC/MS: 392. 51 [M + H+]

¹H NMR (300 MHz, DMSO) δ 11.82 (s, 1H), 10.20 (s, br, 2H), 8.60 (s, 1H), 8.43 (m, 1H), 7.76-7.64 (m, 2H), 7.30 (m, 1H), 2.65 (s, 3H), 1.99 (s, 3H), 1.84 (s, 3H), 1.82 (s, 3H).

### Compound 41. 7-((5-Chloro-4-((2-(dimethylphosphoryl)phenyl)amino)pyrimidin-2-yl)amino)-3,4-dihydroisoquinolin-1(2H)-one

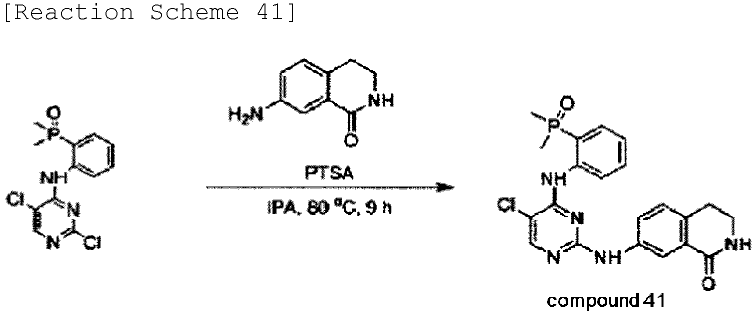

To a solution of 2,5-dichloro-N-(2-(dimethylphosphoryl)phenyl)pyrimidine-4-amine (51 mg, 0.16 mmol) in IPA (1 mL) were added 7-amino-3,4-dihydroisoquinolin-1(2H)-one (31 mg, 0.19 mmol) and then PTSA (30 mg, 0.16 mmol). The reaction mixture was heated overnight at 80°C. The solid thus formed was filtered and washed with EtOH to afford Compound 41.

LC/MS (ESI) m/z [M+H]⁺, [M-H] -

¹H NMR (300 MHz, CDCl₃) δ 10.94 (s, 1H), 8.64-8.60 (m, 1H), 8.14 (s, 1H), 8.01 (d, J = 2.4 Hz, 1H), 7.95-7.91 (m, 1H), 7.55-7.49 (m, 1H), 7.34-7.26 (m, 3H), 7.20-7.11 (m, 1H), 6.14 (s, 1H), 3.61-3.56 (m, 2H), 3.51 (s, 1H), 2.99 (t, J = 6.6 Hz, 2H), 1.88 (s, 3H), 1.835 (s, 3H)

### Compound 42. 1-(7-((5-Chloro-4-((2-(dimethylphosphoryl)phenyl)amino)pyrimidin-2-yl)amino)-1,1-dimethyl-3,4-dihydroisoquinolin-2(1H)-yl)-2,2,2-trifluoroethan-1-one

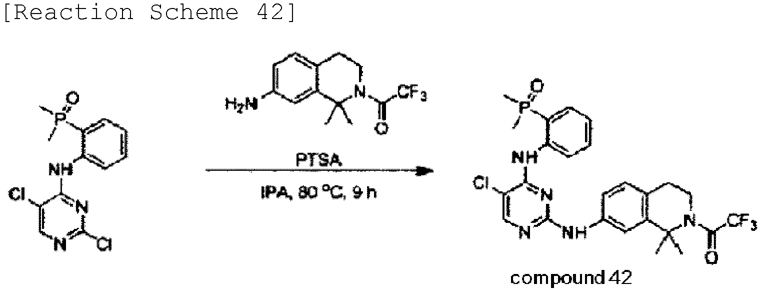

To a solution of 2,5-dichloro-N-(2-(dimethylphosphoryl)phenyl)pyrimidine-4-amine (51 mg, 0.16 mmol) in IPA (1mL) were added 1-(7-amino-1,1-dimethyl-3,4-dihydroisoquinolin-2(1H)-yl)-2,2,2-trifluoroethan-1-one (52 mg, 0.19 mmol) and then PTSA (30 mg, 0.16 mmol). The reaction mixture was heated overnight at 80°C. The solid thus formed was filtered and washed with EtOH to afford Compound 42.

LC/MS (ESI) m/z [M+H]⁺, [M-H] -

¹H NMR (300 MHz, CDCl₃) δ 10.88 (s, 1H), 8.57-8.53 (m, 1H), 8.57-8.53 (m, 1H), 8.14 (s, 1H), 7.52-7.46 (m, 2H), 7.41 (d, J = 2.1 Hz, 1H), 7.35-7.30 (m, 1H), 7.18-7.12 (m, 1H), 7.07 (d, J = 8.1 Hz, 1H), 7.00 (s, 1H), 3.67 (t, J = 3 Hz, 2H), 2.88 (t, J = 6 Hz, 2H), 1.88 (s, 3H), 1.84 (s, 3H), 1.79 (s, 6H)

### Compound 43. 1-(7-((5-Chloro-4-((2-(dimethylphosphoryl)phenyl)amino)pyrimidin-2-yl)(ethyl)amino)-1,1-dimethyl-3,4-dihydroisoquinolin-2(1H)-yl)-2,2,2-trifluoroethan-1-one

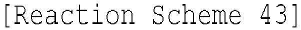

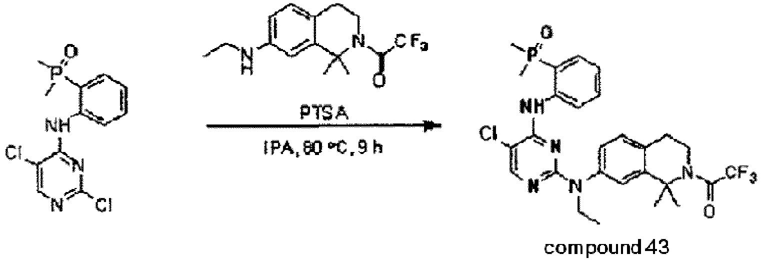

To a solution of 2,5-dichloro-N-(2-(dimethylphosphoryl)phenyl)pyrimidine-4-amine (51 mg, 0.16 mmol) in IPA (1mL) were added 1-(7-(ethylamino)-1,1-dimethyl-3,4-dihydroisoquinolin-2(1H)-yl)-2,2,2-trifluoroethan-1-one (57 mg, 0.19 mmol) and then PTSA (30 mg, 0.16 mmol). The reaction mixture was heated overnight at 80°C. The solid thus formed was filtered, washed with EtOH, and dried to afford Compound 43.

LC/MS (ESI) m/z 580.2 [M+H]⁺

¹H NMR (300 MHz, CDCl₃) δ 10.9 (s, br, 1H), 8.27-8.26 (m, 1H), 8.06 (s, 1H), 7.23-6.99 (m, 6H), 3.99 (q, J = 7.0 Hz, 2H), 3.72-3.70 (m, 2H), 2.99-2.96 (m, 2H), 1.85 (s, 3H), 1.80 (s, 3H), 1.77 (s, 6H), 1.28 (t, J = 7.2 Hz, 3H).

### Compound 44. (2-((5-Chloro-2-((1,1-dimethyl-1,2,3,4-tetrahydroisoquinolin-7-yl)amino)pyrimidin-4-yl)amino)phenyl)dimethylphosphine oxide

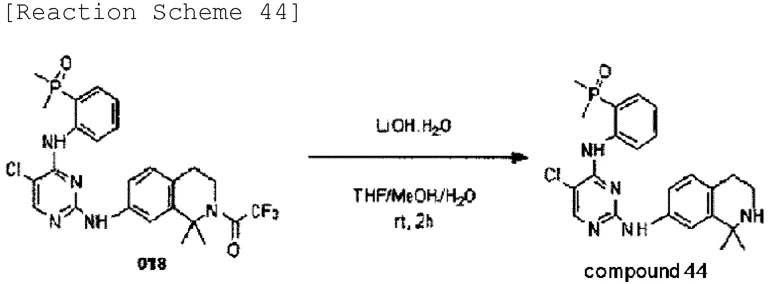

To a solution of 018 Compound (14 mg, 0.03 mmol) in a mixture of THF (2 mL), MeOH (1 mL), and H₂O (0.5 mL) was added lithium hydroxide monohydrate (2.5 mg, 0.06 mmol) at room temperature, and the reaction mixture was stirred overnight at room temperature. The solid thus formed was filtered, washed with EtOH, and dried to afford Compound 44.

LC/MS (ESI) m/z 456.2 [M+H]⁺

¹H NMR (300 MHz, CDCl₃) δ 11.2 (s, br, 1H), 9.30 (s, br, 1H), 8.50 (s, 1H), 8.29 (s, 1H), 7.70-7.49 (m, 4H), 7.40 (s, 1H), 7.19-7.10 (m, 1H), 6.85-6.80 (m, 1H), 2.90-2.85 (m, 2H), 2.68-2.59 (m, 2H), 1.85 (s, 3H), 1.80 (s, 3H), 1.35 (s, 6H)

### Compound 45. (2-((5-Chloro-2-((2-(5-chloro-4-((2-(dimethylphosphoryl)phenyl)amino)pyrimidin-2-yl)-1,2,3,4-tetrahydroisoquinolin-5-yl)amino)pyrimidin-4-yl)amino)phenyl)dimethylphosphine oxide

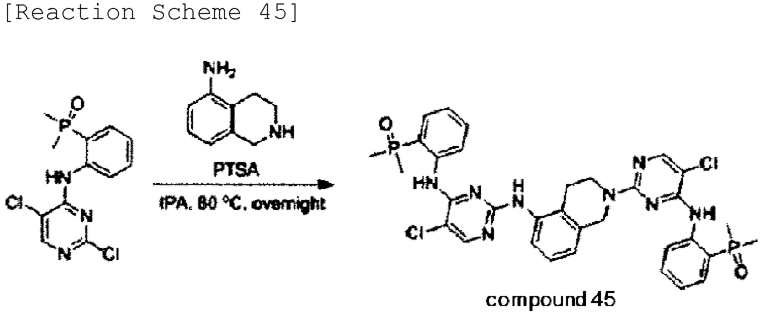

To a solution of 1,2,3,4-tetrahydroisoquinoline-5-amine (28 mg, 0.19 mmol) in IPA (0.6 mL) were added 2,5-dichloro-N-(2-(dimethylphosphoryl)phenyl)pyrimidine-4-amine (50 mg, 0.16 mmol) and then PTSA (36 mg, 0.19 mmol) . The reaction mixture was heated overnight at 80°C. TLC indicated the formation of a new spot. The reaction was quenched with sodium bicarbonate, followed by extraction with DCM (20 mL×2). The organic layer was dried over sodium sulfate and the solvent was removed by evaporation in a vacuum. The crude mixture was subjected to purification by MPLC using 5 % MeOH:DCM as an eluent to afford Compound 45 as a brown solid (9.0 mg, 0.0127 mmol, 8 %).

LCMS: 707.2 [M+H]⁺

LCMS: ¹H NMR (300 MHz, Chloroform-d) δ 11.12 (s, 1H), 10.81 (s, 1H), 8.65 (dd, J = 8.6, 4.4 Hz, 1H), 8.47 (dd, J = 8.6, 4.7 Hz, 1H), 8.06 (d, J = 9.8 Hz, 2H), 7.64-7.50 (m, 2H), 7.33 (dd, J = 7.8, 1.4 Hz, 1H), 7.26-7.05 (m, 5H), 7.05-6.97 (m, 1H), 6.81 (s, 1H), 4.95 (s, 2H), 4.00 (t, J = 5.9 Hz, 2H), 2.88 (t, J = 5.9 Hz, 2H), 1.86 (d, J = 9.8 Hz, 6H), 1.81 (d, J = 9.8 Hz, 6H).

### Compound 46. 1-(7-((5-Chloro-4-((2-(dimethylphosphoryl)phenyl)amino)pyrimidin-2-yl)amino)-3,4-dihydroisoquinolin-2(1H)-yl)-2,2,2-trifluoroethan-1-one

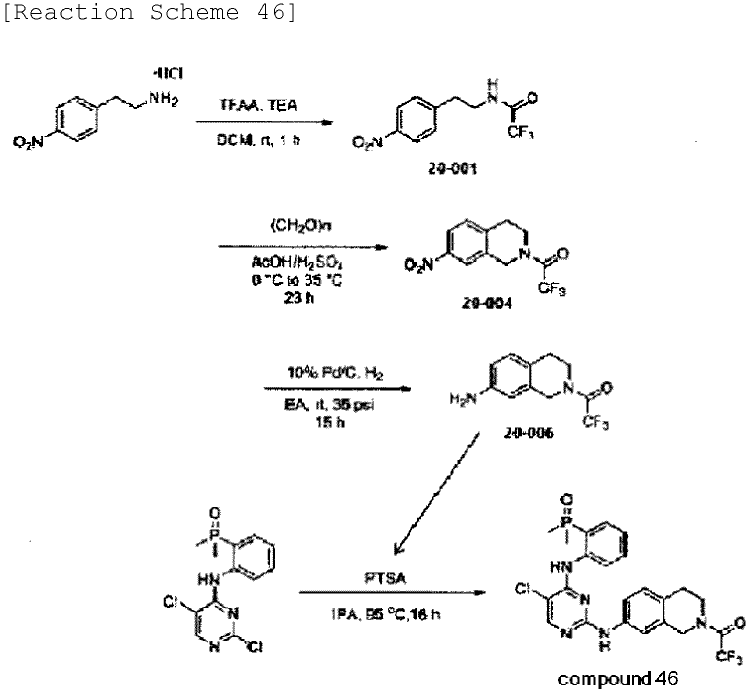

### 1) Synthesis of 2,2,2-trifluoro-N-(4-nitrophenethyl)acetamide

To a solution of 2-(4-nitrophenyl)ethan-1-amine (10.0 g, 49.5 mmol) in DCM (200 mL) was added TFAA (26.0 g, 124 mmol) at room temperature. Using a drip funnel, TEA (13.0 g, 124 mmol) was added to a cold bath, and then stirred at room temperature until completion of the reaction. TLC indicated the consumption of the starting material. The reaction was quenched with 30 mL of water, followed by extraction with DCM/water. The DCM layer was washed with 100 mL of 1 N HCl (aq.). Then, the DCM layer was dried over sodium sulfate and the solvent was evaporated in a vacuum to afford 20-001 as an ivory solid (14.0 g, 53.4 mmol, crude >100 %).

*Without purification, the following reactions were conducted.

¹H NMR (400 MHz, CDCl₃) δ 8.18 (d, J = 8.1 Hz, 2H), 7.37 (d, J = 8.1 Hz, 2H), 6.53 (s, br, 1H), 3.67 (q, J = 6.9 Hz, 2H), 3.02 (t, J = 7.1 Hz, 2H).

### 2) Synthesis of 2,2,2-trifluoro-1-(7-nitro-3,4-dihydroisoquinolin-2(1H)-yl)ethan-1-one

At 0°C, a 250-mL RB was filled with 50 mL of acetic acid and 75 mL of sulfuric acid. To the solution, 20-001 (13.0 g, 79.4 mmol) and paraformaldehyde (0.183 g, 6.11 mmol) were added. After the temperature was elevated to 30°C, the mixture was stirred until completion of the reaction. When the reaction was completed as monitored by TLC, the reaction mixture was carefully poured onto ice and diluted with water. Thereafter, extraction with EtOAc (250 mL×2) was conducted for the aqueous layer. The organic layer was dried over sodium sulfate and the solvent was evaporated in a vacuum to afford 20-004 as a white solid (11.0 g, 42.0 mmol, 75 %).

¹H NMR (400 MHz, CDCl₃) δ 8.08 (q, J = 9.0, 7.6 Hz, 2H), 7.44 - 7.29 (m, 1H), 4.88 (d, J = 17.8 Hz, 2H), 4.01 - 3.81 (m, 2H), 3.08 (t, J = 6.5 Hz, 2H).

### 3) Synthesis of 1-(7-amino-3,4-dihydroisoquinolin-2(1H)-yl)-2,2,2-trifluoroethan-1-one

To a solution of 20-004 (11.0 g, 40.1 mmol) in EA (50 mL) was added 10 % Pd/C (1.10 g) at 35 psi using a parr reactor. The Pd/C was removed by a celite filter and the filtrate was concentrated to afford 20-006 as an ivory solid (9.20 g, 37.7 mmol, 95 %).

¹H NMR (400 MHz, CDCl₃) δ 6.95 (t, J = 8.6 Hz, 1H), 6.57 (t, J = 8.7 Hz, 1H), 6.45 (d, J = 13.1 Hz, 1H), 4.66 (d, J = 22.0 Hz, 2H), 3.82 (d, J = 18.8 Hz, 2H), 3.64 (s, 2H), 2.84 (s, 2H).

### 4) Synthesis of 1-(7-((5-chloro-4-((2-(dimethylphosphoryl)phenyl)amino)pyrimidin-2-yl)amino)-3,4-dihydroisoquinolin-2(1H)-yl)-2,2,2-trifluoroethan-1-one

To a 7-mL vial were added 2,5-dichloro-N-(2-(dimethylphosphoryl)phenyl)pyrimidine-4-amine (100 mg, 0.316 mmol) and then 20-006 (81.2 mg, 0.332 mmol), PTSA (90.3 mg, 0.475 mmol), and IPA (1 mL). The reaction mixture was heated overnight at 95°C. The reaction was quenched with sodium bicarbonate, followed by extraction with DCM (50 mL×2). The organic layer was dried over sodium sulfate and the solvent was evaporated in a vacuum. The crude mixture was subjected to purification by MPLC using 5 % MeOH:DCM to afford Compound 46 as an ivory solid (58.6 mg, 37.7 mmol, 95 %).

¹H NMR (400 MHz, CDCl₃) δ 10.92 (d, J = 8.4 Hz, 1H), 8.54 (d, J = 7.3 Hz, 1H), 8.21-8.07 (m, 1H), 7.57 (dd, J = 26.9, 21.2 Hz, 2H), 7.34 (dd, J = 14.4, 7.9 Hz, 1H), 7.27-7.01 (m, 3H), 6.98 (s, 1H), 4.72 (d, J = 13.8 Hz, 2H), 3.99-3.75 (m, 2H), 2.95 (t, J = 6.9 Hz, 2H), 1.89 (d, J = 2.3 Hz, 3H), 1.85 (d, J = 2.4 Hz, 3H).

### Compound 47. (2-((5-Chloro-2-((1,2,3,4-tetrahydroisoquinolin-7-yl)amino)pyrimidin-4-yl)amino)phenyl)dimethylphosphine oxide

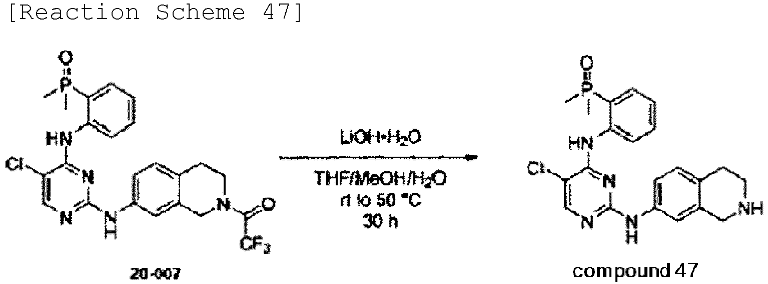

To a solution of 20-007 (46.6 mg, 0.0764 mmol) in THF/MeOH/water (2:1:0.5) (1 mL) was added LiOH.H₂O (24.1 mg, 0.572 mmol) at room temperature. After 30 hours, the solvent was evaporated using a rotary evaporator and EA/MC was added. The solid was removed using a syringe filter. The filtrate was treated with acid/base and purified by pulverization to afford Compound 47 as a white solid (11.0 mg, 0.0257 mmol, 34 %).

LC/MS: 426.19 [M+H]⁺

¹H NMR (400 MHz, CDCl₃) δ 10.87 (s, 1H), 8.58 (dt, J = 7.4, 3.4 Hz, 1H), 8.08 (d, J = 2.4 Hz, 1H), 7.48 (t, J = 8.1 Hz, 1H), 7.27 (d, J = 7.8 Hz, 2H), 7.24 (d, J = 7.9 Hz, 1H), 7.12 (t, J = 7.7 Hz, 1H), 7.08 - 6.94 (m, 2H), 3.97 (s, 2H), 3.15 (dt, J = 6.1, 3.7 Hz, 2H), 2.79 (d, J = 6.2 Hz, 2H), 1.85 (s, 1H), 1.82 (s, 3H).

### Compound 48. 1-(6-((5-Chloro-4-((2-(dimethylphosphoryl)phenyl)amino)pyrimidin-2-yl)amino)-3,4-dihydroisoquinolin-2(1H)-yl)-2,2,2-trifluoroethan-1-one

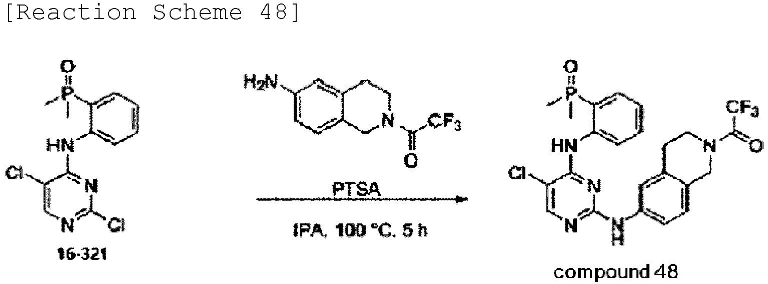

To a 5-mL vial were added 2,5-dichloro-N-(2-(dimethylphosphoryl)phenyl)pyrimidine-4-amine (50.0 mg, 0.316 mmol) and then 16-321 (40.6 mg, 0.166 mmol), PTSA (45.1 mg, 0.237 mmol) and IPA (0.5 mL). The reaction mixture was heated at 100°C for 5 hours. The reaction was quenched with sodium bicarbonate, followed by extraction with DCM (50 mL×2) and water. The organic layer was dried over sodium sulfate and the solvent was evaporated in a vacuum. The mixture was subjected to purification by MPLC using 4% to 9% MeOH in MC (gradient) to afford Compound 48 as a white and pale pink solid (35.8 mg, 0.06835 mmol, 43 %).

¹H NMR (400 MHz, CDCl₃) δ 10.98 (s, 1H), 8.67-8.53 (m, 1H), 8.14 (d, J = 0.8 Hz, 1H), 7.55-7.44 (m, 2H), 7.43-7.29 (m, 2H), 7.17 (dddd, J = 8.6, 7.5, 2.3, 1.1 Hz, 1H), 7.09 (dd, J = 14.8, 8.3 Hz, 1H), 6.98 (d, J = 7.3 Hz, 1H), 4.76 (d, J = 18.4 Hz, 2H), 3.96-3.81 (m, 2H), 3.00-2.86 (m, 2H), 1.89 (s, 3H), 1.84 (s, 3H)

### Compound 49. (2-((5-Chloro-2-((1,2,3,4-tetrahydroisoquinolin-6-yl)amino)pyrimidin-4-yl)amino)phenyl)dimethylphosphine oxide

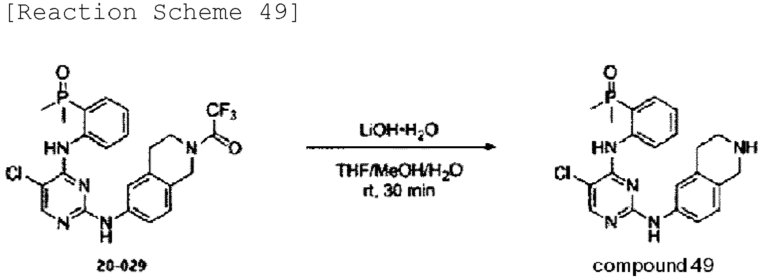

To a solution of 20-029 (7.0 mg, 0.0134 mmol) in THF/MeOH/water (2:1:0.5) (1 mL) was added LiOH.H₂O (2.0 mg, 0.0477 mmol) at room temperature. After 30 minutes, the solvent was evaporated using a rotary evaporator, and the residue was treated with 1N HCl and NaOH to afford Compound 49 as a white solid (5.5 mg, 0.0105 mmol, 96 %) .

¹H NMR (400 MHz, CDCl₃) δ 10.90 (s, 1H), 8.65-8.57 (m, 1H), 8.09 (s, 1H), 7.51-7.44 (m, 1H), 7.34-7.27 (m, 3H), 7.16-7.08 (m, 1H), 6.95 (d, J = 8.1 Hz, 1H), 6.85 (s, 1H), 4.00 (s, 2H), 3.14 (t, J = 5.9 Hz, 2H), 2.76 (t, J = 6.0 Hz, 2H), 1.87 (s, 3H), 1.84 (s, 3H).

### Compound 50. 1-(7-((5-Chloro-4-((2-(dimethylphosphoryl)phenyl)amino)pyrimidin-2-yl)amino)-4,4-dimethyl-3,4-dihydroisoquinolin-2(1H)-yl)-2,2,2-trifluoroethan-1-one

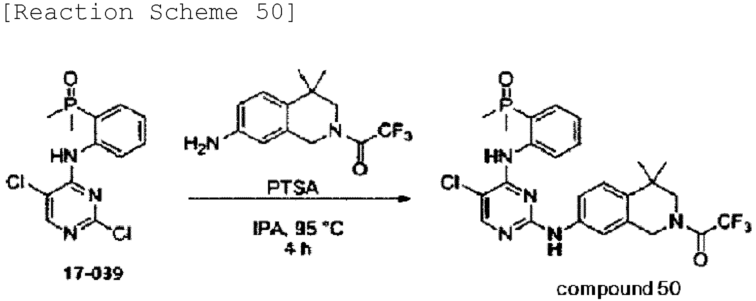

To a 7-mL vial were added 2,5-dichloro-N-(2-(dimethylphosphoryl)phenyl)pyrimidine-4-amine (47.0 mg, 0.149 mmol) and then 17-039 (42.1 mg, 0.156 mmol), PTSA (54.2 mg, 0.285 mmol), and IPA (0.5 mL). The reaction mixture was heated at 95°C for 4 hours. The reaction was quenched with sodium bicarbonate, followed by extraction with DCM (50 mL×2) and water. The organic layer was dried over sodium sulfate and the solvent was evaporated in a vacuum. The mixture was subjected to purification by MPLC using 3% to 7% MeOH in DCM (gradient) to afford Compound 50 as a white solid (35.8 mg, 0.0648 mmol, 44 %) .

LC/MS: 552.6[M+H+]

¹H NMR (400 MHz, CDCl₃) δ 10.90 (d, J = 15.7 Hz, 1H), 8.59-8.43 (m, 1H), 8.11 (d, J = 3.6 Hz, 1H), 7.65-7.41 (m, 2H), 7.37-7.27 (m, 2H), 7.26-7.12 (m, 2H), 6.97 (d, J = 4.0 Hz, 1H), 4.72 (d, J = 4.0 Hz, 2H), 3.67 (s, 1H), 3.59-3.48 (m, 1H), 1.88 (s, 3H), 1.83 (s, 3H), 1.30 (d, J = 2.9 Hz, 6H) .

**Compound 51. (2-((5-Chloro-2-((1-methyl-1,2,3,4-tetrahydroisoquinolin-7-yl)amino)pyrimidin-4-yl)amino)phenyl)dimethylphosphine oxide**

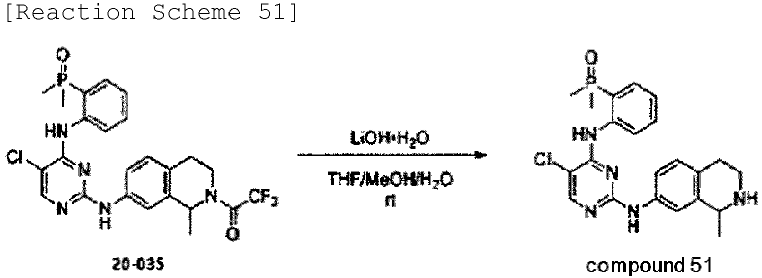

To a solution of 20-035 (20.0 mg, 0.0370 mmol) in THF/MeOH/water (2:1:0.5) (0.5 mL) was added LiOH.H₂O (5.55 mg, 0.132 mmol) at room temperature. After 30 minutes, the solvent was evaporated using a rotary evaporator, and the residue was treated with 1N HCl and NaOH to afford Compound 51 as a white solid (11.3 mg, 0.0210 mmol, 69 %).

LC/MS: 442.5[M+H+]

¹H NMR (400 MHz, CDCl₃) δ 10.87 (s, 1H), 8.61-8.56 (m, 1H), 8.10 (s, 1H), 7.51-7.45 (m, 1H), 7.35 (dd, J = 8.2, 2.3 Hz, 1H), 7.30 (td, J = 7.2, 6.7, 2.0 Hz, 2H), 7.26 (s, 1H), 7.12 (tdd, J = 7.6, 2.4, 1.1 Hz, 1H), 7.03 (d, J = 8.2 Hz, 1H), 6.86 (s, 1H), 4.08 (q, J = 6.6 Hz, 1H), 3.28 (dt, J = 12.6, 5.0 Hz, 1H), 3.02 (ddd, J = 12.9, 8.9, 4.7 Hz, 1H), 2.89-2.78 (m, 1H), 2.71 (dt, J = 16.1, 4.6 Hz, 1H), 1.84 (dd, J = 13.1, 5.8 Hz, 6H), 1.37 (d, J = 6.6 Hz, 3H).

**Compound 52. (2-((5-Chloro-2-((4,4-dimethyl-1,2,3,4-tetrahydroisoquinolin-7-yl)amino)pyrimidin-4-yl)amino)phenyl)dimethylphosphine oxide**

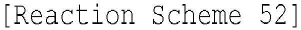

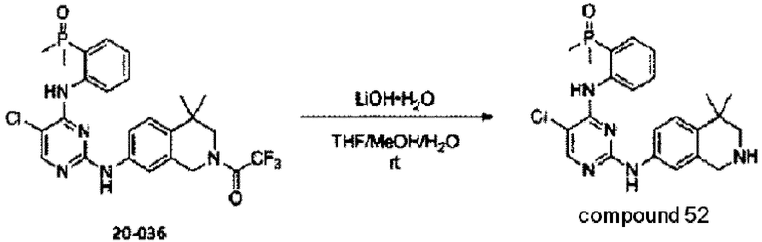

To a solution of 20-036 (10.0 mg, 0.0181 mmol) in THF/MeOH/water (2:1:0.5) (0.5 mL) was added LiOH.H₂O (2.71 mg, 0.0645 mmol) at room temperature. After 30 minutes, the solvent was evaporated using a rotary evaporator, and the residue was treated with 1N HCl and NaOH to afford Compound 52 as a white solid (7.78 mg, 0.0171 mmol, 94 %).

LC/MS: 456.6[M+H+]

¹H NMR (400 MHz, CDCl₃) δ 10.89 (s, 1H), 8.59 (ddd, J = 8.5, 4.4, 1.1 Hz, 1H), 8.09 (d, J = 3.8 Hz, 1H), 7.48 (dd, J = 8.6, 7.2 Hz, 1H), 7.33-7.27 (m, 1H), 7.25 (d, J = 1.5 Hz, 2H), 7.17-7.08 (m, 1H), 6.90 (s, 1H), 3.96 (s, 2H), 2.86 (s, 2H), 1.85 (s, 3H), 1.82 (s, 3H) 1.27 (s, 6H).

### Compound 53. 1-(7-((5-Chloro-4-((2-(dimethylphosphoryl)phenyl)amino)pyrimidin-2-yl)amino)-1-isopropyl-3,4-dihydroisoquinolin-2(1H)-yl)-2,2,2-trifluoroethan-1-one

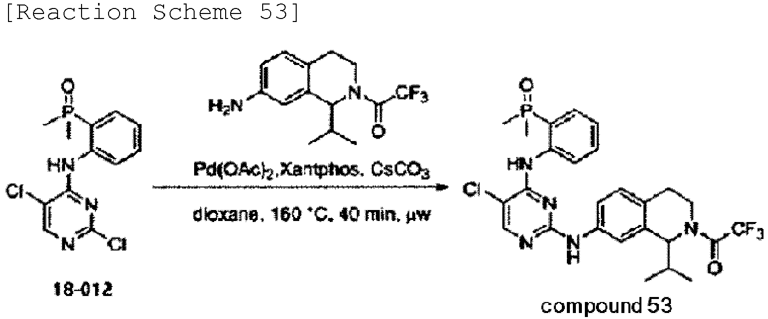

To a solution of 18-012 (45 mg, 0.19 mmol) in dioxane (1mL) were added 2,5-dichloro-N-(2-(dimethylphosphoryl)phenyl)pyrimidine-4-amine (50 mg, 0.16 mmol) and then Pd(OAc)₂ (4.0 mg, 0.016 mmol), xantphos (9.2 mg, 0.016 mmol), and cesium carbonate (104 mg, 0.32 mmol). The reaction mixture was heated 160°C for 40 minutes with microwaves. The resulting mixture was filtered on a celite bed and washed with MeOH, and the solvent was evaporated in a vacuum. The residue was subjected to purification by MPLC using 5 % MeOH in MC to afford Compound 53 as a yellowish green solid (14.8 mg, 0.0262 mmol, 16 %).

LC/MS: 566.7[M+H+]

¹H NMR (300 MHz, DMSO) δ 11.02 (s, 1H), 9.46 (s, 1H), 8.30 (s, 1H), 8.19 (d, J = 2.3 Hz, 1H), 7.69-7.55 (m, 3H), 7.46 (d, J = 9.4 Hz, 1H), 7.21 (s, 1H), 7.06 (d, J = 8.4 Hz, 1H), 4.93 (d, J = 9.5 Hz, 1H), 3.97-3.68 (m, 2H), 2.90 (d, J = 6.8 Hz, 2H), 1.92-1.58 (m, 6H), 0.89-0.74 (m, 6H).

### Compound 54. 1-(7-((5-Chloro-4-((2-(dimethylphosphoryl)phenyl)amino)pyrimidin-2-yl)amino)-1-methyl-3,4-dihydroisoquinolin-2(1H)-yl)-2,2,2-trifluoroethan-1-one

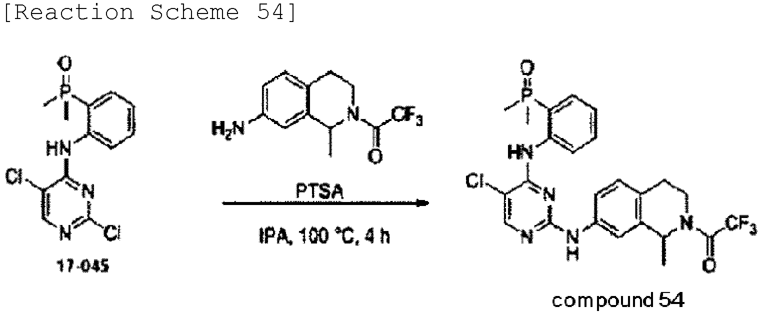

To a 5-mL vial were added 2,5-dichloro-N-(2-(dimethylphosphoryl)phenyl)pyrimidine-4-amine (50.0 mg, 0.316 mmol) and then 17-045 (42.9 mg, 0.166 mmol), PTSA (54.2 mg, 0.285 mmol), and IPA (0.5 mL). The reaction mixture was heated at 100°C for 4 hours. The reaction was quenched with sodium bicarbonate, followed by extraction with DCM (50 mL×2) and water. The organic layer was dried over sodium sulfate and the solvent was evaporated in a vacuum. The mixture was subjected to purification by MPLC using 3 % to 7 % MeOH in DCM (gradient) to afford Compound 54 as a white solid (63.6 mg, 0.118 mmol, 75 %).

¹H NMR (300 MHz, CDCl₃) δ 10.83 (s, 1H), 8.49 (dd, J = 8.4, 4.4 Hz, 1H), 8.11 (s, 1H), 7.64-7.41 (m, 2H), 7.37-7.27 (m, 1H), 7.25 -7.11 (m, 2H), 7.06 (dd, J = 8.4, 4.4 Hz, 1H), 6.93 (s, 1H), 5.50 (q, J = 6.8 Hz, 1H), 4.13-3.98 (m, 1H), 3.67-3.46 (m, 1H), 3.09-2.90 (m, 1H), 2.81 (d, J = 16.2 Hz, 1H), 1.93-1.74 (m, 6H), 1.39 (d, J = 6.6 Hz, 3H).

### Compound 55. (2-((5-Chloro-2-((1-isopropyl-1,2,3,4-tetrahydroisoquinolin-7-yl)amino)pyrimidin-4-yl)amino)phenyl)dimethylphosphine oxide

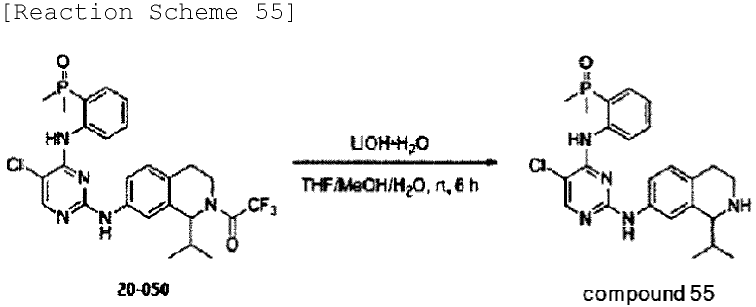

To a solution of 20-050 (9.7 mg, 0.0177 mmol) in THF/MeOH/water (2:1:0.5) (1 mL) was added LiOH.H₂O (2.6 mg, 2.55 mmol) at room temperature. After 6 hours, the solvent was evaporated using a rotary evaporator, and the residue was treated with 1N HCl and NaOH to afford Compound 55 as a yellow solid (1.9 mg, 0.00213 mmol, 14 %).

LC/MS: 468.95 [M + H +]

¹H NMR (400 MHz, CDCl₃) δ 10.87 (s, 1H), 8.58 (dd, J = 8.6, 4.4 Hz, 1H), 8.10 (s, 1H), 7.51-7.44 (m, 1H), 7.38-7.27 (m, 3H), 7.12 (tdd, J = 7.6, 2.4, 1.1 Hz, 1H), 7.02 (d, J = 8.2 Hz, 1H), 6.85 (s, 1H), 3.90 (d, J = 3.7 Hz, 1H), 3.30 (ddd, J = 11.9, 5.2, 3.1 Hz, 1H), 2.92 (ddd, J = 11.9, 10.3, 4.0 Hz, 1H), 2.86 - 2.74 (m, 1H), 2.65 (d, J = 15.7 Hz, 1H), 2.20 (ddd, J = 9.5, 6.7, 3.2 Hz, 1H), 1.84 (dd, J = 13.1, 8.1 Hz, 6H), 1.05 (d, J = 6.9 Hz, 3H), 0.71 (d, J = 6.8 Hz, 3H).

### Compound 56. 2,2,2-trifluoro-1-(7-((5-fluoro-4-((1-(methylsulfonyl)piperidin-4-yl)amino)pyrimidin-2-yl)amino)-3,4-dihydroisoquinolin-2(1H)-yl)ethan-1-one

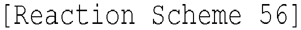

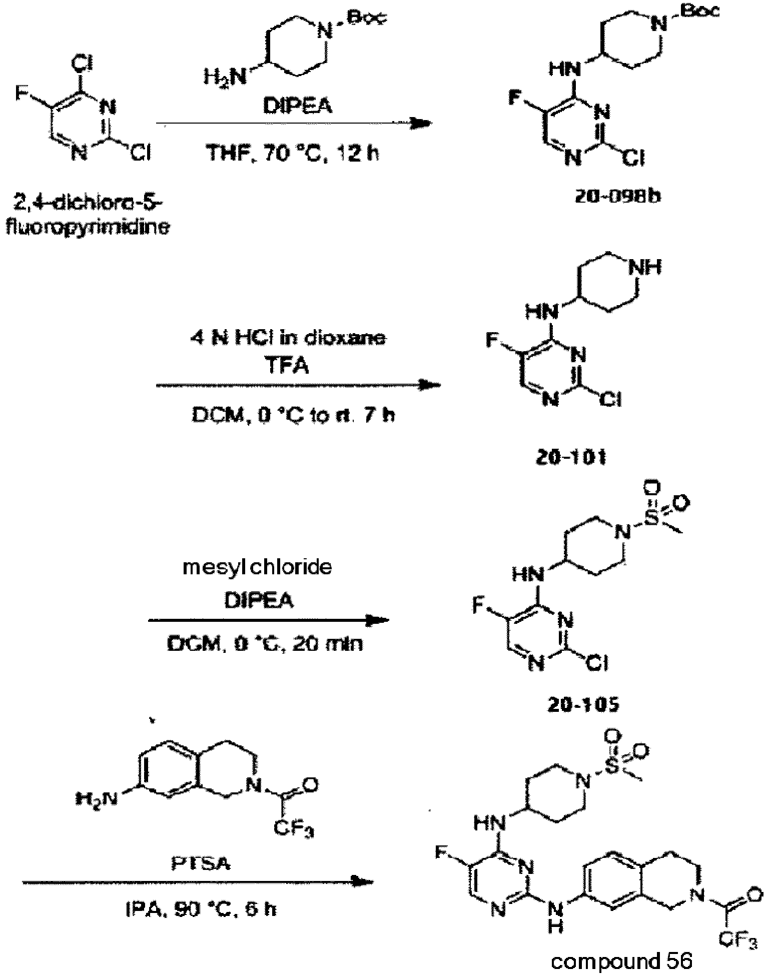

### 1) Tert-butyl 4-((2-chloro-5-fluoropyrimidin-4-yl)amino)piperidine-1-carboxylate

In THF (30 mL) were dissolved 2,4-dichloro-5-fluoropyrimidine (800 mg, 4.80 mmol), tert-butyl4-aminopiperidine-1-carboxylate (996 mg, 4.80 mmol), and DIPEA (948 µL, 5.76 mmol). The reaction mixture was heated overnight under reflux. After the reaction mixture was cooled, separation was made with water/EA. The EA layer was dried over sodium sulfate and the solvent was removed using a rotary evaporator. The reaction mixture was subjected to purification by silica gel column chromatography using 30 % EA in Hex to afford 20-098b as a white solid (1.33 g, 4.02 mmol).

¹H NMR (400 MHz, CDCl₃) δ 7.81 (d, J = 2.8 Hz, 1H), 5.64 (dd, J = 8.0, 2.2 Hz, 1H), 4.13 (ddq, J = 11.0, 7.2, 3.8 Hz, 1H), 4.13- 4.01 (m, 2H), 2.87 (t, J = 12.9 Hz, 2H), 2.10-1.90 (m, 2H), 1.48-1.43 (m, 1H), 1.41 (s, 9H), 1.39 (s, 1H).

### 2) 2-Chloro-5-fluoro-N-(piperidin-4-yl)pyrimidin-4-amine

To a solution of 20-098b (600 mg, 1.81 mmol) in DCM (20 mL) was added 4 N HCl in dioxane (1.80 mL, 3.62 mmol) at 0°C. The resulting mixture was stirred at room temperature for 6 hours and then, together with TFA (4 mL), for an additional 1 hour. After the solvent was evaporated, extraction was conducted with DCM. The aqueous layer was alkalized with sodium bicarbonate, followed by extraction with DCM. The organic layers were pooled and dried over Na₂SO₄, and the solvent was removed in a vacuum to afford 20-101 as a white solid (415 mg, 1.80 mmol, 98 %).

¹H NMR (400 MHz, CDCl₃) δ 7.87 (d, J = 2.8 Hz, 1H), 5.15-4.97 (m, 1H), 4.19-4.05 (m, 1H), 3.12 (dt, J = 13.3, 4.1 Hz, 2H), 2.78 (ddd, J = 12.7, 11.4, 2.6 Hz, 2H), 2.11-2.02 (m, 2H), 1.42 (dtd, J = 12.5, 11.2, 4.0 Hz, 2H).

### 3) 2-Chloro-5-fluoro-N-(1-(methylsulfonyl)piperidin-4-yl)pyrimidin-4-amine

In DCM (50 mL) was dissolved 20-101 (360 mg, 1.56 mmol) which was then cooled to 0°C. The solution was added with DIPEA (2.69 mL, 15.6 mmol) and stirred for 10 minutes. After 10 minutes, mesyl chloride (241 µL, 3.12 mmol) was added, warmed to room temperature, and stirred for 20 minutes. Extraction with DCM (100 mL×2) was conducted for the reaction mixture, followed by washing with brine (50 mL). The organic layer thus pooled was dried over Na₂SO₄ and the solvent was removed by evaporation in a vacuum. The reaction mixture was pulverized with EA and hex and purified to afford 20-105 as a yellow solid (418 mg, 1.35 mmol, 87 %).

¹H NMR (500 MHz, CDCl₃) δ 7.93 (d, J = 2.7 Hz, 1H), 5.24 (d, J = 8.0 Hz, 1H), 4.19 (tdt, J = 11.7, 8.2, 4.2 Hz, 1H), 3.94-3.83 (m, 2H), 2.92 (td, J = 12.2, 2.6 Hz, 2H), 2.25-2.17 (m, 2H), 1.70 (dtd, J = 12.9, 11.5, 4.2 Hz, 2H).

### 4) 2,2,2-Trifluoro-1-(7-((5-fluoro-4-((1-(methylsulfonyl)piperidin-4-yl)amino)pyrimidin-2-yl)amino)-3,4-dihydroisoquinolin-2(1H)-yl)ethan-1-one

To a solution of 20-105 (50.0 mg, 0.162 mmol) in IPA (1 mL) were added HYP-19 (41.5 mg, 0.170 mmol) and PTSA (55.4 mg, 0.243 mmol). The reaction mixture was heated at 90°C for 6 hours. The solid was filtered and alkalinized with sodium bicarbonate, followed by extraction with DCM. The reaction mixture was subjected to purification by silica gel column chromatography using 5% MeOH in DCM to afford 56 as a white solid (38.8 mg, 0.0751 mmol, 46 %).

LC/MS: 518.27 [M + H+]

¹H NMR (400 MHz, DMSO) δ 9.09 (d, J = 11.7 Hz, 1H), 7.90 (d, J = 3.7 Hz, 1H), 7.72 (d, J = 55.9 Hz, 1H), 7.50-7.33 (m, 2H), 7.08 (t, J = 8.0 Hz, 1H), 4.72 (s, 2H), 4.11-3.99 (m, 1H), 3.81 (s, 2H), 3.65 (d, J = 11.7 Hz, 2H), 2.91 (d, J = 6.7 Hz, 3H), 2.84 (d, J = 10.8 Hz, 4H), 2.02 (d, J = 12.0 Hz, 2H), 1.67 (tt, J = 12.3, 6.3 Hz, 2H).

### Compound 57. 5-fluoro-N⁴-(1-(methylsulfonyl)piperidin-4-yl)-N²-(1,2,3,4-tetrahydroisoquinolin-7-yl)pyrimidine-2,4-diamine

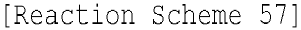

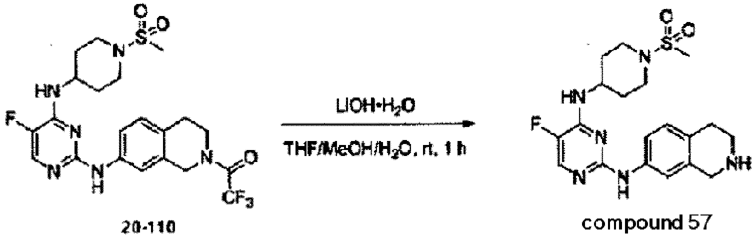

To a solution of 20-110 (27.0 mg, 0.0523 mmol) in THF/MeOH/water (2:1:0.5) (7 mL) was added LiOH.H₂O (7.68 mg, 0.183 mmol) at room temperature. After 30 minutes, the solvent was evaporated using a rotary evaporator, and the residue was treated with 1N HCl and NaOH to afford Compound 57 as a white solid (22.0 mg, 0.0523 mmol, 99 %).

LC/MS: 421.33 [M + H+]

¹H NMR (500 MHz, CDCl₃) δ 7.83 (d, J = 3.2 Hz, 1H), 7.27 (s, 1H), 7.04 (d, J = 8.0 Hz, 1H), 6.74 (s, 1H), 4.83 (d, J = 7.5 Hz, 1H), 4.15-4.07 (m, 1H), 4.03 (s, 2H), 3.86 (d, J = 12.5 Hz, 2H), 3.17 (t, J = 6.0 Hz, 2H), 2.94 (td, J = 12.0, 2.7 Hz, 2H), 2.87 (s, 3H), 2.78 (t, J = 6.0 Hz, 2H), 2.24 (d, J = 13.6 Hz, 2H), 1.76-1.66 (m, 2H).

### Compound 58. 2,2,2-Trifluoro-1-(7-((5-fluoro-4-((1-(methylsulfonyl)piperidin-4-yl)amino)pyrimidin-2-yl)amino)-1-isopropyl-3,4-dihydroisoquinolin-2(1H)-yl)ethan-1-one

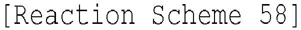

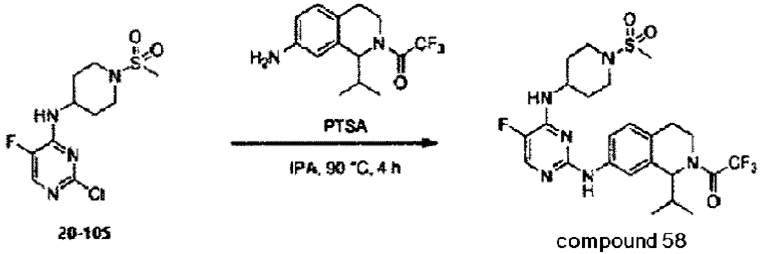

To a solution of 20-105 (50.0 mg, 0.162 mmol) in IPA (1 mL) were added GM-17 (48.7 mg, 0.170 mmol) and then PTSA (46.2 mg, 0.243 mmol). The reaction mixture was heated at 90°C for 4 hours. The reaction mixture was alkalinized with sodium bicarbonate, followed by extraction with DCM. The reaction mixture was subjected to purification by silica gel column chromatography using 5% MeOH in DCM to afford 58 as a white solid (47.9 mg, 0.0236 mmol, 54 %).

LC/MS: 560.33 [M + H+]

¹H NMR (400 MHz, CDCl₃) δ 7.81 (d, J = 3.2 Hz, 1H), 7.73 (d, J = 2.1 Hz, 1H), 7.12-7.03 (m, 2H), 6.84 (s, 1H), 5.18 (d, J = 9.3 Hz, 1H), 4.85 (d, J = 7.7 Hz, 1H), 4.13 (tdt, J = 11.6, 8.2, 4.2 Hz, 1H), 4.05-3.89 (m, 2H), 3.88-3.74 (m, 2H), 3.07 (td, J = 11.9, 2.6 Hz, 1H), 3.00-2.94 (m, 2H), 2.92 (d, J = 2.7 Hz, 1H), 2.91 (s, 3H), 2.29-2.17 (m, 3H), 2.14 - 2.03 (m, 1H), 1.76-1.62 (m, 2H), 1.04 (d, J = 6.6 Hz, 3H), 0.98 (d, J = 6.8 Hz, 3H) .

### Compound 59. 2,2,2-trifluoro-1-(7-((5-fluoro-4-((1-(methylsulfonyl)piperidin-4-yl)amino)pyrimidin-2-yl)amino)-1-methyl-3,4-dihydroisoquinolin-2(1H)-yl)ethan-1-one

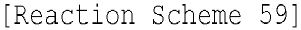

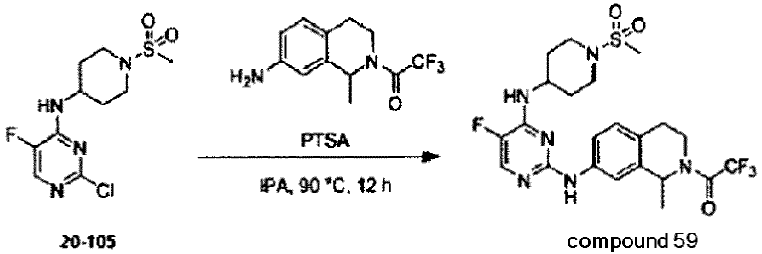

To a solution of 20-105 (50.0 mg, 0.162 mmol) in IPA (1 mL) were added GM-17 (43.9 mg, 0.170 mmol) and PTSA (46.2 mg, 0.243 mmol). The reaction mixture was heated 90°C for 12 hours. The reaction mixture was alkalinized with sodium bicarbonate, followed by extraction with DCM. The reaction mixture was subjected to purification by silica gel column chromatography using 5% MeOH in DCM to afford 59 as a white solid (63.2 mg, 0.119 mmol, 74 %).

LC/MS: 531.31 [M + H+]

¹H NMR (400 MHz, CDCl₃) δ 7.82 (dd, J = 4.9, 3.2 Hz, 1H), 7.69 (d, J = 2.2 Hz, 1H), 7.11 (dd, J = 8.3, 2.3 Hz, 1H), 7.04 (d, J = 8.2 Hz, 1H), 6.80 (d, J = 18.4 Hz, 1H), 5.55 (q, J = 6.9 Hz, 1H), 4.86 (d, J = 7.6 Hz, 1H), 4.19-4.09 (m, 1H), 4.09-4.01 (m, 1H), 3.89 (dd, J = 21.3, 12.5 Hz, 3H), 3.68-3.55 (m, 1H), 3.04-2.93 (m, 2H), 2.91 (s, 3H), 2.89-2.78 (m, 3H), 2.23 (t, J = 16.5 Hz, 2H), 1.71 (ddt, J = 19.1, 12.8, 6.0 Hz, 2H), 1.54 (d, J = 6.9 Hz, 3H).

### Compound 60. 5-fluoro-N²-(1-isopropyl-l,2,3,4-tetrahydroisoquinolin-7-yl)-N⁴-(1-(methylsulfonyl)piperidin-4-yl)pyrimidine-2,4-diamine

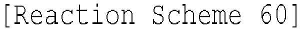

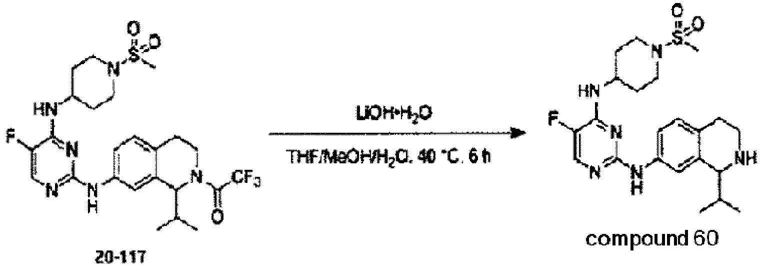

To a solution of 20-117 (27.0 mg, 0.0523 mmol) in THF/MeOH/water (2:1:0.5) (7 mL) was added LiOH.H₂O (7.68 mg, 0.183 mmol) at room temperature. After 30 minutes, the solvent was evaporated using a rotary evaporator, and the residue was treated with 1N HCl and NaOH to afford Compound 60 as a white solid (19.4 mg, 0.0523 mmol, 78 %).

LC/MS: 464.33 [M + H+]

¹H NMR (400 MHz, CDCl₃) δ 7.80 (d, J = 3.2 Hz, 1H), 7.35 (dd, J = 8.3, 2.3 Hz, 1H), 7.28 (d, J = 2.3 Hz, 1H), 7.00 (d, J = 8.2 Hz, 1H), 6.83 (s, 1H), 4.85 (dd, J = 7.9, 2.2 Hz, 1H), 4.09 (dt, J = 7.3, 3.8 Hz, 1H), 3.93 (d, J = 3.7 Hz, 1H), 3.87-3.77 (m, 2H), 3.29 (ddd, J = 11.9, 5.2, 3.1 Hz, 1H), 2.91 (dddd, J = 11.8, 10.2, 5.7, 3.2 Hz, 3H), 2.78 (td, J = 10.3, 3.7 Hz, 1H), 2.63 (dt, J = 15.7, 3.5 Hz, 1H), 2.32 (td, J = 6.9, 3.8 Hz, 1H), 2.19 (dt, J = 11.3, 3.4 Hz, 2H), 1.68-1.58 (m, 2H), 1.13 (d, J = 6.9 Hz, 3H), 0.76 (d, J = 6.7 Hz, 3H).

### Compound 61. 5-fluoro-N²-(1-methyl-1,2,3,4-tetrahydroisoquinolin-7-yl)-N⁴-(1-(methylsulfonyl)piperidin-4-yl)pyrimidine-2,4-diamine

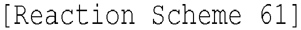

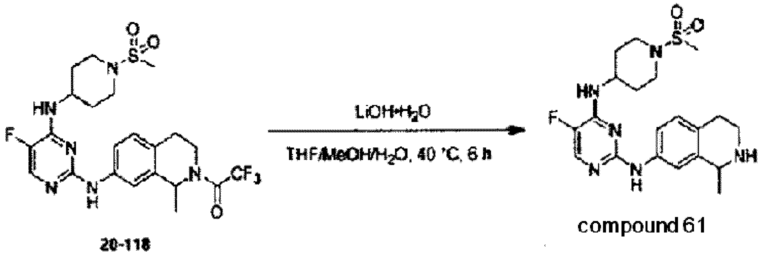

To a solution of 20-118 (28.0 mg, 0.0528 mmol) in THF/MeOH/water (2:1:0.5) (7 mL) was added LiOH.H₂O (7.36 mg, 0.185 mmol) at room temperature. After 1 hour, the solvent was evaporated using a rotary evaporator, and the residue was treated with 1N HCl and NaOH to afford Compound 61 as a white solid (17.6 mg, 0.0528 mmol, 80 %).

LC/MS: 435.31 [M + H+]

¹H NMR (500 MHz, DMSO) δ 8.87 (s, 1H), 7.88 (d, J = 3.7 Hz, 1H), 7.48 (d, J = 2.2 Hz, 1H), 7.43 (dd, J = 8.3, 2.2 Hz, 1H), 7.35 (d, J = 7.9 Hz, 1H), 6.92 (d, J = 8.3 Hz, 1H), 4.06 (d, J = 7.4 Hz, 1H), 3.90 (d, J = 6.8 Hz, 1H), 3.67-3.59 (m, 2H), 3.39 (d, J = 7.0 Hz, 2H), 3.06 (dt, J = 10.4, 5.0 Hz, 1H), 2.92 (s, 3H), 2.83 (d, J = 12.0 Hz, 2H), 2.76 (d, J = 4.4 Hz, 1H), 2.70-2.63 (m, 1H), 2.56 (s, 1H), 1.99 (d, J = 12.5 Hz, 2H), 1.66 (t, J = 6.2 Hz, 2H), 1.33 (d, J = 6.6 Hz, 3H).

### Compound 62. 1-(7-((5-chloro-4-((2-(dimethylphosphoryl)phenyl)amino)pyrimidin-2-yl)amino)-6-methoxy-3,4-dihydroisoquinolin-2(1H)-yl)-2,2,2-trifluoroethan-1-one

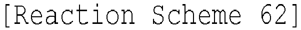

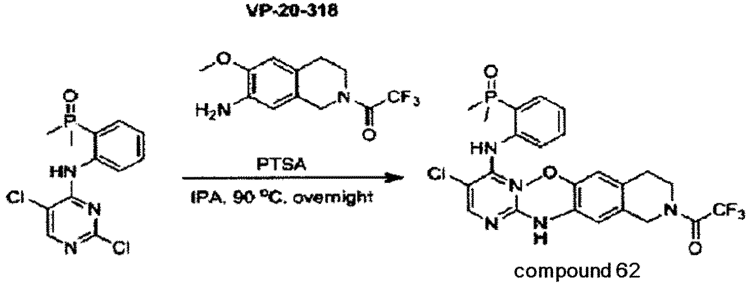

To a solution of 2,5-dichloro-N-(2-(dimethylphosphoryl)phenyl)pyrimidine-4-amine (30.0 mg, 0.0949 mmol) in IPA (2.0 mL) were added 20-318 (26.0 mg, 0.0949 mmol) and then PTSA (36.0 mg, 0.189 mmol). The reaction mixture was heated overnight at 90°C. TLC indicated the formation of a new spot. The solid was filtered, dissolved in water, alkalinized with sodium bicarbonate (pH 7-8), and washed with 10 ml of MC. After washing with DCM (20 mL×2), the organic layer was dried. The sodium sulfate and solvent were removed by evaporation in a vacuum, and the residue was subjected to purification by MPLC using 5% MeOH:MC to afford Compound 62 as a gray, softy solid (28.0 mg, 0.0505 mmol, 53 %).

¹H NMR (500 MHz, Chloroform-d) δ 10.79 (d, J = 7.4 Hz, 1H), 8.49 (dd, J = 8.3, 4.1 Hz, 1H), 8.21 (s, 1H), 8.19-8.16 (m, 1H), 8.15 (s, 1H), 7.68-7.59 (m, 2H), 7.59-7.53 (m, 1H), 7.39-7.33 (m, 1H), 6.68-6.63 (m, 1H), 4.66-4.57 (m, 2H), 3.93-3.83 (m, 5H), 2.96-2.88 (m, 2H), 1.88 (d, J = 1.7 Hz, 3H), 1.86 (d, J = 1.7 Hz, 3H).

### Compound 63. (2-((5-Chloro-2-((6-methoxy-1,2,3,4-tetrahydroisoquinolin-7-yl)amino)pyrimidin-4-yl)amino)phenyl)dimethylphosphine oxide

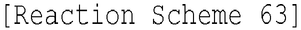

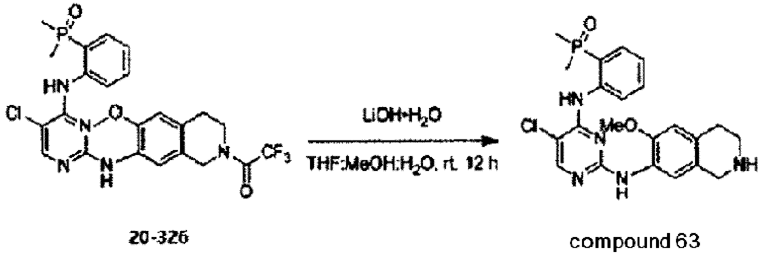

To a solution of 20-326 (28.0 mg, 0.0505 mmol) in THF:MeOH:H2O (2:10:0.5) was added lithium hydroxide monohydrate (5.4 mg, 0.126 mmol) at room temperature, followed by stirring for 12 hours. After the reaction was completed as monitored by TLC, the solvent was evaporated. The residue was acidified with 1 N HCl (aq) and washed with MC (20 mL×2). The aqueous liquid was alkalinized with solid sodium bicarbonate before extraction with DCM (20 mL×2). The organic layers thus obtained were pooled, dried over sodium sulfate, and filtered. Evaporation of the solvent in a vacuum afforded Compound 63 as a whitish brown solid (16 mg, 0.0349 mmol, 9 %).

LCMS: 458.2 [M+H]⁺

¹H NMR (500 MHz, Chloroform-d) δ 10.79 (s, 1H), 8.58 (dd, J = 8.4, 4.3 Hz, 1H), 8.13 (s, 1H), 7.99 (s, 1H), 7.59-7.51 (m, 2H), 7.32 (ddd, J = 14.0, 7.7, 1.6 Hz, 1H), 7.20-7.13 (m, 1H), 6.61 (s, 1H), 3.90 (d, J = 3.2 Hz, 2H), 3.89 (s, 2H), 3.18-3.11 (m, 2H), 2.77 (t, J = 6.0 Hz, 2H), 2.20 (s, 1H), 1.86 (d, J = 13.2 Hz, 6H).

### Compound 64. (2-((2-((1H-Indazol-6-yl)amino)-5-chloropyrimidin-4-yl)amino)phenyl)dimethylphosphine oxide

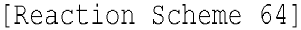

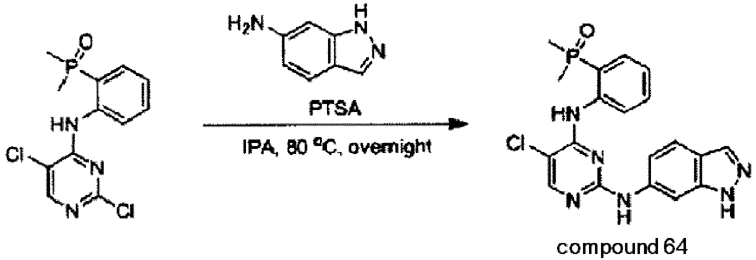

To a solution of 1H-indazol-6-amine (26 mg, 0.19 mmol) in IPA (0.6 mL) were added 2,5-dichloro-N-(2-(dimethylphosphoryl)phenyl)pyrimidine-4-amine (50 mg, 0.16 mmol) and then PTSA (30 mg, 0.16 mmol). The crude mixture was subjected to purification by silica gel column chromatography using EA:MeOH (9.5/0.5) to afford compound 64 as a solid (22 mg, 0.053 mmol, 33 %).

LC/MS (ESI) m/z 413.5 [M + H]

¹H NMR (300 MHz, DMSO-d6) δ 12.79 (s, 1H), 11.28 (s, 1H), 9.56 (s, 1H), 8.78-8.68 (m, 1H), 8.25 (s, 1H), 8.03 (bs, 1H), 7.94 (bs, 1H), 7.66-7.48 (m, 3H), 3.32 (dd, J = 9.0 Hz, 1H), 7.18 (t, J = 9.0 Hz, 1H), 1.82 (s, 3H), 1.78 (s, 3H)

### Compound 65. (2-((2-((1H-Indol-5-yl)amino)-5-chloropyrimidin-4-yl)amino)phenyl)dimethylphosphine oxide

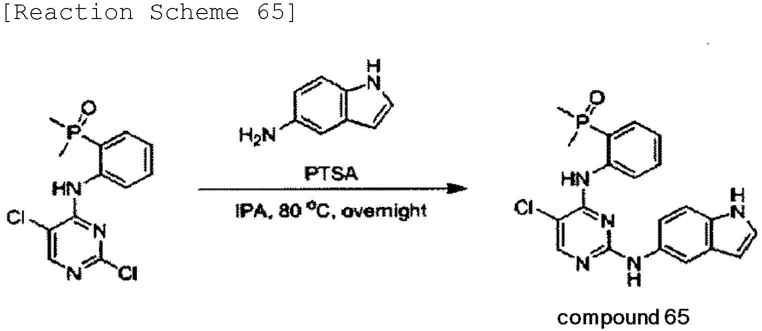

To a solution of 1H-indole-5-amine (25 mg, 0.19 mmol) in IPA (0.6 mL) were added 2,5-dichloro-N-(2-(dimethylphosphoryl)phenyl)pyrimidine-4-amine (50 mg, 0.16 mmol) and then PTSA (30 mg, 0.16 mmol). The reaction mixture was heated overnight at 80°C. The crude mixture was subjected to purification by silica gel column chromatography using EA:MeOH (9.5/0.5) to afford compound 65 as a gray solid (15 mg, 0.036 mmol, 23 %).

LC/MS (ESI) m/z 412.6 [M+H]⁺

¹H NMR (300 MHz, DMSO-d6) δ 11.12 (s, 1H), 10.96 (s, 1H), 9.20 (s, 1H), 8.64 (bs, 1H), 8.15 (s, 1H), 7.86 (s, 1H), 7.57 (dd, J = 15, 9.0 Hz, 1H), 7.38 (t, J = 9.0 Hz, 1H), 7.33-7.26 (m, 2H), 7.24-7.10 (m, 2H), 6.35-6.31 (m, 1H), 1.80 (s, 3H), 1.76 (s, 3H)

### Compound 66. (2-((2-((1H-Indazol-5-yl)amino)-5-chloropyrimidin-4-yl)amino)phenyl)dimethylphosphine oxide

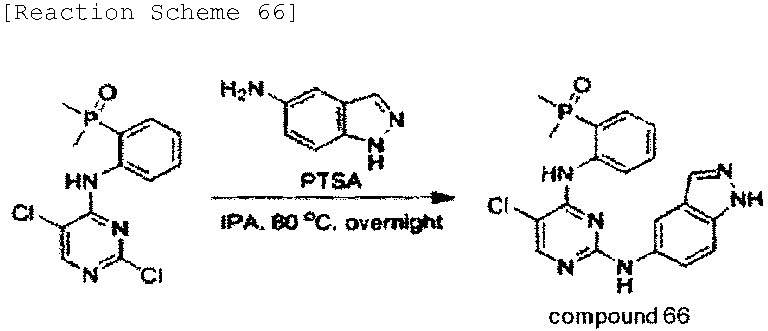

To a solution of 1H-indazol-5-amine (28 mg, 0.17 mmol) in IPA (0.6 mL) were added 2,5-dichloro-N-(2-(dimethylphosphoryl)phenyl)pyrimidine-4-amine (50 mg, 0.16 mmol) and then PTSA (46 mg, 0.19 mmol) . The reaction mixture was heated overnight at 80°C. TLC indicated the formation of a new spot. The reaction was quenched with sodium bicarbonate, followed by extraction with DCM (20 mL×2). The organic layer was dried over sodium sulfate and the solvent was removed by evaporation in a vacuum. The crude mixture was subjected to purification by MPLC using 5 % MeOH:DCM to afford Compound 66 as a downy solid (20 mg, 0.048 mmol, 30 %).

LCMS: 413.2 [M+H]⁺

¹H NMR (400 MHz, DMSO-d6) δ 12.91 (s, 1H), 11.09 (s, 1H), 9.39 (s, 1H), 8.56 (s, 1H), 8.18 (d, J = 2.6 Hz, 1H), 8.08 (s, 1H), 7.93 (s, 1H), 7.68-7.53 (m, 1H), 7.51-7.40 (m, 3H), 7.19 (t, J = 7.2 Hz, 1H), 1.79 (s, 3H), 1.76 (s, 3H).

### Compound 67. (2-((2-((3-Bromo-1H-indazol-5-yl)amino)-5-chloropyrimidin-4-yl)amino)phenyl)dimethylphosphine oxide

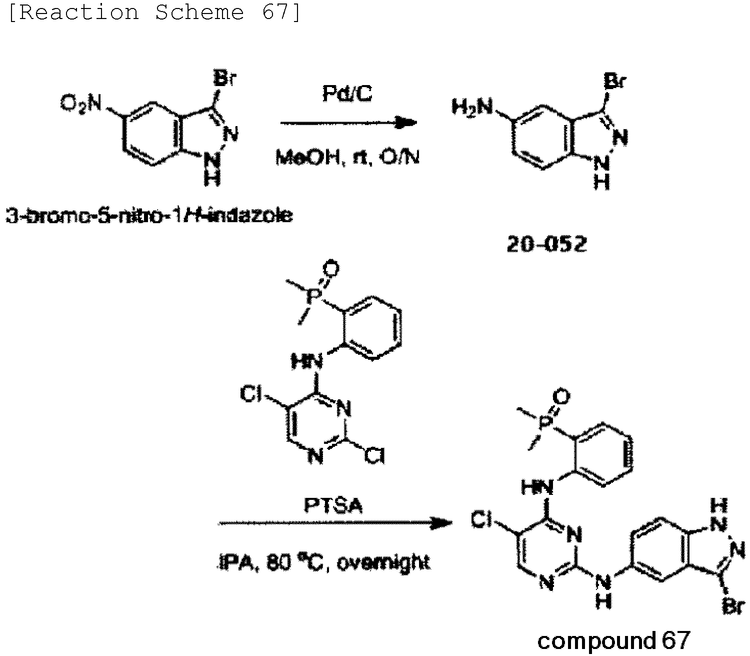

### 1) Synthesis of 3-bromo-1H-indazol-5-amine

To a solution of 2,5-dichloro-N-(2-(dimethylphosphoryl)phenyl)pyrimidine-4-amine (100 mg, 0.413 mmol) in 10 mL of MeOH was added Pd/C (10 mg, 10 mol%), followed by shaking overnight in a parr shaker. When TLC indicated completion of the reaction, the catalyst was filtrated off and the solvent was evaporated to afford 20-052 as a purple solid (77 mg, 0.363 mmol, 88 %).

¹H NMR (400 MHz, DMSO-d6) δ 13.33 (s, 1H), 9.85 (s, 2H), 8.19 (s, 1H), 7.78 (s, 1H), 7.68 (d, J = 8.9 Hz, 1H), 7.31 (d, J = 9.1 Hz, 1H).

### 2) 2-((2-((3-Bromo-1H-indazol-5-yl)amino)-5-chloropyrimidin-4-yl)amino)phenyl)dimethylphosphine oxide.

To a solution of 20-052 (36.0 mg, 0.17 mmol) in IPA (0.6 mL) were added 2,5-dichloro-N-(2-(dimethylphosphoryl)phenyl)pyrimidine-4-amine (50 mg, 0.16 mmol) and then PTSA (46 mg, 0.19 mmol). The reaction mixture was heated overnight at 80°C. TLC indicated the formation of a new spot. The reaction was quenched with sodium bicarbonate, followed by extraction with DCM (20 mL×2). The organic layer was dried over sodium sulfate and the solvent was removed by evaporation in a vacuum. The crude mixture was subjected to purification by MPLC using 5 % MeOH:DCM as an eluent to afford Compound 67 as a downy solid (25 mg, 0.050 mmol, 31 %).

LCMS: 492.2 [M+H]⁺

¹H NMR (400 MHz, DMSO-d6) δ 12.92 (s, 1H), 11.08 (s, 1H), 9.40 (s, 1H), 8.57 (s, 1H), 8.19 (s, 1H), 8.08 (s, 1H), 7.93 (s, 1H), 7.60 (dd, J = 14.0, 7.8 Hz, 1H), 7.45 (d, J = 6.7 Hz, 3H), 7.20 (t, J = 7.8 Hz, 1H), 1.79 (s, 3H), 1.76 (s, 3H).

### Compound 68. (2-((2-((1H-Indol-4-yl)amino)-5-chloropyrimidin-4-yl)amino)phenyl)dimethylphosphine oxide

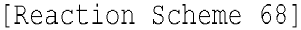

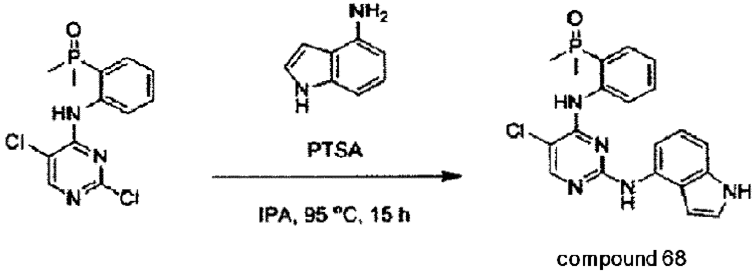

To a 5-mL vial were added 2,5-dichloro-N-(2-(dimethylphosphoryl)phenyl)pyrimidine-4-amine (114 mg, 0.360 mmol) and then 1H-indol-4-amine (50.0 mg, 0.378 mmol), PTSA (103 mg, 0.541 mmol), and IPA (1 mL). The reaction mixture was heated at 95 °C for 15 hours. The reaction was quenched with sodium bicarbonate, followed by extraction with DCM (50 mL×2) and water. The organic layer was dried over sodium sulfate and the solvent was evaporated in a vacuum. The solid was pulverized and purified to afford Compound 68 as a gray solid (60.5 mg, 0.147 mmol, 41 %).

LC/MS: 412.30 [M+H]⁺

¹H NMR (300 MHz, CDCl₃) δ 10.92 (s, 1H), 8.63 (dd, J = 8.6, 4.5 Hz, 1H), 8.25 (s, br, 1H), 7.81 (dd, J = 5.2, 3.4 Hz, 1H), 7.42 (t, J = 7.8 Hz, 1H), 7.29 (s, 1H), 7.24-7.13 (m, 5H), 7.09 (t, J = 7.5 Hz, 1H), 6.58 (t, J = 2.8 Hz, 1H), 1.88 (s, 3H), 1.84 (s, 3H).

### Compound 69. (2-((5-Chloro-2-((2-methyl-1H-indol-5-yl)amino)pyrimidin-4-yl)amino)phenyl)dimethylphosphine oxide

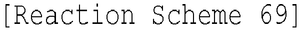

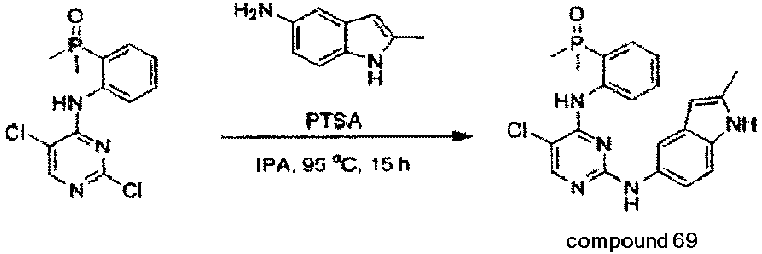

To a 5-mL vial were added 2,5-dichloro-N-(2-(dimethylphosphoryl)phenyl)pyrimidine-4-amine (103 mg, 0.326 mmol) and then, 2-methyl-1H-indole-5-amine (50.0 mg, 0.342 mmol), PTSA (93 mg, 0.541 mmol), and IPA (1 mL). The reaction mixture was heated at 95 °C for 15 hours. The reaction was quenched with sodium bicarbonate, followed by extraction with DCM (50 mL×2) and water. The organic layer was dried over sodium sulfate and the solvent was evaporated in a vacuum. The mixture was subjected to purification by MPLC using 3 % to 5 % (gradient) MeOH in MC to afford Compound 69 as a white solid (34.2 mg, 0.147 mmol, 25 %).

¹H NMR (400 MHz, CDCl₃) δ 10.88 (s, 1H), 8.68 (s, 1H), 8.07 (s, 1H), 7.87 (s, 1H), 7.66 (d, J = 60.2 Hz, 1H), 7.32 (d, J = 25.7 Hz, 1H), 7.24-6.96 (m, 4H), 6.91 (d, J = 12.4 Hz, 1H), 6.16 (s, 1H), 3.49 (s, 3H), 1.84 (s, 3H), 1.82 (s, 3H).

### Compound 70. 7-((5-chloro-4-((2-(dimethylphosphoryl)phenyl)amino)pyrimidin-2-yl)amino)-4-methyl-1H-indole-3-carbonitrile

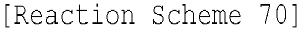

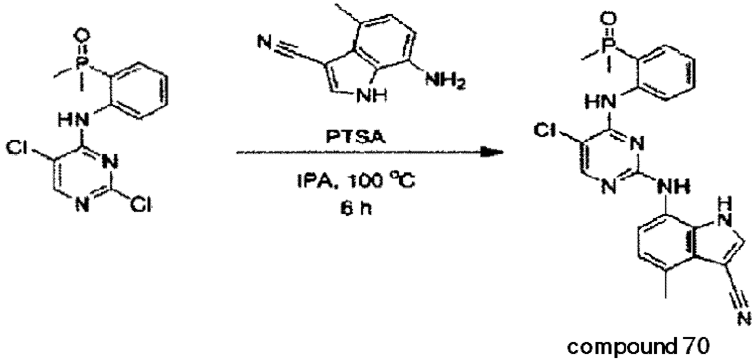

To a 5-mL vial were added 2,5-dichloro-N-(2-(dimethylphosphoryl)phenyl)pyrimidine-4-amine (100 mg, 0.316 mmol) and then, 7-amino-4-methyl-1H-indole-3-carbonitrile (43.3 mg, 0.253 mmol), PTSA (96.3 mg, 0.506 mmol), and IPA(1 mL). The reaction mixture was heated at 100°C for 6 hours. The reaction was quenched with sodium bicarbonate, followed by extraction with DCM (50 mL×2) and water. The organic layer was dried over sodium sulfate and the solvent was evaporated in a vacuum. The mixture was subjected to purification by MPLC using 5 % MeOH in MC to afford Compound 70 as a white solid (46.1 mg, 0.102 mmol, 32 %).

¹H NMR (400 MHz, DMSO) δ 11.96(s, br,1H),11.21 (s, 1H), 9.18 (s, 1H), 8.12 (d, J = 16.6 Hz, 2H), 8.02 (s, 1H), 7.51 (d, J = 10.7 Hz, 1H), 7.24 (d, J = 7.6 Hz, 1H), 7.05 (s, 2H), 6.91 (d, J = 7.7 Hz, 1H), 2.66 (s, 3H), 1.77 (s, 3H), 1.73 (s, 3H).

### Compound 71. (2-((2-((1H-Indol-7-yl)amino)-5-chloropyrimidin-4-yl)amino)phenyl)dimethylphosphine oxide

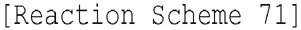

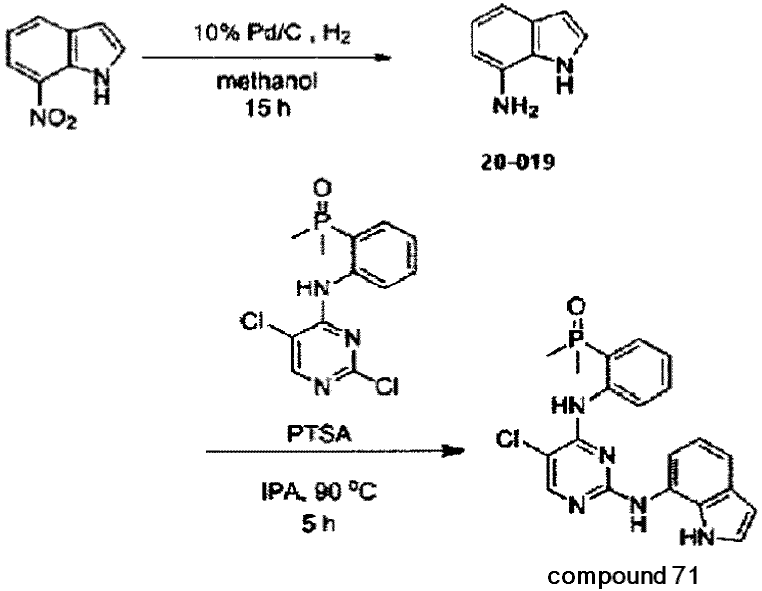

### 1) Synthesis of 20-019

A 50-mL RB was filled with methanol. To this solution, 7-nitroindole (350 mg, 2.16 mmol) and 10 % Pd/C (35 mg) were added. After 15 hours, Pd/C was removed using a syringe filter. After 30 minutes, the solvent was evaporated using a rotary evaporator, and the residue was treated with 1N HCl and NaOH to afford Compound 20-019 as a light purple solid (302 mg, 2.28 mmol, 96 %).

¹H NMR (400 MHz, CDCl₃) δ 8.06 (s, 1H), 7.22 - 7.15 (m, 1H), 7.11 (s, 1H), 6.96 (td, J = 7.9, 2.5 Hz, 1H), 6.58 (dd, J = 7.5, 2.6 Hz, 1H), 6.52 (d, J = 3.2 Hz, 1H) .

### 2) (2-((2-((1H-Indol-7-yl)amino)-5-chloropyrimidin-4-yl)amino) phenyl)dimethylphosphine oxide

To a 5-mL vial were added 2,5-dichloro-N-(2-(dimethylphosphoryl)phenyl)pyrimidine-4-amine (100 mg, 0.316 mmol) and then, 20-019 (44.2 mg, 0.332 mmol), PTSA (96.3 mg, 0.506 mmol), and IPA (1 mL). The reaction mixture was heated at 90°C for 6 hours. The reaction was quenched with sodium bicarbonate, followed by extraction with DCM (50 mL×2) and water., The organic layer was dried over sodium sulfate and the solvent was evaporated in a vacuum. The mixture was subjected to purification by MPLC using 4% to 8% MeOH in MC (gradient) to afford Compound 71 as a white and light pink solid (85.8 mg, 0.208 mmol, 66 %).

¹H NMR (400 MHz, DMSO) δ 11.18 (s, 1H), 10.93 (s, 1H), 9.11 (s, 1H), 8.20(s, br, 1H), 8.17 (s, 1H), 7.50 (ddd, J = 14.0, 7.7, 1.7 Hz, 1H), 7.33 (d, J = 7.7 Hz, 2H), 7.28 (t, J = 2.8 Hz, 1H), 7.13 (s, 1H), 7.05 (d, J = 7.3 Hz, 1H), 6.94 (t, J = 7.7 Hz, 1H), 6.46 (dd, J = 3.1, 1.9 Hz, 1H), 1.75 (s, 3H), 1.73 (s, 3H)

### Compound 72. 1-(5-((5-Chloro-4-((2-(dimethylphosphoryl)phenyl)amino)pyrimidin-2-yl)amino)isoindolin-2-yl)-2,2,2-trifluoroethan-1-one

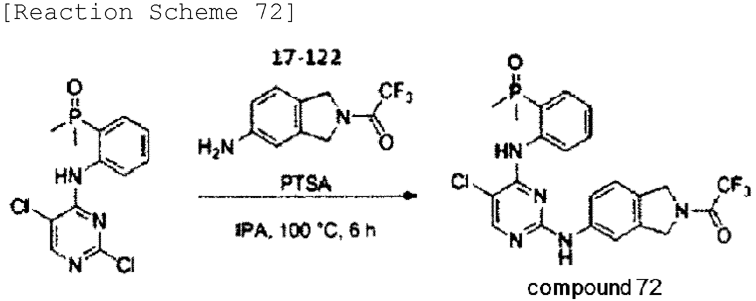

To a 5-mL vial were added 2,5-dichloro-N-(2-(dimethylphosphoryl)phenyl)pyrimidine-4-amine (50.0 mg, 0.316 mmol) and then, 17-122 (38.2 mg, 0.166 mmol), PTSA (45.1 mg, 0.237 mmol), and IPA (0.5 mL). The reaction mixture was heated at 100°C for 4 hours. The reaction was quenched with sodium bicarbonate, followed by extraction with DCM (50 mL×2) and water., The organic layer was dried over sodium sulfate and the solvent was evaporated in a vacuum. The mixture was subjected to purification by MPLC using 4% to 7% MeOH in MC (gradient) to afford Compound 72 as a white solid (47.8 mg, 0.0938 mmol, 59 %).

LCMS: 510.2 [M+H]⁺

¹H NMR (300 MHz, CDCl₃) δ 10.94 (d, J = 6.8 Hz, 1H), 8.52 (dt, J = 9.5, 5.2 Hz, 1H), 8.12 (d, J = 1.5 Hz, 1H), 7.77 (d, J = 4.8 Hz, 1H), 7.48 (t, J = 8.1 Hz, 1H), 7.38 - 7.27 (m, 2H), 7.25 - 7.12 (m, 2H), 7.02 (d, J = 3.6 Hz, 1H), 4.99 (d, J = 7.8 Hz, 2H), 4.87 (d, J = 8.4 Hz, 2H), 1.87 (s, 3H), 1.83 (s, 3H)

### Compound 73. (2-((5-Chloro-2-(isoindolin-5-ylamino)pyrimidin-4-yl)amino)phenyl)dimethylphosphine oxide

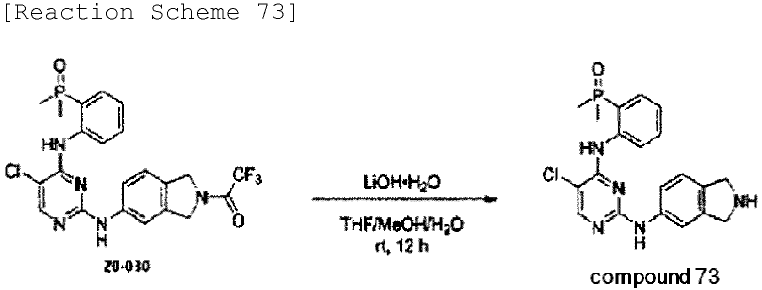

To a solution of 20-030 (20.0 mg, 0.0392 mmol) in THF/MeOH/water (2:1:0.5) (1 mL) was added LiOH.H₂O (4.0 mg, 0.0953 mmol) at room temperature. After 12 hours the solvent was evaporated using a rotary evaporator, and the residue was treated with 1N HCl and NaOH to afford Compound 73 as a white solid (10.0 mg, 0.0105 mmol, 96 %).

¹H NMR (400 MHz, CDCl₃) δ 10.92 (s, 1H), 8.61 (dt, J = 8.3, 3.8 Hz, 1H), 8.12 (d, J = 3.3 Hz, 1H), 7.61 (s, 1H), 7.51 (d, J = 8.5 Hz, 1H), 7.36-7.30 (m, 1H), 7.25-7.09 (m, 2H), 7.02 (s, 1H), 4.24 (d, J = 3.3 Hz, 4H), 1.88 (s, 3H), 1.85 (s, 3H)

### Compound 74. (2-((5-Chloro-2-(pyrrolo[2,1-f] [1,2,4] triazin-4-ylamino)pyrimidin-4-yl)amino)phenyl)dimethylphosphine oxide

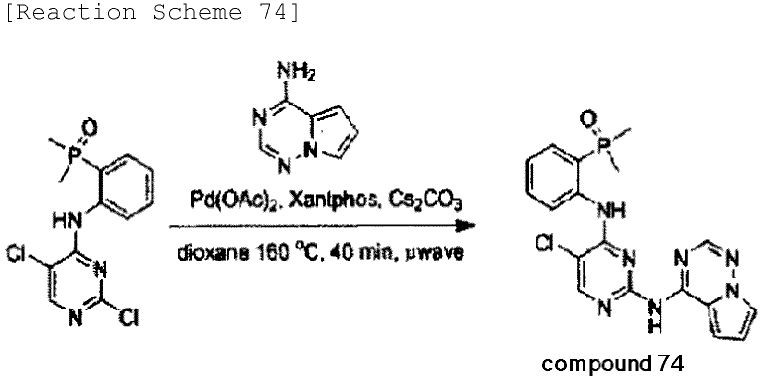

To a solution of pyrrolo[2,1-f] [1,2,4] triazin-4-amine (26 mg, 0.19 mmol) in dioxane (0.6 mL) were added 2,5-dichloro-N-(2-(dimethylphosphoryl)phenyl)pyrimidine-4-amine (50 mg, 0.16 mmol) and then, Pd(OAc)₂ (0.71 mg, 0.0032 mmol), xantphos (2.8 mg, 0.0048 mmol), and cesium carbonate (104 mg, 0.32 mmol). The reaction mixture was heated at 140°C for 40 minutes with microwaves. The resulting mixture was filtered on a celite bed and washed with EA, and the solvent was evaporated in a vacuum. The residue was subjected to purification by MPLC using 5 % MeOH:MC to afford Compound 74 as a white solid (26.0 mg, 0.0628 mmol 40 %).

¹H NMR (400 MHz, Chloroform-d) δ 11.49 (s, 1H), 9.31 (dd, J = 8.6, 4.6 Hz, 1H), 8.27 (s, 2H), 7.81 (s, 1H), 7.72 (t, J = 2.0 Hz, 1H), 7.60 (t, J = 7.9 Hz, 1H), 7.45 (d, J = 10.8 Hz, 1H), 7.16 (t, J = 7.2 Hz, 1H), 6.86-6.82 (m, 1H), 6.81-6.77 (m, 1H), 1.87 (s, 3H), 1.84 (s, 3H).

### Compound 75. (2-((5-Chloro-2-((2-(trifluoromethyl)-1H-benzo[d] imidazol-5-yl)amino)pyrimidin-4-yl)amino)phenyl)dimethylphosphine oxide

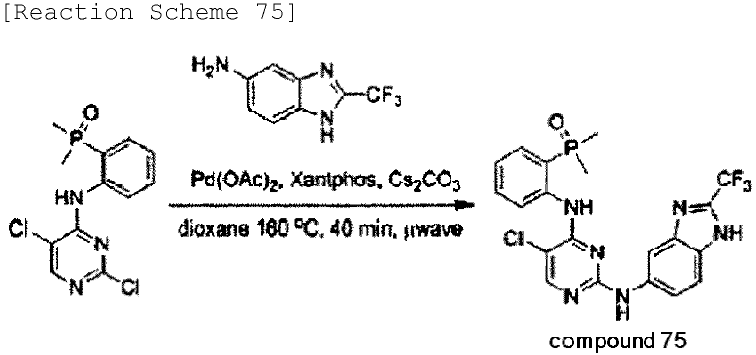

To a solution of 6-methoxybenzo[d] thiazol-2-amine (32 mg, 0.19 mmol) in dioxane (0.6 mL) were added 2,5-dichloro-N-(2-(dimethylphosphoryl)phenyl)pyrimidine-4-amine (50 mg, 0.16 mmol) and then, Pd(OAc)₂ (0.71 mg, 0.0032 mmol), xantphos (2.8 mg, 0.0048 mmol), and cesium carbonate (104 mg, 0.32 mmol). The reaction mixture was heated at 140°C for 40 minutes with microwaves. The resulting mixture was filtered on a celite bed and washed with EA, and the solvent was evaporated in a vacuum. The residue was subjected to purification by MPLC using 5 % MeOH:MC to afford Compound 75 as a brown solid (31.0 mg, 0.0645 mmol 40 %).

LCMS: 381.2 [M+H]⁺

¹H NMR (500 MHz, DMSO-d6) δ 13.70 (s, 1H), 11.29 (s, 1H), 9.60 (s, 1H), 8.71 (s, 1H), 8.24 (s, 1H), 8.12 (s, 1H), 7.67 (s, 1H), 7.63-7.55 (m, 2H), 7.49 (t, J = 7.8 Hz, 1H), 7.17 (t, J = 7.5 Hz, 1H), 1.81 (s, 3H), 1.78 (s, 3H).

### Compound 76. (2-((5-Chloro-2-((2-mercapto-1H-benzo[d] imidazol-5-yl)amino)pyrimidin-4-yl)amino)phenyl)dimethylphosphine oxide

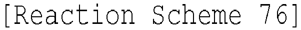

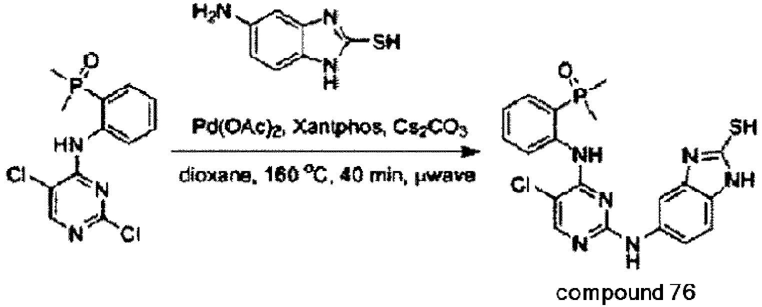

To a solution of 6-methoxybenzo[d] thiazol-2-amine (26 mg, 0.19 mmol) in dioxane (0.6 mL) were added 2,5-dichloro-N-(2-(dimethylphosphoryl)phenyl)pyrimidine-4-amine (50 mg, 0.16 mmol) and then, Pd(OAc)₂ (0.71 mg, 0.0032 mmol), xantphos (2.8 mg, 0.0048 mmol), and cesium carbonate (104 mg, 0.32 mmol). The reaction mixture was heated at 140°C for 40 minutes with microwaves. The resulting mixture was filtered on a celite bed and washed with EA, and the solvent was evaporated in a vacuum. The residue was subjected to purification by MPLC using 5 % MeOH:MC to afford Compound 76 as a yellow solid (16.0 mg, 0.0359 mmol 22 %).

LCMS: 445.1 [M+H]⁺

¹H NMR (500 MHz, DMSO-d6) δ 12.54 (s, 1H), 11.82 (s, 1H), 8.40 (s, 1H), 7.61 (dd, J = 8.5, 4.3 Hz, 1H), 7.45 (ddd, J = 14.1, 7.7, 1.6 Hz, 1H), 7.39 (d, J = 8.6 Hz, 1H), 6.90-6.84 (m, 1H), 6.65 (dd, J = 8.6, 2.1 Hz, 1H), 6.63 (d, J = 2.0 Hz, 1H), 6.09 (t, J = 7.8 Hz, 1H), 5.15 (s, 2H), 1.76 (s, 3H), 1.74 (s, 3H).

### Compound 77. (2-((5-Chloro-2-(imidazo[1,2-a] pyridin-8-ylamino)pyrimidin-4-yl)amino)phenyl)dimethylphosphine oxide

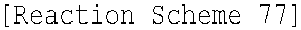

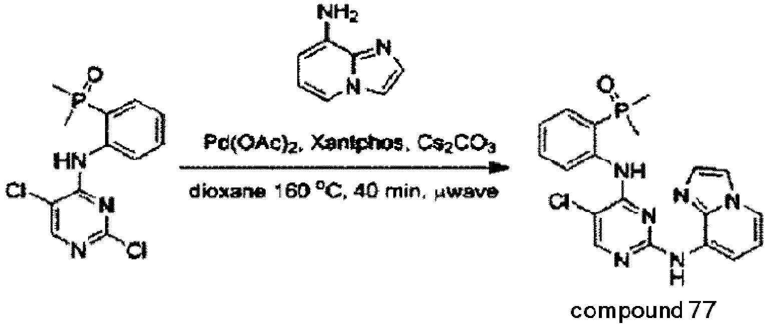

To a solution of imidazo[1,2-a] pyridin-8-amine (26.4 mg, 0.19 mmol) in dioxane (0.6 mL) were added 2,5-dichloro-N-(2-(dimethylphosphoryl)phenyl)pyrimidine-4-amine (50 mg, 0.16 mmol) and then Pd(OAc)₂ (0.71 mg, 0.0032 mmol), xantphos (2.8 mg, 0.0048 mmol), and cesium carbonate (104 mg, 0.32 mmol). The reaction mixture was heated at 140°C for 40 minutes with microwaves. The resulting mixture was filtered on a celite bed and washed with EA, and the solvent was evaporated in a vacuum. The residue was subjected to purification by MPLC using 5 % MeOH:MC to afford Compound 77 as a yellow solid (16.0 mg, 0.0359 mmol, 22 %).

LCMS: 413.1 [M+H]⁺

¹H NMR (500 MHz, DMSO-d6) δ 12.54 (s, 1H), 11.82 (s, 1H), 8.40 (s, 1H), 7.61 (dd, J = 8.5, 4.3 Hz, 1H), 7.45 (ddd, J = 14.1, 7.7, 1.6 Hz, 1H), 7.39 (d, J = 8.6 Hz, 1H), 6.90-6.84 (m, 1H), 6.65 (dd, J = 8.6, 2.1 Hz, 1H), 6.63 (d, J = 2.0 Hz, 1H), 6.09 (t, J = 7.8 Hz, 1H), 5.15 (s, 2H), 1.76 (s, 3H), 1.74 (s, 3H).

### Compound 78. (2-((5-Chloro-2-((1-methyl-1H-pyrazolo[3,4-b] pyridin-5-yl)amino)pyrimidin-4-yl)amino)phenyl)dimethylphosphine oxide

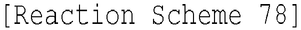

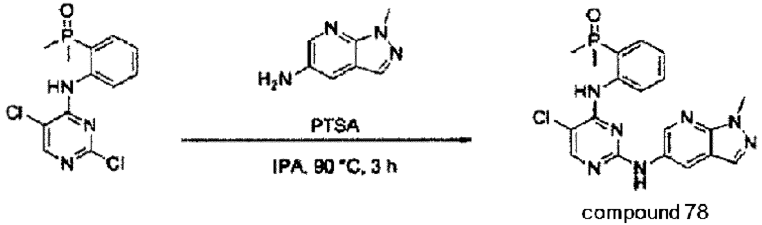

To a solution of 2,5-dichloro-N-(2-(dimethylphosphoryl)phenyl)pyrimidine-4-amine (50.0 mg, 0.158 mmol) in IPA (1 mL) were added 1-methyl-1H-pyrazolo[3,4-b] pyridin-5-amine (24.6 mg, 0.166 mmol) and then PTSA (54.2 mg, 0.285 mmol). The reaction mixture was heated at 90°C for 3 hours. The reaction mixture was filtered, and the solid was washed with MC and alkalinized with sodium bicarbonate to afford Compound 78 as a white solid (7.12 mg, 0.0166 mmol, 10 %).

LC/MS: 426.38 [M + H+]

¹H NMR (400 MHz, CDCl₃) δ 10.99 (s, 1H), 8.53-8.49 (m, 1H), 8.49 (s, 1H), 8.12 (s, 1H), 7.92 (s, 1H), 7.37 (t, J = 7.9 Hz, 1H), 7.32-7.27 (m, 1H), 7.17-7.09 (m, 1H), 7.00 (d, J = 1.6 Hz, 1H), 4.17 (s, 3H), 1.86 (s, 3H), 1.83 (s, 3H).

### Compound 79. (2-((2-((1H-Pyrrolo[2,3-b] pyridin-5-yl)amino)-5-chloropyrimidin-4-yl)amino)phenyl)dimethylphosphine oxide

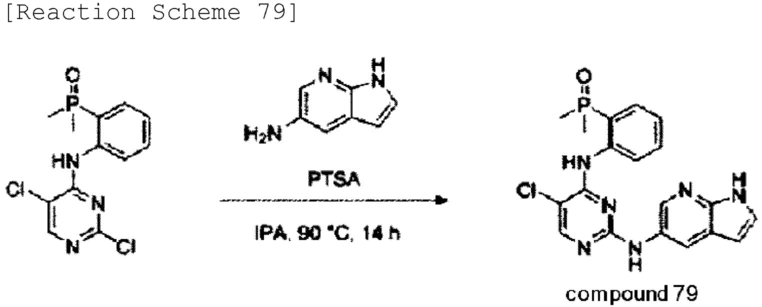

To a solution of 2,5-dichloro-N-(2-(dimethylphosphoryl)phenyl)pyrimidine-4-amine (50.0 mg, 0.158 mmol) in IPA (1 mL) were added 1H-pyrrolo [2,3-b] pyridin-5-amine (23.2 mg, 0.174 mmol) and then, PTSA (45.1 mg, 0.316 mmol). The reaction mixture was heated at 90°C for 14 hours. The reaction was filtered, and the solid was washed with MC and alkalinized with sodium bicarbonate. The reaction mixture was subjected to purification by silica gel column chromatography using 5% MeOH in DCM to afford Compound 79 as a white solid (43.8 mg, 0.106 mmol, 67 %).

LC/MS: 411.82 [M + H+]

¹H NMR (400 MHz, DMSO) δ 11.51 (s, 1H), 11.13 (s, 1H), 9.34 (s, 1H), 8.53 (s, 1H), 8.29 (d, J = 2.4 Hz, 1H), 8.22 (d, J = 2.4 Hz, 1H), 8.17 (s, 1H), 7.57 (ddd, J = 13.9, 7.7, 1.6 Hz, 1H), 7.46-7.41 (m, 1H), 7.34 (d, J = 9.5 Hz, 1H), 7.15 (tdd, J = 7.5, 2.2, 1.1 Hz, 1H), 6.36 (dd, J = 3.4, 1.9 Hz, 1H), 1.79 (s, 3H), 1.76 (s, 3H)

### Compound 80. (2-((5-Chloro-2-((1-isopropyl-1H-pyrazolo[4,3-c] pyridin-6-yl)amino)pyrimidin-4-yl)amino)phenyl)dimethylphosphine oxide

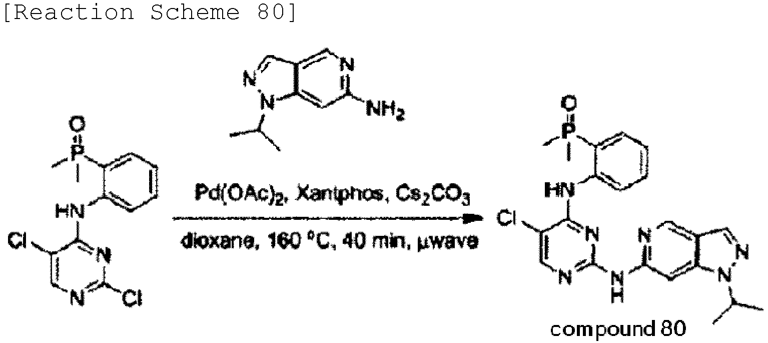

To a solution of 1-isopropyl-1H-pyrazolo[4,3-c] pyridin-6-amine (15.1 mg, 0.086 mmol) in dioxane (2 mL) were added 2,5-dichloro-N-(2-(dimethylphosphoryl)phenyl)pyrimidine-4-amine (25.0 mg, 0.0799 mmol) and then, Pd(OAc)₂ (1.77 mg, 0.00799 mmol), xantphos (4.56 mg, 0.00790 mmol), and cesium carbonate (52.0 mg, 0.160 mmol). The reaction mixture was heated at 160°C for 40 minutes with microwaves. The reaction mixture was filtered on celite bed and washed with EA, and the solvent was evaporated in a vacuum. The residue was subjected to purification by MPLC using 10% MeOH:MC as an eluent to afford Compound 80 as a yellow solid (16.0 mg, 0.0350 mmol 43 %).

LCMS: 456.2 [M+H]⁺.

¹H NMR (500 MHz, Chloroform-d) δ 11.01 (s, 1H), 8.78 (d, J = 1.1 Hz, 1H), 8.72-8.62 (m, 1H), 8.34 (d, J = 1.1 Hz, 1H), 8.30 (s, 1H), 8.28 (s, 1H), 8.04 (s, 1H), 7.56-7.50 (m, 1H), 7.40-7.31 (m, 1H), 7.21-7.14 (m, 1H), 4.66 (h, J = 6.5 Hz, 1H), 1.89 (d, J = 13.2 Hz, 6H), 1.55 (d, J = 6.6 Hz, 6H).

### Compound 81. (2-((2-((3-bromo-1H-pyrazolo[3,4-b] pyridin-5-yl)amino)-5-chloropyrimidin-4-yl)amino)phenyl)dimethylphosphine oxide

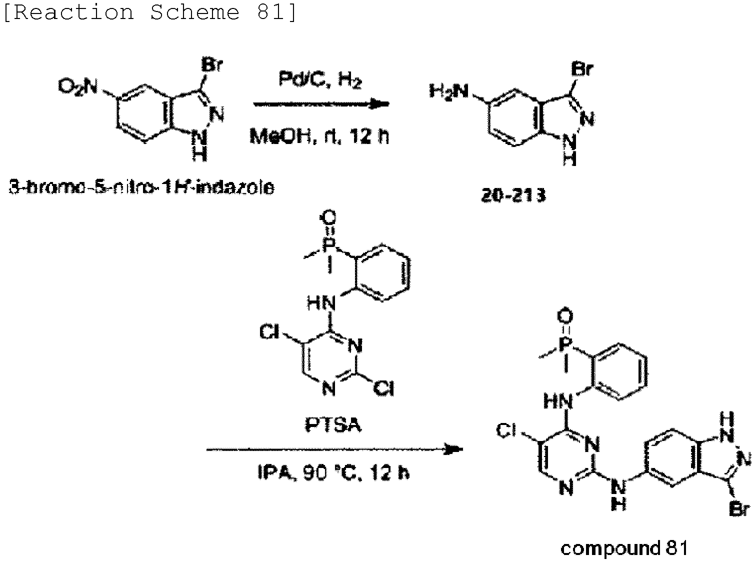

### 1) Synthesis of 3-bromo-1H-indazol-5-amine

To a solution of 3-bromo-5-nitro-1H-indazole (1.0 g, 4.13 mmol) in 10 mL of MeOH was added Pd/C (100 mg, 10 mol%), followed by shaking overnight in a parr shaker. After the reaction was completed as monitored by TLC, the catalyst was filtered off and the solvent was evaporated to afford 3-bromo-1H-indazol-5-amine as a slightly purple solid (691 mg, 3.25 mmol, 78 %).

¹H NMR (400 MHz, DMSO-d6) δ 12.91 (s, 1H), 7.27 (d, J = 8.8 Hz, 1H), 6.86 (dd, J = 8.9, 2.1 Hz, 1H), 6.54 (d, J = 1.9Hz, 1H), 5.02 (s, 2H).

### 2) (2-((2-((3-Bromo-1H-pyrazolo[3,4-b] pyridin-5-yl)amino)-5-chloropyrimidin-4-yl)amino)phenyl)dimethylphosphine oxide

A solution of 3-bromo-1H-indazol-5-amine (890 mg, 2.82 mmol) in IPA (10 mL) were added 2,5-dichloro-N-(2-(dimethylphosphoryl)phenyl)pyrimidine-4-amine (890 mg, 2.82 mmol) and then, PTSA (804 mg, 4.23 mmol). The reaction mixture was heated overnight at 80°C. TLC indicated the formation of a new spot. The reaction was quenched with sodium bicarbonate, followed by extraction with DCM (20 mL×2). The organic layer was dried over sodium sulfate and the solvent was removed by evaporation in a vacuum. The crude mixture was subjected to purification by MPLC using 5 % MeOH:DCM as an eluent to afford Compound 81 as a downy solid (350 mg, 0.713 mmol, 25 %).

LCMS: 493.2 [M+H]⁺.

¹H NMR (400 MHz, DMSO-d6) δ 12.92 (s, 1H), 11.09 (s, 1H), 9.40 (s, 1H), 8.57 (s, 1H), 8.19 (s, 1H), 8.09 (s, 1H), 7.93 (s, 1H), 7.60 (ddd, J = 13.8, 7.7, 1.6 Hz, 1H), 7.52-7.41 (m, 3H), 7.24-7.15 (m, 1H), 1.80 (s, 3H), 1.76 (s, 3H).

### Compound 82. (2-((2-((1H-Pyrazolo[3,4-b] pyridin-5-yl)amino)-5-chloropyrimidin-4-yl)amino)phenyl)dimethylphosphine oxide

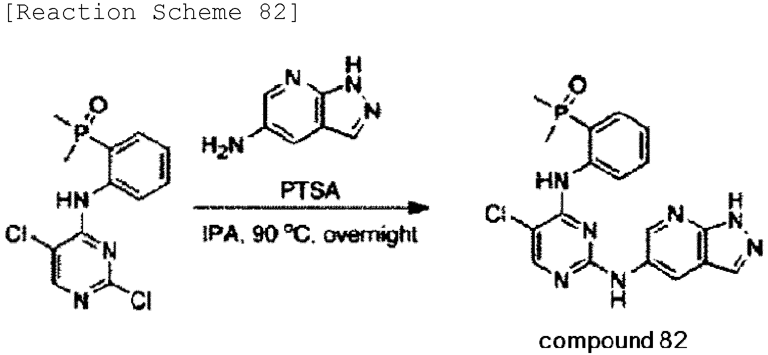

To a solution of 3-bromo-1H-pyrazolo[3,4-b] pyridin-5-amine (22.8 mg, 0.17 mmol) in IPA (0.6 mL) were added 2,5-dichloro-N-(2-(dimethylphosphoryl)phenyl)pyrimidine-4-amine (50 mg, 0.16 mmol) and then PTSA (46 mg, 0.19 mmol) . The reaction mixture was heated overnight at 90°C. TLC indicated the formation of a new spot. The solid was filtered, dissolved in water, alkalinized with sodium bicarbonate (pH 7-8), and washed with 10 mL of MC. After extraction with DCM (20 mL×2), the organic layer was dried over sodium sulfate. The solvent was evaporated in a vacuum to afford Compound 82 as a sponge-type solid (21 mg, 0.050 mmol, 31 %).

LCMS: 414.3 [M+H]⁺

¹H NMR (400 MHz, DMSO-d6) δ 13.52 (s, 1H), 11.11 (s, 1H), 9.58 (s, 1H), 8.62 (d, J = 2.4 Hz, 1H), 8.47 (s, 2H), 8.22 (s, 1H), 8.03 (d, J = 1.4 Hz, 1H), 7.61 (ddd, J = 13.9, 7.7, 1.6 Hz, 1H), 7.43 (t, J = 7.9 Hz, 1H), 7.25 - 7.17 (m, 1H), 1.78 (d, J = 13.5 Hz, 6H)

### Compound 83. (2-((5-Chloro-2-((3-(2-fluoropyridin-3-yl)-1H-indazol-5-yl)amino)pyrimidin-4-yl)amino)phenyl)dimethylphosphine oxide

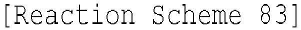

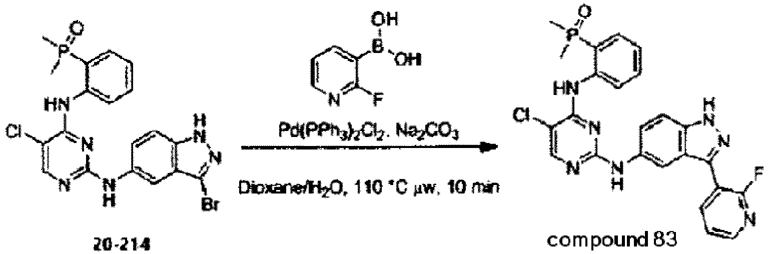

To a solution of 20-214 (50.0 mg, 0.101 mmol) in dioxane/water (2 mL) 2-fluoropyridine-3-boronic acid (17.3 mg, 0.122 mmol) and then, bis(triphenylphosphine)palladium chloride (14.1 mg, 0.0202 mmol) and sodium carbonate (32.1 mg, 0.303 mmol). The reaction mixture was heated at 110°C for 10 hours with microwaves. TLC indicated the formation of a new spot. The solid was filtered and the solvent was evaporated in a vacuum. The crude mixture was subjected to purification by MPLC using 5 % MeOH:DCM to afford Compound 83 as a white solid (7.0 mg, 0.014 mmol, 13 %).

LCMS: 508 [M+H]⁺

¹H NMR (400 MHz, DMSO-d6) δ 13.45 (s, 1H), 11.15 (s, 1H), 9.47 (s, 1H), 8.51 (s, 1H), 8.33-8.25 (m, 2H), 8.19 (s, 1H), 8.09 (d, J = 2.6 Hz, 1H), 7.67 (dd, J = 9.4, 1.8 Hz, 1H), 7.56 (d, J = 9.0 Hz, 1H), 7.53-7.42 (m, 2H), 7.11 (s, 1H), 7.01-6.88 (m, 1H), 1.81 (s, 3H), 1.77 (s, 3H).

### Compound 84. (2-((2-((3-(1H-Pyrazol-4-yl)-1H-indazol-5-yl)amino)-5-chloropyrimidin-4-yl)amino)phenyl)dimethylphosphine oxide

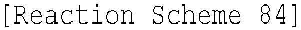

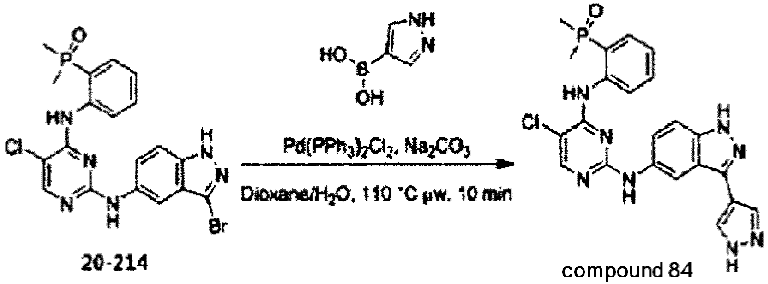

To a solution of 20-214 (50.0 mg, 0.101 mmol) in dioxane/water ( 2mL) were added (1H-pyrazol-4-yl)boronic acid (20.2 mg, 0.181 mmol) and bis(triphenylphosphine)palladium dichloride(20.2 mg, 0.181 mmol) and then bis(triphenylphosphine)palladium dichloride(14.1 mg, 0.0202 mmol) and sodium carbonate (32.1 mg, 0.303 mmol). The reaction mixture was heated at 110°C for 10 hours with microwaves. TLC indicated the formation of a new spot. The solid was filtered and the solvent was evaporated in a vacuum. The crude mixture was subjected to purification by MPLC using 5 % MeOH:DCM as an eluent to afford Compound 84 as a white solid (9.0 mg, 0.018 mmol, 18 %).

LCMS: 479.1 [M+H]⁺

### Compound 85. Tert-butyl tert-butyl 4-(5-((5-chloro-4-((2-(dimethylphosphoryl)phenyl)amino)pyrimidin-2-yl)amino)-1H-indazol-3-yl)-3,6-dihydropyridine-1(2H)-carboxylate

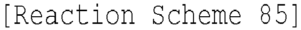

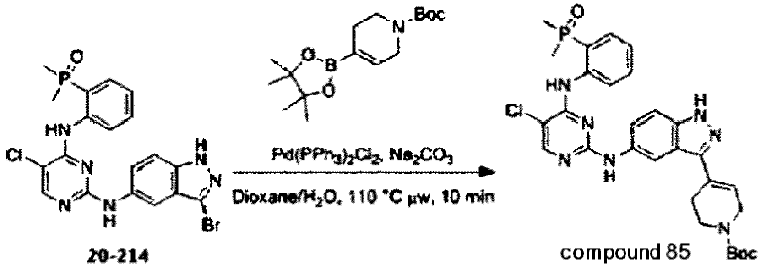

To a solution of 20-214 (50.0 mg, 0.101 mmol) in dioxane/water (2 mL) were added bis(triphenylphosphine)palladium dichloride (20.2 mg, 0.181 mmol) and then, bis(triphenylphosphine)palladium dichloride (14.1 mg, 0.0202 mmol), and sodium carbonate (32.1 mg, 0.303 mmol). The reaction mixture was heated at 110°C for 10 hours with microwaves. TLC indicated the formation of a new spot. The solid was filtered and the solvent was evaporated in a vacuum. The crude mixture was subjected to purification by MPLC using 5 % MeOH:DCM to afford Compound 85 as a white solid (19.0 mg, 0.0319 mmol, 31 %).

LCMS: 594.1 [M+H]⁺

¹H NMR (400 MHz, DMSO-d6) δ 12.89 (s, 1H), 11.21 (s, 1H), 9.35 (s, 1H), 8.59 (s, 1H), 8.27-8.14 (m, 2H), 7.62-7.49 (m, 2H), 7.46 (d, J = 8.9 Hz, 1H), 7.25 (s, 1H), 7.08 (t, J = 7.5 Hz, 1H), 6.29 (s, 1H), 3.91 (s, 2H), 3.61-3.48 (m, 2H), 2.71-2.60 (m, 2H), 1.81 (s, 3H), 1.78 (s, 3H), 1.43 (s, 9H).

### Compound 86. 5-Fluoro-N²-(1H-indazol-5-yl)-N⁴-(1-(methylsulfonyl)piperidin-4-yl)pyrimidine-2,4-diamine

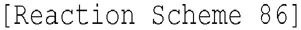

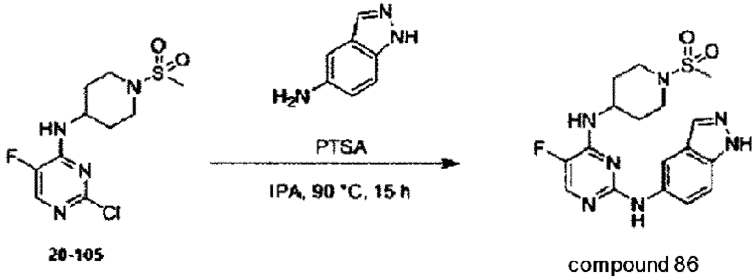

To a solution of 20-105 (50.0 mg, 0.162 mmol) in IPA (1 mL) were added 5-amino-1H-indazole (22.6 mg, 0.170 mmol) and then PTSA (46.2 mg, 0.243 mmol). The reaction mixture was heated at 90°C for 15 hours, and alkalinized with sodium bicarbonate, followed by extraction with DCM. The extract was subjected to silica gel column chromatography using 80 % EA in hex for 20 minutes and 5 % MeOH in MC for 20 minutes to afford Compound 86 as a white solid (9.25 mg, 0.0228 mmol, 14 %).

LC/MS: 406.21 [M + H+]

¹H NMR (400 MHz, DMSO) δ 12.83 (s, 1H), 9.00 (s, 1H), 8.248.18 (m, 1H), 7.95 (s, 1H), 7.89 (d, J = 3.8 Hz, 1H), 7.50 (dd, J = 9.0, 2.0 Hz, 1H), 7.41 (d, J = 8.9 Hz, 1H), 7.34 (d, J = 7.7 Hz, 1H), 4.084.00 (m, 1H), 3.63 (d, J = 12.2 Hz, 2H), 2.92 (s, 3H), 2.892.80 (m, 2H), 2.02 (d, J = 12.7 Hz, 2H), 1.65 (qd, J = 12.0, 4.1 Hz, 2H).

### Compound 87. 5-Fluoro-N²-(1H-indazol-6-yl)-N⁴-(1-(methylsulfonyl)piperidin-4-yl)pyrimidine-2,4-diamine

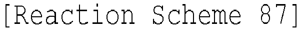

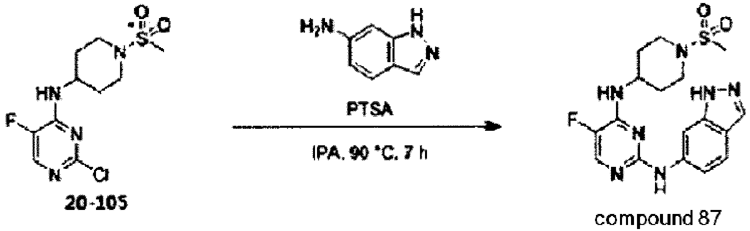

To a solution of 20-105 (50.0 mg, 0.162 mmol) in IPA (1 mL) were added 5-amino-1H-indazole (22.6 mg, 0.170 mmol) and then PTSA (46.2 mg, 0.243 mmol). The reaction mixture was heated at 90°C for 6 hours. The solid was filtered and alkalinized with sodium bicarbonate, followed by extraction with DCM. The reaction mixture was subjected to purification by silica gel column chromatography using 80 % EA in Hex to afford Compound 87 as a white solid (9.25 mg, 0.0228 mmol, 14 %).

¹H NMR (500 MHz, DMSO) δ 12.73 (s, 1H), 9.23 (s, 1H), 8.19 (s, 1H), 7.95 (d, J = 3.6 Hz, 1H), 7.89 (s, 1H), 7.56 (d, J = 8.7 Hz, 1H), 7.41 (d, J = 7.7 Hz, 1H), 7.25 (dd, J = 8.8, 1.8 Hz, 1H), 4.10 (dt, J = 7.6, 3.9 Hz, 1H), 3.62 (d, J = 12.1 Hz, 2H), 2.93 (s, 3H), 2.88 (d, J = 10.3 Hz, 2H), 2.02 (d, J = 12.4 Hz, 2H), 1.73 - 1.61 (m, 2H).

### Compound 88. N²-(3-Bromo-1H-indazol-5-yl)-5-fluoro-N⁴-(1-(methylsulfonyl)piperidin-4-yl)pyrimidine-2,4-diamine

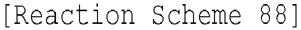

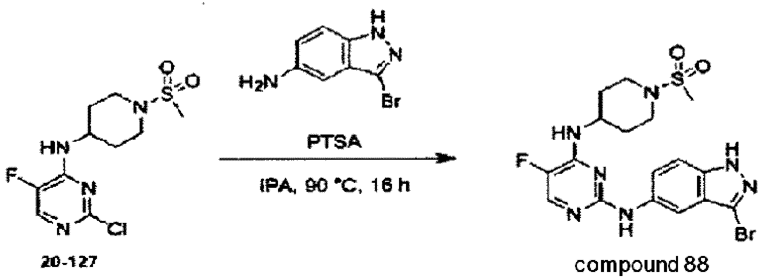

To a solution of 20-127 (50.0 mg, 0.162 mmol) in IPA (1 mL) were added 20-126 (36.1 mg, 0.170 mmol) and then PTSA (46.2 mg, 0.243 mmol). The reaction mixture was heated at 90°C for 16 hours. The solid was filtered and alkalinized with sodium bicarbonate, followed by extraction with DCM. The reaction mixture was subjected to purification by silica gel column chromatography using 80 % EA in Hex to afford Compound 88 as a white solid (61.1 mg, 0.126 mmol, 78 %).

LC/MS: 486.21 [M + H+]

¹H NMR (400 MHz, DMSO) δ 13.21 (s, 1H), 9.21 (s, 1H), 8.16 (d, J = 1.9 Hz, 1H), 7.93 (d, J = 3.8 Hz, 1H), 7.54 (dd, J = 9.1, 2.0 Hz, 1H), 7.45 (d, J = 9.0 Hz, 1H), 7.39 (d, J = 7.8 Hz, 1H), 4.21-4.07 (m, 1H), 3.60 (d, J = 12.2 Hz, 2H), 2.90 (s, 3H), 2.90-2.83 (m, 2H), 2.00 (d, J = 14.4 Hz, 2H), 1.74-1.59 (q, 2H).

### Compound 89. 5-Fluoro-N²-(1H-indol-5-yl)-N⁴-(1-(methylsulfonyl)piperidin-4-yl)pyrimidine-2,4-diamine

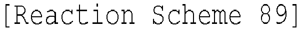

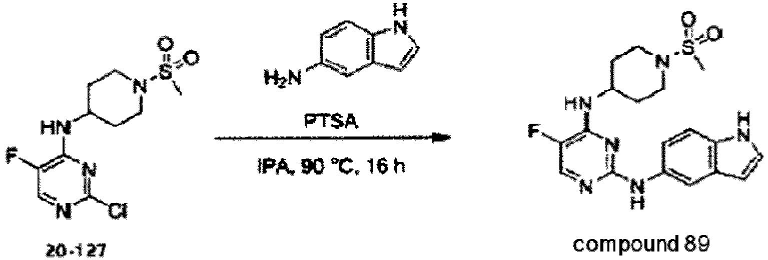

To a solution of 20-127 (50.0 mg, 0.162 mmol) in IPA (1 mL) were added 5-amino-indole (22.5 mg, 0.170 mmol) and then PTSA (46.2 mg, 0.243 mmol). The reaction mixture was heated at 90°C for 7 hours. The solid was filtered and alkalinized with sodium bicarbonate, followed by extraction with DCM. The reaction mixture was subjected to purification by silica gel column chromatography using 80 % EA in Hex to afford Compound 89 as a white solid (29.0 mg, 0.0717 mmol, 45 %).

LC/MS: 405.27 [M + H+]

¹H NMR (400 MHz, DMSO) δ 10.84 (s, 1H), 8.74 (s, 1H), 7.95 (s, 1H), 7.85 (d, J = 3.8 Hz, 1H), 7.25 (d, J = 1.9 Hz, 3H), 6.31 (t, J = 2.5 Hz, 1H), 4.03 (q, J = 7.0 Hz, 1H), 3.63 (d, J = 11.9 Hz, 2H), 2.91 (s, 3H), 2.88-2.78 (m, 2H), 2.01 (d, J = 17.1 Hz, 2H), 1.63 (q, J = 11.6, 10.9 Hz, 2H).

### Compound 90. 5-Fluoro-N²-(2-methyl-1H-indol-5-yl)-N⁴-(1-(methylsulfonyl)piperidin-4-yl)pyrimidine-2,4-diamine

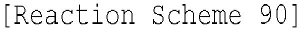

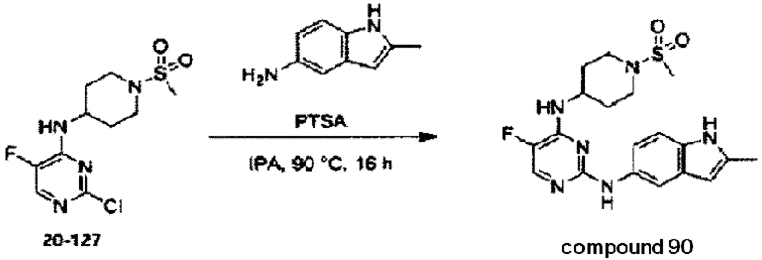

To a solution of 20-127 (50.0 mg, 0.162 mmol) in IPA (1 mL) were added 5-amino-2-methyl indole (24.9 mg, 0.170 mmol) and then PTSA (46.2 mg, 0.243 mmol). The reaction mixture was heated at 90°C for 16 hours. The reaction mixture was alkalinized with sodium bicarbonate, followed by extraction with DCM. The reaction mixture was subjected to purification by silica gel column chromatography using 80 % EA in Hex to afford Compound 90 as a white solid (25.4 mg, 0.0607 mmol, 38 %).

LC/MS: 419.37 [M + H+]

¹H NMR (400 MHz, DMSO) δ 10.64 (s, 1H), 8.67 (s, 1H), 7.81 (dd, J = 15.8, 2.9 Hz, 2H), 7.25 (d, J = 7.7 Hz, 1H), 7.21-6.99 (m, 2H), 6.00 (s, 1H), 4.03 (d, J = 7.5 Hz, 1H), 3.63 (d, J = 11.9 Hz, 2H), 2.91 (s, 3H), 2.89-2.73 (m, 2H), 2.34 (s, 3H), 2.06-1.94 (m, 2H), 1.74-1.55 (m, 2H).

### Compound 91. 2-((5-Chloro-2-((3-(1,2,3,6-tetrahydropyridin-4-yl)-1H-indazol-5-yl)amino)pyrimidin-4-yl)amino)phenyl)dimethylphosphine oxide

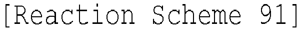

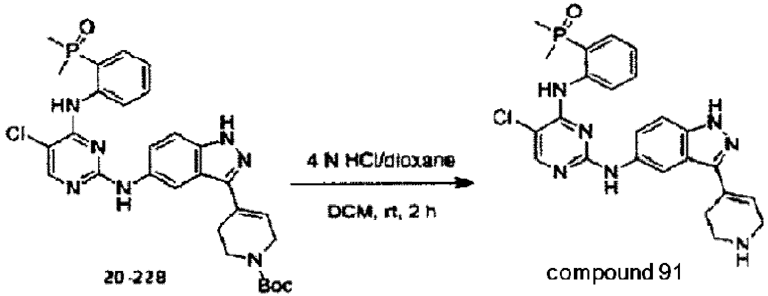

To a solution of 20-228 (1.0 g, 1.3 mol) in 0.25 mL of DCM was added HCl/dioxane 4 N (0.0105 mL, 0.042 mmol) at room temperature. After stirring for 2 hours, the solvent was evaporated. The residue was dissolved in water and washed with DCM. The aqueous layer was alkalinized with sodium bicarbonate, followed by extraction with DCM (15 mL×2). The organic layer was dried over sodium sulfate and the organic layer was evaporated in a vacuum to afford Compound 91 as a white solid (6.0 mg, 0.012 mmol, 75 %).

LCMS: 494.6 [M+H]⁺

¹H NMR (400 MHz, DMSO-d6) δ 12.80 (s, 1H), 11.23 (s, 1H), 9.33 (s, 1H), 8.62 (s, 1H), 8.18 (s, 1H), 8.15 (s, 1H), 7.64 - 7.52 (m, 2H), 7.44 (d, J = 8.9 Hz, 1H) , 7.31 (s, 1H), 7.11 (t, J = 7.4 Hz, 1H), 6.36 (s, 1H), 2.93 (t, J = 5.6 Hz, 2H), 2.55 (s, 2H), 1.80 (s, 3H), 1.77 (s, 3H).

### Compound 92. 5-(5-((5-Chloro-4-((2-(dimethylphosphoryl)phenyl)amino)pyrimidin-2-yl)amino)-1H-indazol-3-yl)furan-2-carbaldehyde

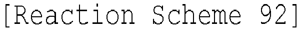

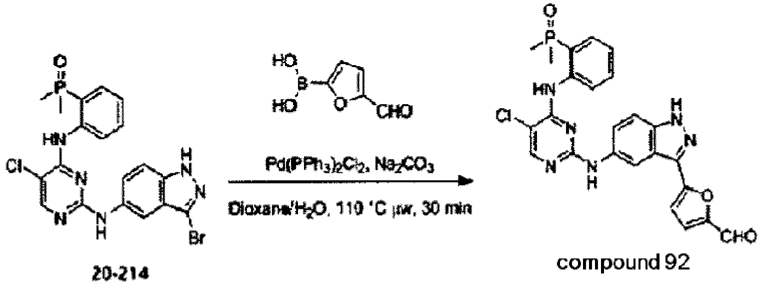

To a solution of 20-214 (50.0 mg, 0.101 mmol) in dioxane/water (2 mL) were added (5-formylfuran-2-yl)boronic acid (25.3 mg, 0.181 mmol) and then, bis(triphenylphosphine)palladium dichloride(14.1 mg, 0.0202 mmol) and sodium carbonate (32.1 mg, 0.303 mmol). The reaction mixture was heated at 110°C for 10 hours with microwaves. TLC indicated the formation of a new spot. The solid was filtered and the solvent was evaporated in a vacuum. The crude mixture was subjected to purification by MPLC using 5 % MeOH:DCM as an eluent to afford Compound 92 as a white solid (9.0 mg, 0.018 mmol, 18 %).

LCMS: 507.2 [M+H]⁺

¹H NMR (400 MHz, DMSO-d6) δ 13.59 (s, 1H), 11.27 (s, 1H), 9.57 (s, 1H), 9.50 (s, 1H), 8.61 (s, 1H), 8.51 (s, 1H), 8.23 (s, 1H), 7.66-7.55 (m, 3H), 7.50 (dd, J = 14.5, 8.0 Hz, 1H), 7.07 (d, J = 3.7 Hz, 2H), 6.99-6.90 (m, 1H), 1.81 (s, 3H), 1.77 (s, 3H).

### Compound 93. (2-((5-Chloro-2-((3-(1-isopropyl-1H-pyrazol-4-yl)-1H-indazol-5-yl)amino)pyrimidin-4-yl)amino)phenyl)dimethylphosphine oxide

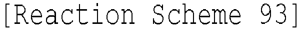

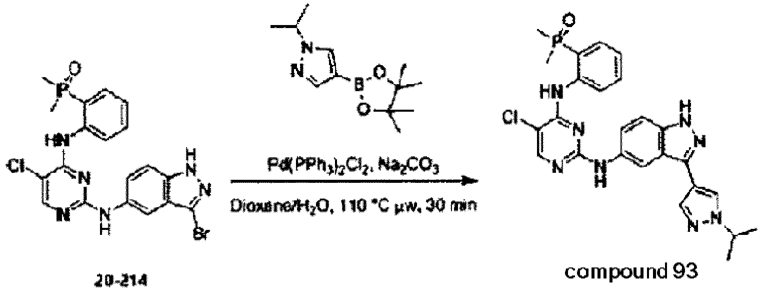

To a solution of 20-214 (50.0 mg, 0.101 mmol) in dioxane/water (2 mL) were added 4-(4,5-dimethyl-1,3,2-dioxaborolan-2-yl)-1-isopropyl-1H-pyrazole (42.7 mg, 0.181 mmol) and then, bis(triphenylphosphine)palladium dichloride(14.1 mg, 0.0202 mmol) and sodium carbonate (32.1 mg, 0.303 mmol). The reaction mixture was heated at 110°C for 30 minutes with microwaves. TLC indicated the formation of a new spot. The solid was filtered and the solvent was evaporated in a vacuum. The crude mixture was subjected to purification by MPLC using 5% MeOH:DCM as an eluent to afford Compound 93 as a brown solid (16.0 mg, 0.0307 mmol, 30 %).

LCMS: 521.96 [M+H]⁺

¹H NMR (500 MHz, DMSO-d6) δ 12.83 (s, 1H), 11.19 (s, 1H), 9.32 (s, 1H), 8.58 (d, J = 16.6 Hz, 1H), 8.21 (s, 1H), 8.19 (s, 1H), 8.15 (s, 1H), 7.89 (s, 1H), 7.84-7.67 (m, 1H), 7.61-7.41 (m, 4H), 6.99 (s, 2H), 4.58-4.47 (m, 1H), 1.78 (s, 3H), 1.76 (s, 3H), 1.42 (d, J = 6.7 Hz, 6H).

### Compound 94. (2-((5-Chloro-2-((3-(1-methyl-1H-pyrazol-4-yl)-1H-indazol-5-yl)amino)pyrimidin-4-yl)amino)phenyl)dimethylphosphine oxide

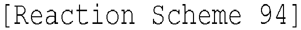

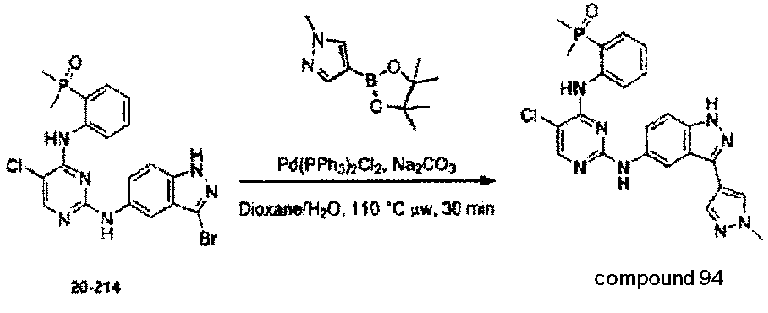

To a solution of 20-214 (50.0 mg, 0.101 mmol) in dioxane/water (2 mL) were added 4-(4,5-dimethyl-1,3,2-dioxaborolan-2-yl)-1-isopropyl-1H-pyrazole (42.7 mg, 0.181 mmol) and then bis(triphenylphosphine)palladium dichloride (14.1 mg, 0.0202 mmol) and sodium carbonate (32.1 mg, 0.303 mmol). The reaction mixture was heated at 110°C for 30 minutes with microwaves. TLC indicated the formation of a new spot. The solid was filtered and the solvent was evaporated in a vacuum. The crude mixture was subjected to purification by MPLC using 5% MeOH:DCM as an eluent to afford Compound 94 as a white solid (24.0 mg, 0.0486 mmol, 48 %).

LCMS: 493.6 [M+H]⁺

¹H NMR (400 MHz, DMSO-d6) δ 12.83 (s, 1H), 11.21 (s, 1H), 9.33 (s, 1H), 8.57 (s, 1H), 8.19 (s, 1H), 8.15 (s, 1H), 8.11 (s, 1H), 7.86 (s, 1H), 7.84-7.65 (m, 1H), 7.59-7.42 (m, 3H), 7.13-6.92 (m, 1H), 3.85 (s, 3H), 1.80 (s, 3H), 1.77 (s, 3H).

### Compound 95. (2-((5-Chloro-2-((3-(1-propyl-1H-pyrazol-4-yl)-1H-indazol-5-yl)amino)pyrimidin-4-yl)amino)phenyl)dimethylphosphine oxide

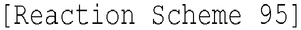

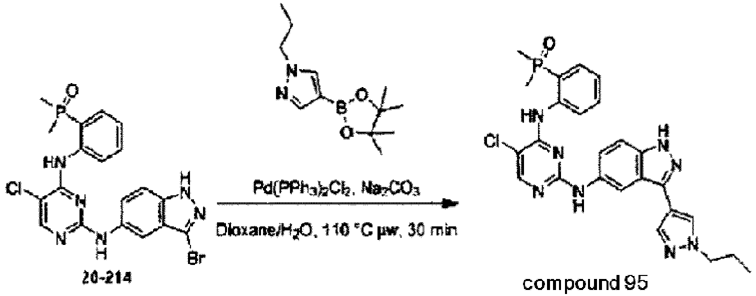

To a solution of 20-214 (50.0 mg, 0.101 mmol) in dioxane/water (2 mL) were added 1-propyl-4-(4,4,5,5-tetramethyl-1,3,2-dioxaborolan-2-yl)-1H-pyrazole (42.7 mg, 0.181 mmol) and then, bis(triphenylphosphine)palladium dichloride (14.1 mg, 0.0202 mmol) and sodium carbonate (32.1 mg, 0.303 mmol). The reaction mixture was heated at 110°C for 30 minutes with microwaves. TLC indicated the formation of a new spot. The solid was filtered and the solvent was evaporated in a vacuum. The crude mixture was subjected to purification by MPLC using 5 % MeOH:DCM as an eluent to afford Compound 95 as a white solid (27.0 mg, 0.0518 mmol, 51 %).

LCMS: 521.6 [M+H]⁺

¹H NMR (400 MHz, DMSO-d6) δ 12.83 (s, 1H), 11.19 (s, 1H), 9.31 (s, 1H), 8.65-8.49 (m, 1H), 8.19 (d, J = 3.0 Hz, 2H), 8.16-8.11 (m, 1H), 7.89 (s, 1H), 7.79 (td, J = 7.9, 3.6 Hz, 1H), 7.70 (ddd, J = 13.6, 8.4, 1.3 Hz, 1H), 7.56-7.48 (m, 2H), 7.46 (d, J = 8.8 Hz, 1H), 6.98 (s, 1H), 4.08 (t, J = 6.9 Hz, 2H), 1.82-1.77 (m, 5H), 1.76 (s, 3H), 0.82 (t, J = 7.4 Hz, 3H).

### Compound 96. (2-((5-chloro-2-((3-phenyl-1H-indazol-5-yl)amino)pyrimidin-4-yl)amino)phenyl)dimethylphosphine oxide

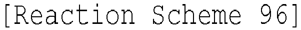

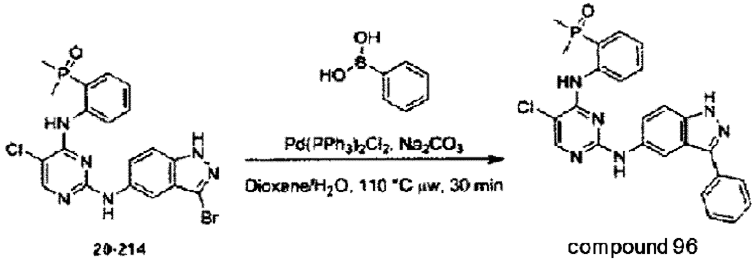

To a solution of 20-214 (50.0 mg, 0.101 mmol) in dioxane/water (2 mL) were added phenylboronic acid (18.5 mg, 0.151 mmol) and then, bis(triphenylphosphine)palladium dichloride (14.1 mg, 0.0202 mmol) and sodium carbonate (32.1 mg, 0.303 mmol). The reaction mixture was heated at 110°C for 30 minutes with microwaves. TLC indicated the formation of a new spot. The solid was filtered and the solvent was evaporated in a vacuum. The crude mixture was subjected to purification by MPLC using 5% MeOH:DCM as an eluent to afford Compound 96 as a white solid (14.0 mg, 0.0286 mmol, 28 %).

LCMS: 489.6 [M+H]⁺

¹H NMR (400 MHz, DMSO-d6) δ 11.40 (s, 1H), 9.62 (s, 1H), 8.53 (s, 1H), 8.28-8.18 (m, 2H), 7.92 (dd, J = 7.3, 1.6 Hz, 2H), 7.62 (dd, J = 8.9, 1.9 Hz, 1H), 7.58-7.50 (m, 2H), 7.46 (t, J = 7.6 Hz, 2H), 7.40-7.34 (m, 1H), 7.03 (s, 2H), 1.79 (d, J = 13.5 Hz, 6H).

### Compound 97. (2-((5-Chloro-2-((3-(3-nitrophenyl)-1H-indazol-5-yl)amino)pyrimidin-4-yl)amino)phenyl)dimethylphosphine oxide

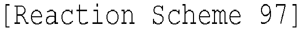

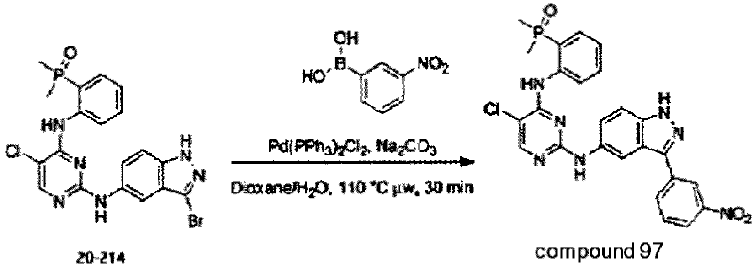

To a solution of 20-214 (50.0 mg, 0.101 mmol) in dioxane/water (2 mL) were added bis(triphenylphosphine)palladium dichloride (14.1 mg, 0.0202 mmol) and sodium carbonate (32.1 mg, 0.303 mmol). The reaction mixture was heated at 110°C for 30 minutes with microwaves. TLC indicated the formation of a new spot. The solid was filtered and the solvent was evaporated in a vacuum. The crude mixture was subjected to purification by MPLC using 5% MeOH:DCM as an eluent to afford Compound 97 as a bright green (16.0 mg, 0.0299 mmol, 29 %).

LCMS: 534.6. [M+H]⁺,

¹H NMR (500 MHz, DMSO-d6) δ 13.44 (s, 1H), 11.26 (s, 1H), 9.57 (s, 1H), 8.58 (d, J = 39.6 Hz, 3H), 8.34 (d, J = 7.8 Hz, 1H), 8.23 (s, 1H), 8.20-8.12 (m, 1H) , 7.71 (t, J = 8.0 Hz, 1H), 7.64 (d, J = 9.1 Hz, 1H), 7.59 (d, J = 8.9 Hz, 1H), 7.53-7.41 (m, 1H), 7.24-6.73 (m, 2H), 1.81 (s, 3H), 1.78 (s, 3H).

### Compound 98. (2-((5-Chloro-2-((3-(2-fluoropyridin-3-yl)-1H-pyrazolo[3,4-b] pyridin-5-yl)amino)pyrimidin-4-yl)amino)phenyl)dimethylphosphine oxide

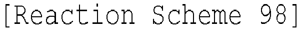

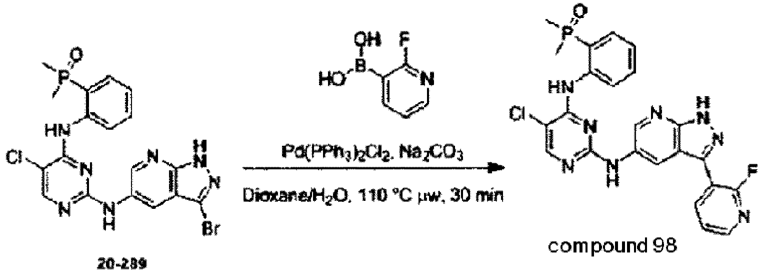

To a solution of 20-289 (30.0 mg, 0.0610 mmol) in dioxane/water (2 mL) were added (2-fluoropyridin-3-yl)boronic acid (13.0 mg, 0.0915 mmol) and then, bis(triphenylphosphine)palladium dichloride(8.5 mg, 0.0122 mmol) and sodium carbonate (20.1 mg, 0.183 mmol). The reaction mixture was heated at 110°C for 100 minutes with microwaves. TLC indicated the formation of a new spot. The solid was filtered and the solvent was evaporated in a vacuum. The crude mixture was subjected to purification by MPLC using 5% MeOH:DCM as an eluent to afford Compound 98 as a white solid (5.0 mg, 0.0098 mmol, 16 %).

LCMS: 509.2 [M+H]⁺

¹H NMR (400 MHz, Chloroform-d) δ 10.90 (s, 1H), 8.79 (dd, J = 12.6, 2.5 Hz, 2H), 8.73-8.47 (m, 2H), 8.45-8.38 (m, 1H), 8.31 (dd, J = 8.4, 4.3 Hz, 1H), 8.27-8.22 (m, 1H), 8.20 (s, 1H), 7.38-7.32 (m, 1H), 7.15 (ddd, J = 14.0, 7.7, 1.6 Hz, 1H), 6.98 (t, J = 7.9 Hz, 1H), 6.72 (t, J = 7.3 Hz, 1H), 1.93 (s, 3H), 1.90 (s, 3H) .

### Compound 99. 1-(4-(5-((5-Chloro-4-((2-(dimethylphosphoryl)phenyl)amino)pyrimidin-2-yl)amino)-1H-indazol-3-yl)-3,6-dihydropyridin-1(2H)-yl)ethan-1-one

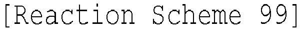

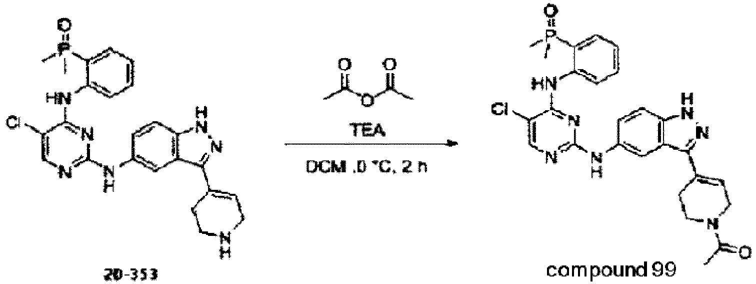

To a suspension of 20-353 (20.0 mg, 0.0405 mmol) in 1 mL of DCM was added acetic anhydride (6.21 mg, 0.0608 mmol) at 0°C and then, TEA (12.8 mg, 0.121 mmol) before stirring for 2 hours. TLC indicated the consumption of the starting materials. The reaction was quenched with 5 mL of water, followed by extraction with DCM (20 mL×2). The organic layer was dried over sodium sulfate and the solvent was evaporated to afford Compound 99 as a white solid (7.00 mg, 0.013 mmol, 32 %).

The product was found to exist as rotamers at 3:2 as measured by NMR.

LCMS: 536.6 [M+H]⁺, 558.6 [M+ Na] +.

¹H NMR (400 MHz, Chloroform-d) δ 11.18-11.02 (m, 1H), 8.69-8.55 (m, 1H), 8.30 (s, 0.6H), 8.19 (s, 0.4H), 8.16-8.10 (m, 1H), 7.46 (s, 1H), 7.46-7.28 (m, 1.6H), 7.28-7.13 (m, 2.4H), 7.10-6.97 (m, 1H), 6.42-6.35 (m, 0.4H), 6.28-6.21 (m, 0.6H), 4.14-4.05 (m, 0.80H), 3.99 (s, 1.20H), 3.82 (t, J = 5.6 Hz, 0.8H), 3.67 (t, J = 5.6 Hz, 1.2H), 2.89-2.80 (m, 1.2H), 2.81-2.73 (m, 0.8H), 2.20 (s, 2.0H), 2.13 (s, 1H), 1.93 (s, 2H), 1.90 (s, 3H), 1.86 (s, 1H).

### Compound 100. (2-((2-((3-(3-Aminophenyl)-1H-indazol-5-yl)amino)-5-chloropyrimidin-4-yl)amino)phenyl)dimethylphosphine oxide

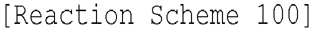

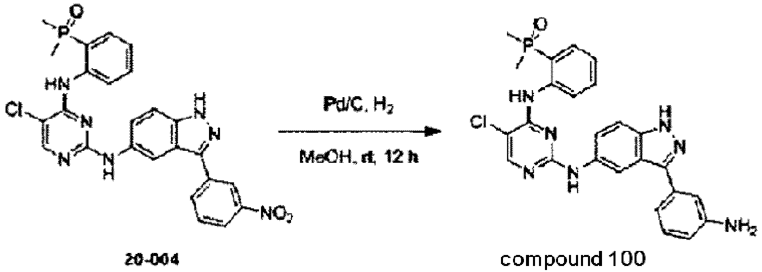

To a suspension of 20-004 (30.0 mg, 0.0561 mmol) in 5 mL of MeOH was added Pd/C (3.0 mg, 10 mol%) at room temperature. The reaction mixture was shaken for 12 hours under a hydrogen atmosphere (50 psi) in a parr reactor. When the reaction was completed as monitored by TLC, the catalyst was filter off through a celite bed. The filtrate was washed with MeOH and the solvent was completely evaporated in a vacuum. The crude mixture was subjected to purification by MPLC using15 % MeOH:MC as an eluent to afford Compound 100 as a gray solid (9.0 mg, 0.0178 mmol, 31 %).

LCMS: 504.1 [M+H]⁺.

¹H NMR (500 MHz, DMSO-d6) δ 11.11-11.04 (m, 1H), 9.18-9.09 (m, 1H), 8.65-8.58 (m, 1H), 8.46-8.39 (m, 1H), 8.17-8.05 (m, 2H), 7.78 (d, J = 8.9 Hz, 1H), 7.66 (dd, J = 9.0, 1.8 Hz, 1H), 7.35 (dd, J = 14.2, 7.6 Hz, 1H), 7.16 (d, J = 7.6 Hz, 1H), 7.08 (t, J = 7.7 Hz, 1H), 6.98-6.89 (m, 2H), 6.72 (s, 1H), 6.65 (dd, J = 8.2, 2.6 Hz, 1H), 5.97 (d, J = 5.9 Hz, 2H), 1.76-1.68 (m, 6H).

### Compound 101. (2-((5-chloro-2-((3-(5-(hydroxymethyl)furan-2-yl)-1H-indazol-5-yl) amino)pyrimidin-4-yl)amino)phenyl)dimethylphosphine oxide

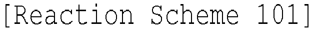

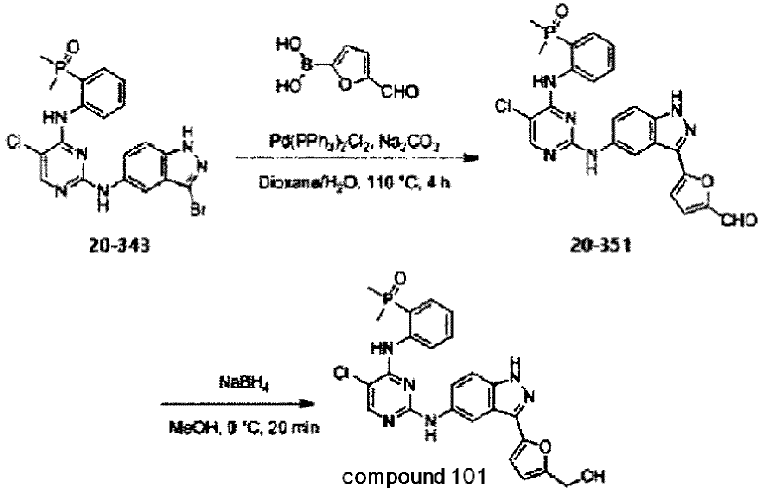

### 1) 5-(5-((5-chloro-4-((2-(dimethylphosphoryl)phenyl)amino)pyrimidin-2-yl)amino)-1H-indazol-3-yl)furan-2-carbaldehyde.

To a solution of 20-343 (200 mg, 0.406 mmol) in dioxane: water (5 mL) were added (5-formylfuran-2-yl)boronic acid (102 mg, 0.488 mmol) and then, bis(triphenylphosphine)palladium dichloride (57.0 mg, 0.0812 mmol) and sodium carbonate (129 mg, 1.21 mmol). The reaction mixture was heated at 110°C for 4 hours. TLC indicated the formation of a new spot. The solid was filtered and the solvent was evaporated in a vacuum. The crude mixture was subjected to purification by MPLC using 5% MeOH:EA as an eluent to afford Compound 20-351 as a reddish yellow solid (30.0 mg, 0.059 mmol, 14.5 %).

¹H NMR (400 MHz, DMSO-d6) δ 13.59 (s, 1H), 11.27 (s, 1H), 9.57 (s, 1H), 9.50 (s, 1H), 8.61 (s, 1H), 8.51 (s, 1H), 8.23 (s, 1H), 7.66-7.55 (m, 3H), 7.50 (dd, J = 14.5, 8.0 Hz, 1H), 7.07 (d, J = 3.7 Hz, 2H), 6.99-6.90 (m, 1H), 1.81 (s, 3H), 1.77 (s, 3H).

### 2) (2-((5-Chloro-2-((3-(5-(hydroxymethyl)furan-2-yl)-1H-indazol-5-yl)amino)pyrimidin-4-yl)amino)phenyl)dimethylphosphine oxide.

To a suspension of 20-351 (15.0 mg, 0.0295 mmol) in MeOH (1 mL) was added sodium borohydride (1.67 mg, 0.0355 mmol) at 0°C, followed by stirring for 15 minutes at the same temperature. TLC indicated the formation of a new spot. The reaction was quenched with water and the solvent was completely evaporated in a vacuum. The residue was diluted with water, followed by extraction with DCM (15 mL×2). The organic layers thus obtained were pooled and dried over sodium sulfate and the solvent was evaporated in a vacuum. The crude mixture was subjected to purification by MPLC using 10 % MeOH:MC as an eluent to afford Compound 101 as a white solid (3.50 mg, 0.00687 mmol, 23 %).

LCMS: 509 [M+H]⁺

¹H NMR (500 MHz, Chloroform-d) δ 11.07 (s, 1H), 8.67 (dd, J = 8.5, 4.4 Hz, 1H), 8.29 (d, J = 1.9 Hz, 1H), 8.14 (s, 1H), 7.54 (dd, J = 8.9, 2.0 Hz, 1H), 7.46 (d, J = 8.9 Hz, 1H), 7.27-7.21 (m, 2H), 7.20-7.13 (m, 1H), 7.01-6.96 (m, 1H), 6.79 (d, J = 3.3 Hz, 1H), 6.40 (d, J = 3.3 Hz, 1H), 4.64 (s, 2H), 2.09-2.04 (m, 1H), 1.88 (s, 3H), 1.85 (s, 3H).

### Compound 102. Tert-butyl4-(5-((4-((2-(dimethylphosphoryl)phenyl)amino)pyrimidin-2-yl)amino)-1H-indazol-3-yl)piperidine-1-carboxylate

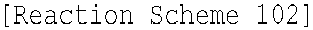

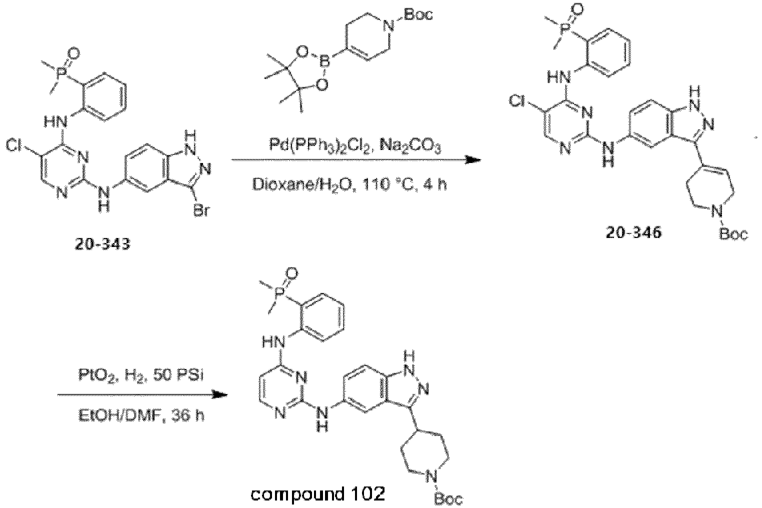

### 1) Tert-butyl 4-(5-((5-chloro-4-((2-(dimethylphosphoryl)phenyl)amino)pyrimidin-2-yl)amino)-1H-indazol-3-yl)-3,6-dihydropyridine-1(2H)-carboxylate.

To a solution of 20-343 (150 mg, 0.305 mmol) in dioxane/water (2 mL) were added tert-butyl4-(4,4,5,5-tetramethyl-1,3,2-dioxaborolan-2)-yl)-3,6-dihydropyridine-1(2H)-carboxylate (170 mg, 0.549 mmol) and then bis(triphenylphosphine)palladium dichloride (42.8 mg, 0.061 mmol) and sodium carbonate (97.0 mg, 0.915 mmol). The reaction mixture was heated at 110°C for 10 minutes with microwaves. TLC indicated the formation of a new spot. The solid was filtered and the solvent was evaporated in a vacuum. The crude mixture was subjected to purification by MPLC using 5 % MeOH:DCM as an eluent to afford Compound 20-346 as a gray solid (176 mg, 0.296 mmol, 72 %).

LCMS: 594.1 [M+H]⁺

¹H NMR (400 MHz, DMSO-d6) δ 12.89 (s, 1H), 11.21 (s, 1H), 9.35 (s, 1H), 8.59 (s, 1H), 8.27-8.14 (m, 2H), 7.62-7.49 (m, 2H), 7.46 (d, J = 8.9 Hz, 1H), 7.25 (s, 1H), 7.08 (t, J = 7.5 Hz, 1H), 6.29 (s, 1H), 3.91 (s, 2H), 3.61-3.48 (m, 2H), 2.71-2.60 (m, 2H), 1.81 (s, 3H), 1.78 (s, 3H), 1.43 (s, 9H).

### 2) Tert-butyl 4-(5-((4-((2-(dimethylphosphoryl)phenyl)amino)pyrimidin-2-yl)amino)-1H-indazol-3-yl)piperidine-1-carboxylate.

To a suspension of 20-346 (50.0 mg, 0.0843 mmol) in DMF (0.5 mL) and EtOH (4.5 mL) were added Pt/O₂ (7.5 mg, 15 mol%) at room temperature. The resulting mixture was shaken for 36 hours under a hydrogen atmosphere (50 psi) in a parr reactor. TLC indicated the formation of a new spot. The mixture was filtered through a celite bed and washed with EtOH, and the solvent was completely evaporated in a vacuum. The crude mixture was subjected to purification by MPLC using 15 % MeOH:MC as an eluent to afford Compound 102 as a purple solid (7.0 mg, 0.0124 mmol, 14 %).

¹H NMR (500 MHz, DMSO-d6) δ 12.53 (s, 1H), 10.95 (s, 1H), 9.18 (s, 1H), 8.52 (s, 1H), 8.13-8.02 (m, 2H), 7.66-7.53 (m, 2H), 7.43-7.32 (m, 2H), 7.09 (t, J = 7.4 Hz, 1H), 6.09 (d, J = 5.7 Hz, 1H), 4.10-3.97 (m, 2H), 3.19-3.09 (m, 1H), 2.89 (s, 2H), 1.93 (d, J = 13.3 Hz, 2H), 1.81 (s, 3H), 1.78 (s, 3H), 1.75-1.64 (m, 2H), 1.42 (s, 9H).

### Compound 103. Dimethyl(2-((2-((3-(piperidin-4-yl)-1H-indazol-5-yl)amino)pyrimidin-4-yl)amino)phenyl)phosphine oxide hydrochloride

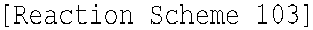

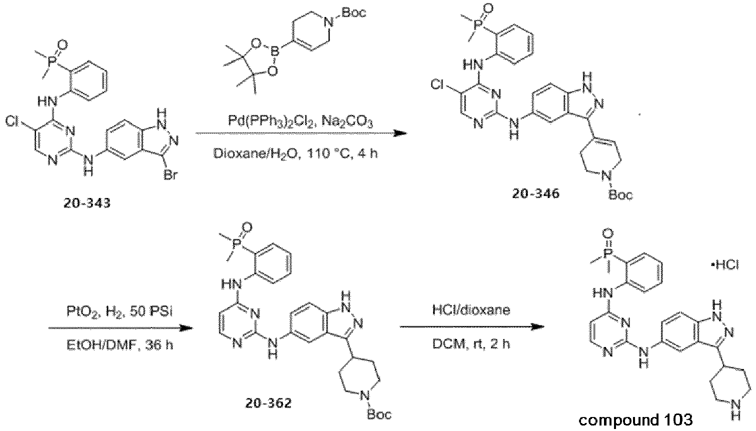

### 1) Tert-butyl 4-(5-((5-chloro-4-((2-(dimethylphosphoryl)phenyl)amino)pyrimidin-2-yl)amino)-1H-indazol-3-yl)-3,6-dihydropyridine-1(2H)-carboxylate.

To a solution of 20-343 (150 mg, 0.305 mmol) in dioxane/water (2 mL) were added tert-butyl4-(4,4,5,5-tetramethyl-1,3,2-dioxaborolan-2)-yl)-3,6-dihydropyridine-1(2H)-carboxylate (170 mg, 0.549 mmol) and then, bis(triphenylphosphine)palladium dichloride (42.8 mg, 0.061 mmol) and sodium carbonate (97.0 mg, 0.915 mmol). The reaction mixture was heated at 110°C for 10 minutes with microwaves. TLC indicated the formation of a new spot. The solid was filtered and the solvent was evaporated in a vacuum. The crude mixture was subjected to purification by MPLC using 5 % MeOH:DCM as an eluent to afford Compound 20-346 as a gray (176 mg, 0.296 mmol, 72 %).

LCMS: 594.1 [M+H]⁺

¹H NMR (400 MHz, DMSO-d6) δ 12.89 (s, 1H), 11.21 (s, 1H), 9.35 (s, 1H), 8.59 (s, 1H), 8.27-8.14 (m, 2H), 7.62-7.49 (m, 2H), 7.46 (d, J = 8.9 Hz, 1H), 7.25 (s, 1H), 7.08 (t, J = 7.5 Hz, 1H), 6.29 (s, 1H), 3.91 (s, 2H), 3.61-3.48 (m, 2H), 2.71-2.60 (m, 2H), 1.81 (s, 3H), 1.78 (s, 3H), 1.43 (s, 9H).

### 2) tert-butyl 4-(5-((4-((2-(dimethylphosphoryl)phenyl)amino)pyrimidin-2-yl)amino)-1H-indazol-3-yl)piperidine-1-carboxylate.

To a suspension of 20-346 (50.0 mg, 0.0843 mmol) in DMF (0.5 mL) and EtOH (4.5 mL) were added Pt/O₂ (7.5 mg, 15 mol%) at room temperature. The resulting mixture was shaken for 36 hours under a hydrogen atmosphere (50 psi) in a parr reactor. TLC indicated the formation of a new spot. The mixture was filtered through a celite bed. The filtrate was washed with EtOH and the solvent was completely evaporated in a vacuum. The crude mixture was subjected to purification by MPLC using 15 % MeOH:MC as an eluent to afford Compound 20-362 as a purple solid (7.0 mg, 0.0124 mmol, 14 %).

¹H NMR (500 MHz, DMSO-d6) δ 12.53 (s, 1H), 10.95 (s, 1H), 9.18 (s, 1H), 8.52 (s, 1H), 8.13-8.02 (m, 2H), 7.66-7.53 (m, 2H), 7.43-7.32 (m, 2H), 7.09 (t, J = 7.4 Hz, 1H), 6.09 (d, J = 5.7 Hz, 1H), 4.10-3.97 (m, 2H), 3.19-3.09 (m, 1H), 2.89 (s, 2H), 1.93 (d, J = 13.3 Hz, 2H), 1.81 (s, 3H), 1.78 (s, 3H), 1.75-1.64 (m, 2H), 1.42 (s, 9H).

### 3) Dimethyl(2-((2-((3-(piperidin-4-yl)-1H-indazol-5-yl)amino)pyrimidin-4-yl)amino)phenyl)phosphine oxide hydrochloride.

To a suspension of 20-362 (5.0 mg, 0.0084 mmol) in DCM 1 mL was added HCl (0.005 mL, 0.020 mmol) at room temperature, followed by stirring for 2 hours. When the reaction was completed as monitored by TLC, the solvent was completely evaporated in a vacuum. The residue was washed with diethylether to afford Compound 103 as a hygroscopic purple solid (2.6 mg, 0.0050 mmol, 59 %).

¹H NMR (400 MHz, DMSO-d6) δ 13.01 (s, 1H), 12.06 (s, 1H), 10.86 (s, 1H), 9.42-8.87 (m, 3H), 7.96 (d, J = 45.0 Hz, 3H), 7.72 (s, 1H), 7.56 (d, J = 8.8 Hz, 1H), 7.49-7.12 (m, 3H), 6.37 (d, J = 7.2 Hz, 1H), 3.42-3.23 (m, 3H), 3.03 (s, 2H), 2.08 (s, 3H), 1.80 (d, J = 13.6 Hz, 6H).

### Compound 104. (2-((5-Chloro-2-((3-(pyridin-4-yl)-1H-indazol-5-yl)amino)pyrimidin-4-yl)amino)phenyl)dimethylphosphine oxide

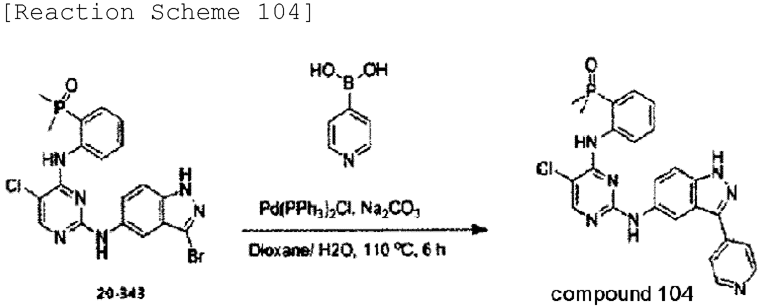

To a solution of 20-343 (50.0 mg, 0.102 mmol) in dioxane (5 mL) were added 4-pyridylboronic acid (20.7 mg, 0.122 mmol) and then, bis(triphenylphosphine)palladium dichloride (14.3 mg, 0.0204 mmol) and sodium carbonate (32.4 mg, 0.306 mmol). The reaction mixture was heated overnight at 110°C. TLC indicated the formation of a new spot. The solvent was evaporated in a vacuum. The residue was subjected to purification by MPLC using 10 % MeOH:MC as an eluent to afford Compound 104 as a yellow solid (6.10 mg, 0.012 mmol 12.3 %).

¹H NMR (300 MHz, Methanol-d4) δ 8.52-8.42 (m, 4H), 8.14 (s, 1H), 7.95-7.90 (m, 5H), 7.56 (d, J = 1.3 Hz, 2H), 7.52-7.43 (m, 1H), 7.11-7.04 (m, 1H), 6.99-6.90 (m, 1H), 1.92 (s, 3H), 1.88 (s, 3H).

### Compound 105. 4-(5-((5-Chloro-4-((2-(dimethylphosphoryl)phenyl)amino)pyrimidin-2-yl)amino)-1H-indazol-3-yl)benzonitrile

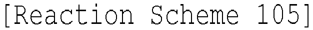

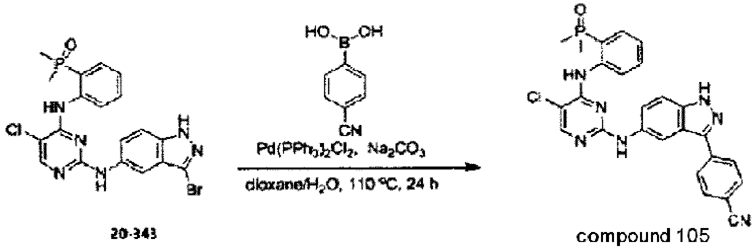

To a solution of 20-343 (50.0 mg, 0.102 mmol) in dioxane/water 9:1/H₂O (5 mL) were added 4-cyanophenylboronic acid (17.9 mg, 0.122 mmol) and then, bis(triphenylphosphine)palladium chloride (14.3 mg, 0.0204 mmol) and sodium carbonate (32.4 mg, 0.306 mmol). The reaction mixture was heated at 110°C for 24 hours. TLC indicated the formation of a new spot. The solvent was evaporated in a vacuum. The residue was subjected to purification by MPLC using 10 % MeOH:MC as an eluent to afford Compound 105 as a white solid (26.2 mg, 0.051 mmol, 50.0 %).

LC/MS:514.5 [M+H]⁺

¹H NMR (300 MHz, DMSO-d6) δ 13.38 (s, 1H), 11.23 (s, 1H), 9.48 (s, 1H), 8.62-8.49 (m, 1H), 8.36 (s, 1H), 8.25-8.18 (m, 3H), 7.83-7.74 (m, 1H), 7.73-7.44 (m, 4H), 7.12-6.85 (m, 2H), 1.82 (d, J = 3.5 Hz, 3H), 1.77 (d, J = 3.5 Hz, 3H).

### Compound 106. 1-(4-(5-((5-chloro-4-((2-(dimethylphosphoryl)phenyl)amino)pyrimidin-2-yl)amino)-1H-indazol-3-yl)phenyl)ethan-1-one

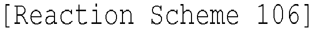

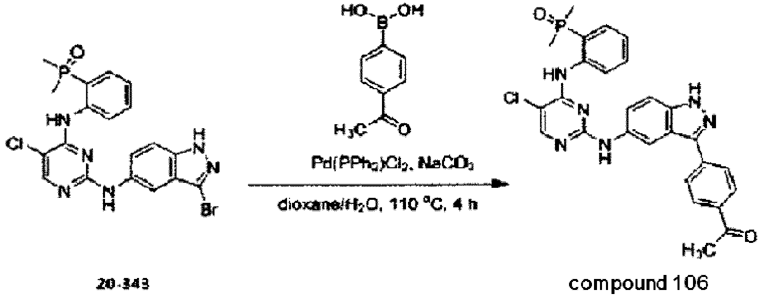

To a solution of 20-343 (50.0 mg, 0.102 mmol) in dioxane/water 9:1 (5 mL) were added (4-acetylphenyl)boronic acid (24.4 mg, 0.122 mmol) and then, bis(triphenylphosphine)palladium dichloride (14.3 mg, 0.0204 mmol), and sodium carbonate (32.4 mg, 0.306 mmol). The reaction mixture was heated at 110°C for 4 hours. TLC indicated the formation of a new spot. The solvent was evaporated in a vacuum. The residue was subjected to purification by MPLC using 10 % MeOH:MC as an eluent to afford Compound 106 as a yellow solid (28.4 mg, 0.053 mmol, 52.4 %).

LC/MS: 531.2 [M+H]⁺

¹H NMR (300 MHz, DMSO-d6) δ 13.33 (d, J = 21.4 Hz, 1H), 11.22 (d, J = 5.8 Hz, 1H), 9.49 (s, 1H), 8.72-8.39 (m, 2H), 8.23 (s, 1H), 8.01 (q, 2H), 7.93-7.82 (m, 1H), 7.71-7.36 (m, 4H), 7.18 -7.08 (m, 1H), 6.91 (s, 1H), 2.61 (s, 1H), 1.82 (d, J = 3.4 Hz, 3H), 1.77 (s, 2H).

### Compound 107. (2-((5-Chloro-2-((3-(3-chloro-4-fluorophenyl)-1H-indazol-5-yl)amino)pyrimidin-4-yl)amino)phenyl)dimethylphosphine oxide

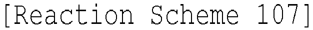

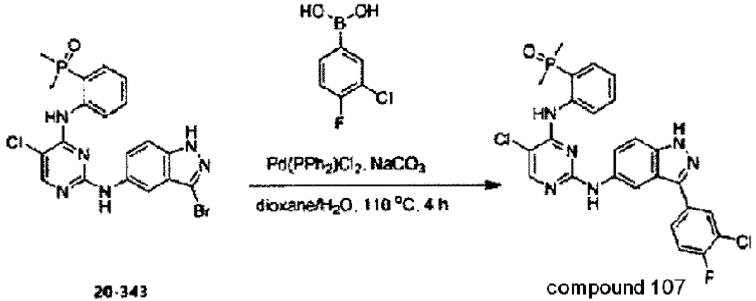

To a solution of 20-343 (50.0 mg, 0.102 mmol) in dioxane/water 9:1 (5 mL) were added (3-chloro-4-fluorophenyl)boronic acid (21.3 mg, 0.122 mmol) and then, bis(triphenylphosphine)palladium chloride (14.3 mg, 0.0204 mmol) and sodium carbonate (65.8 mg, 0.202 mmol) . The reaction mixture was heated overnight at 110°C. TLC indicated the formation of a new spot. The solvent was evaporated in a vacuum. The residue was subjected to purification by MPLC using 10 % MeOH:MC as an eluent to afford Compound 107 as a white solid (3.6 mg, 0.0067 mmol 6.58 %).

LC/MS:514.4 [M+H]⁺

¹H NMR (300 MHz, DMSO-d6) δ 13.29 (s, 1H), 11.24 (d, J = 17.3 Hz, 1H), 9.52-9.47 (m, 1H), 8.22 (d, J = 8.1 Hz, 1H), 8.04-7.93 (m, 1H), 7.90-7.84 (m, 1H), 7.66 (d, J = 9.1 Hz, 2H), 7.60 (d, J = 7.8 Hz, 0H), 7.58-7.39 (m, 3H), 7.15 (t, J = 7.6 Hz, 1H), 6.99-6.88 (m, 1H), 1.82 (s, 3H), 1.77 (s, 3H).

### Compound 108. (2-((5-chloro-2-((3-(4-hydroxyphenyl)-1H-indazol-5-yl)amino)pyrimidin-4-yl)amino)phenyl)dimethylphosphine oxide

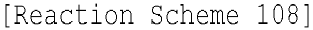

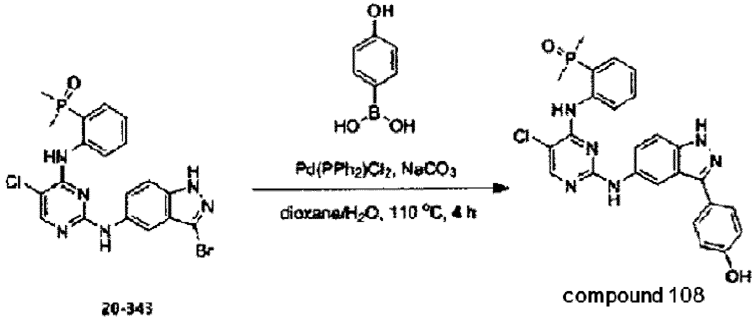

To a solution of 20-343 (50.0 mg, 0.102 mmol) in dioxane/water 9:1 (5 mL) were added (4-hydroxyphenyl)boronic acid (16.8 mg, 0.122 mmol) and then, bis(triphenylphosphine)palladium dichloride (14.3 mg, 0.0204 mmol) and sodium carbonate (65.8 mg, 0.202 mmol). The reaction mixture was heated at 110°C for 4 hours. TLC indicated the formation of a new spot. The solvent was evaporated in a vacuum. The residue was subjected to purification by MPLC using 10 % MeOH:MC as an eluent to afford Compound 108 as a white solid (18.2 mg, 0.036 mmol, 35.7 %).

LC/MS:505.31[M+H]⁺

¹H NMR (300 MHz, DMSO-d6) δ 12.92 (s, 1H), 11.17 (s, 1H), 9.56 (s, 1H), 9.36 (s, 1H), 8.57 (s, 1H), 8.19 (s, 2H), 7.77-7.66 (m, 2H), 7.65-7.58 (m, 1H), 7.57-7.44 (m, 3H), 7.08-6.97 (m, 1H), 6.84 (d, J = 8.5 Hz, 2H), 1.81 (s, 3H), 1.76 (s, 3H).

### Compound 109. (2-((5-Chloro-2-((3-(3-fluoro-4-methoxyphenyl)-1H-indazol-5-yl)amino)pyridin-4-yl)amino)phenyl)dimethylphosphine oxide

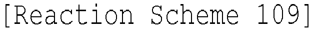

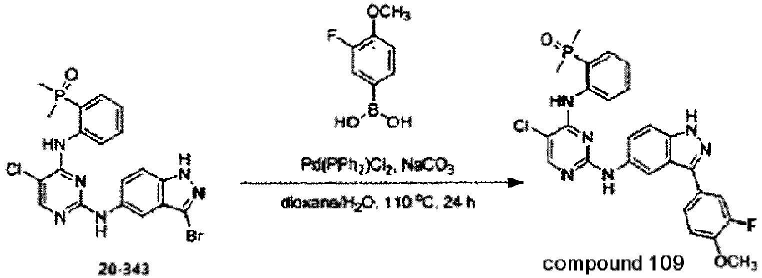

To a solution of 20-343 (50.0 mg, 0.102 mmol) in dioxane/water 9:1 (5 mL) were added (3-fluoro-4-methoxyphenyl)boronic acid (20.7 mg, 0.122 mmol) and then, bis(triphenylphosphine)palladium dichloride (14.3 mg, 0.0204 mmol) and sodium carbonate (65.8 mg, 0.202 mmol) . The reaction mixture was heated at 110°C for 24 hours. TLC indicated the formation of a new spot. The solvent was evaporated in a vacuum. The residue was subjected to purification by MPLC using 10 % MeOH:MC as an eluent to afford Compound 109 as a white solid (17.8 mg, 0.033 mmol, 32.8 %).

LC/MS:537.17[M+H]⁺

¹H NMR (300 MHz, DMSO-d6) δ 13.11 (s, 1H), 11.23 (s, 1H), 9.43 (s, 1H), 8.59 (s, 1H), 8.20 (s, 1H), 8.20 (s, 1H), 7.70-7.61 (m, 3H), 7.58-7.47 (m, 2H), 7.28-7.15 (m, 2H), 7.06-6.93 (m, 1H), 3.88 (s, 3H), 1.81 (s, 3H), 1.77 (s, 3H).

### Compound 110. (2-((5-Chloro-2-((3-(4-(trifluoromethoxy)phenyl)-1H-indazol-5-yl) amino)pyrimidin-4-yl)amino)phenyl)dimethylphosphine oxide

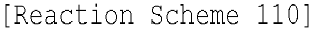

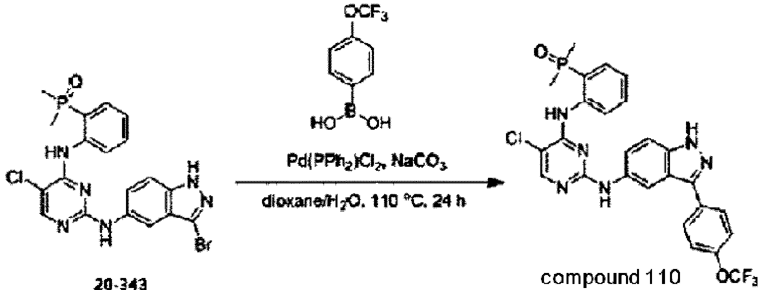

To a solution of 20-343 (50.0 mg, 0.102 mmol) in dioxane/water 9:1 (5 mL) were added (4-(trifluoromethoxy)phenyl)boronic acid (25.1 mg, 0.122 mmol) and then, bis(triphenylphosphine)palladium dichloride (14.3 mg, 0.0204 mmol) and sodium carbonate (65.8 mg, 0.202 mmol). The reaction mixture was heated at 110°C for 24 hours. TLC indicated the formation of a new spot. The solvent was evaporated in a vacuum. The residue mixture was subjected to purification by MPLC using 10 % MeOH:MC as an eluent to afford Compound 110 as a white solid (30.9 mg, 0.054 mmol, 53.4 %).

LC/MS:573.15 [M+H]⁺

¹H NMR (300 MHz, DMSO-d6) δ 13.26 (s, 1H), 11.24 (s, 1H), 9.45 (s, 1H), 8.64-8.53 (m, 1H), 8.34 (s, 1H), 8.21 (s, 1H), 8.01 (d, J = 8.6 Hz, 2H), 7.64 (d, J = 9.1 Hz, 1H), 7.58-7.46 (m, 2H), 7.42 (d, 2H), 7.17-6.87 (m, 2H), 1.81 (s, 3H), 1.76 (s, 3H).

### Compound 111. (2-((5-Chloro-2-((3-(4-chloro-3-(trifluoromethyl)phenyl)-1H-indazol-5-yl) amino)pyrimidin-4-yl)amino)phenyl)dimethylphosphine oxide

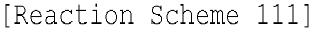

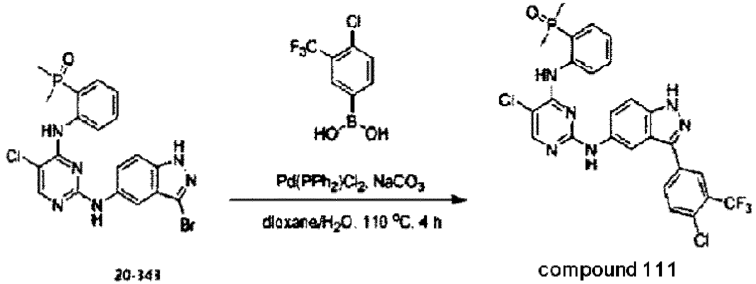

To a solution of were added 20-343 (50.0 mg, 0.102 mmol) in dioxane/water 9:1 (5 mL) were added (4-chloro-3-(trifluoromethyl)phenyl)boronic acid (72.1 mg, 0.122 mmol) and then, bis(triphenylphosphine)palladium dichloride (14.3 mg, 0.0204 mmol) and sodium carbonate (65.8 mg, 0.202 mmol). The reaction mixture was heated at 110°C for 4 hours. TLC indicated the formation of a new spot. The solvent was evaporated in a vacuum. The residue was subjected to purification by MPLC using 10 % MeOH:MC as an eluent to afford Compound 111 as a gray solid (29.2 mg, 0.049 mmol, 48.9 %).

LC/MS:591.08 [M+H]⁺

¹H NMR (300 MHz, DMSO-d6) δ 13.42 (s, 1H), 11.33 (s, 1H), 9.56 (s, 1H), 8.58 (s, 1H), 8.49 (s, 1H), 8.28 (d, J = 2.1 Hz, 1H), 8.21 (s, 1H), 8.12 (d, J = 8.4 Hz, 1H), 7.73-7.61 (m, 2H), 7.60-7.45 (m, 2H), 7.12-6.93 (m, 1H), 6.91-6.79 (m, 1H), 1.83 (s, 3H), 1.79 (s, 3H).

### Compound 112. (2-((5-Chloro-2-((3-(3-chloro-4-methylphenyl)-1H-indazol-5-yl)amino)pyrimidin-4-yl)amino)phenyl)dimethylphosphine oxide

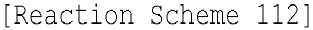

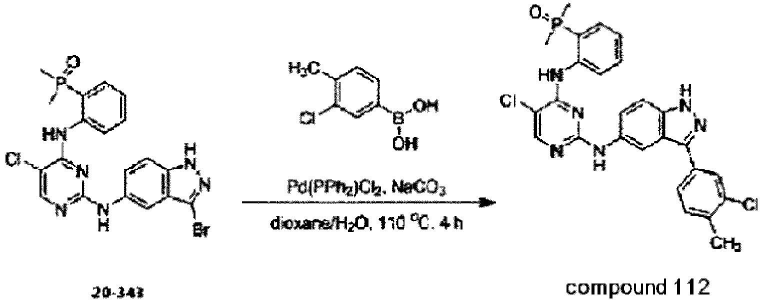

To a solution of 20-343 (50.0 mg, 0.102 mmol) in dioxane/water 9:1 (5 mL) were added 3-chloro-4-methylphenyl)boronic acid (20.8 mg, 0.122 mmol) and then, bis(triphenylphosphine)palladium dichloride (14.3 mg, 0.0204 mmol) and sodium carbonate (65.8 mg, 0.202 mmol). The reaction mixture was heated at 110°C for 4 hours. TLC indicated the formation of a new spot. The solvent was evaporated in a vacuum. The residue was subjected to purification by MPLC using 10 % MeOH:MC as an eluent to afford Compound 112 as a gray solid (42.2 mg, 0.079 mmol, 77.7 %).

LC/MS:537.24 [M+H]⁺

¹H NMR (300 MHz, DMSO-d6) δ 13.21 (s, 1H), 11.27 (s, 1H), 9.48 (s, 1H), 8.67-8.53 (m, 1H), 8.20 (s, 1H), 7.90-7.85 (m, 1H), 7.75 (dd, J = 7.9, 1.7 Hz, 1H), 7.68-7.60 (m, 2H), 7.60-7.51 (m, 3H), 7.39 (d, J = 8.0 Hz, 1H), 7.21-7.05 (m, 1H), 7.00-6.89 (m, 1H), 2.37 (s, 3H), 1.82 (s, 3H), 1.77 (s, 3H).

### Compound 113. (2-((5-Chloro-2-((3-(3-chloro-5-methoxyphenyl)-1H-indazol-5-yl)amino)pyrimidin-4-yl)amino)phenyl)dimethylphosphine oxide

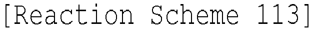

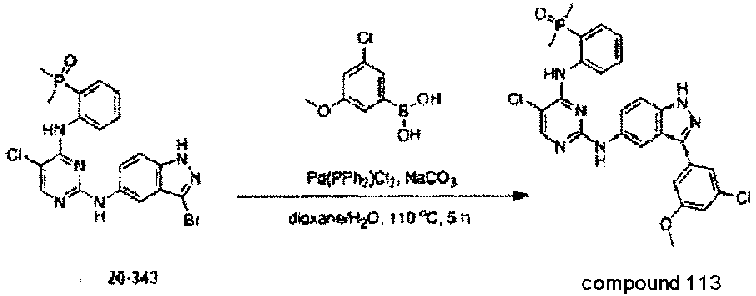

To a solution of 20-343 (50.0 mg, 0.102 mmol) in dioxane/water 9:1 (5 mL) were added 3-chloro-5-methoxybenzen boronic acid (22.7 mg, 0.122 mmol) and then, bis(triphenylphosphine)palladium dichloride (14.3 mg, 0.0204 mmol) and sodium carbonate (65.8 mg, 0.202 mmol) . The reaction mixture was heated 110°C for 5 hours. TLC indicated the formation of a new spot. The solvent was evaporated in a vacuum. The residue was subjected to purification by MPLC using 10 % MeOH:MC as an eluent to afford Compound 113 as a white solid (22.4 mg, 0.040 mmol, 40 %).

LC/MS:553.24[M+H]⁺

¹H NMR (300 MHz, DMSO-d6) δ 13.29 (s, 1H), 11.25 (d, J = 7.7 Hz, 1H), 9.49 (s, 1H), 8.62 (s, 1H), 8.35-8.21 (m, 1H), 8.19 (s, 1H), 7.78-7.70 (m, 1H), 7.60-7.47 (m, 3H), 7.42-7.37 (m, 1H), 7.24-7.11 (m, 1H), 7.04-6.97 (m, 2H), 3.84 (s, 3H), 1.83-1.79 (m, 3H), 1.79-1.75 (m, 3H) .

### Compound 114. (2-((5-Chloro-2-((3-(4-(methylsulfonyl)phenyl)-1H-indazol-5-yl)amino)pyrimidin-4-yl)amino)phenyl)dimethylphosphine oxide

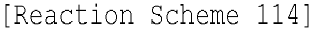

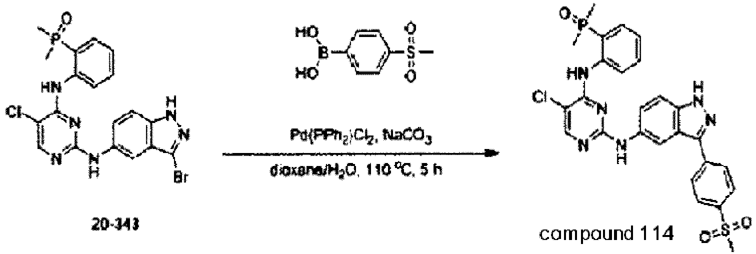

To a solution of 20-343 (50.0 mg, 0.102 mmol) in dioxane/water 9:1 (5 mL) were added (4-(methyl sulfonyl)phenyl)boronic acid (24.4 mg, 0.122 mmol) and then, bis(triphenylphosphine)palladium dichloride (14.3 mg, 0.0204 mmol) and sodium carbonate (65.8 mg, 0.202 mmol) . The reaction mixture was heated 110°C for 5 hours. TLC indicated the formation of a new spot. The solvent was evaporated in a vacuum. The residue was subjected to purification by MPLC using 10 % MeOH:MC as an eluent to afford Compound 114 as a white solid (27.9 mg, 0.049 mmol, 49 %).

LC/MS:567.21[M+H]⁺

¹H NMR (300 MHz, DMSO-d6) δ 13.43 (s, 1H), 11.22 (s, 1H), 9.54 (s, 1H), 8.56-8.48 (m, 2H), 8.15-8.06 (m, 2H), 7.95-7.86 (m, 2H), 7.67-7.53 (m, 3H), 7.55-7.40 (m, 2H), 6.88-6.83 (m, 1H), 3.25 (s, 3H), 1.84 (s, 3H), 1.79 (s, 3H).

### Compound 115. (2-((5-Chloro-2-((3-(3-fluoro-5-isopropoxyphenyl)-1H-indazol-5-yl)amino)pyrimidin-4-yl)amino)phenyl)dimethylphosphine oxide

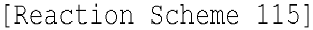

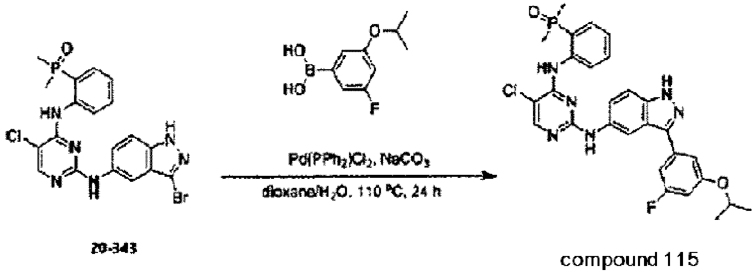

To a solution of 20-343 (50.0 mg, 0.102 mmol) in dioxane/water 9:1 (5 mL) were added (3-fluoro-5-isopropoxyphenyl)boronic acid (24.2 mg, 0.122 mmol) and then, bis(triphenylphosphine)palladium dichloride (14.3 mg, 0.0204 mmol) and sodium carbonate (65.8 mg, 0.202 mmol) . The reaction mixture was heated overnight at 110°C. TLC indicated the formation of a new spot. The solvent was evaporated in a vacuum. The residue was subjected to purification by MPLC using 10 % MeOH:MC as an eluent to afford Compound 115 as a gray solid (38.1 mg, 0.067 mmol, 0.67 %).

LC/MS:565.26 [M+H]⁺

¹H NMR (300 MHz, DMSO-d6) δ 13.25 (s, 1H), 11.25 (s, 1H), 9.47 (s, 1H), 8.71 -8.53 (m, 1H), 8.24 (s, 1H), 8.18 (s, 1H), 7.78-7.67 (m, 1H), 7.61-7.48 (m, 2H), 7.27 (s, 1H), 7.20 (d, J = 9.6 Hz, 2H), 7.07-6.95 (m, 1H), 6.84-6.76 (m, 1H), 4.77-4.65 (m, 1H), 1.81 (s, 3H), 1.76 (s, 3H), 1.31 (d, J = 6.0 Hz, 6H).

### Compound 116. 3-(5-((5-Chloro-4-((2-(dimethylphosphoryl)phenyl)amino)pyrimidin-2-yl)amino)-1H-indazol-3-yl)benzonitrile

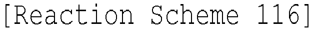

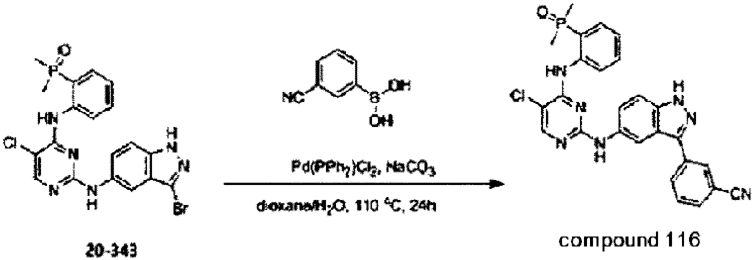

To a solution of 20-343 (50.0 mg, 0.102 mmol) in dioxane/water 9:1 (5 mL) were added (3-cyanophenyl)boronic acid (17.9 mg, 0.122 mmol) and then, bis(triphenylphosphine)palladium dichloride (14.3 mg, 0.0204 mmol) and sodium carbonate (65.8 mg, 0.202 mmol). The reaction mixture was heated at 110°C for 24 hours. TLC indicated the formation of a new spot. The solvent was evaporated in a vacuum. The residue was subjected to purification by MPLC using 10 % MeOH:MC as an eluent to afford Compound 116 as a gray solid (34.2 mg, 0.067 mmol, 66 %).

LC/MS:541.09[M+H]⁺

¹H NMR (400 MHz, DMSO-d6) δ 13.39 (s, 1H), 11.33 (s, 1H), 9.55 (s, 1H), 8.55 (s, 1H), 8.36 (d, J = 1.8 Hz, 1H), 8.23 (d, J = 10.0 Hz, 3H), 7.82-7.76 (m, 1H), 7.71-7.62 (m, 2H), 7.58 (d, J = 8.9 Hz, 1H), 7.52 (dd, J = 14.0, 7.6 Hz, 1H), 7.15-6.99 (m, 1H), 6.94 (s, 1H), 1.80 (d, J = 13.5 Hz, 6H).

### Compound 117. Tert-butyl 4-(4-(5-((5-chloro-4-((2-(dimethylphosphoryl)phenyl)amino)pyrimidin-2-yl)amino)-1H-indazol-3-yl) phenyl)piperazine-1-carboxylate

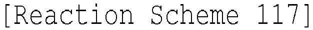

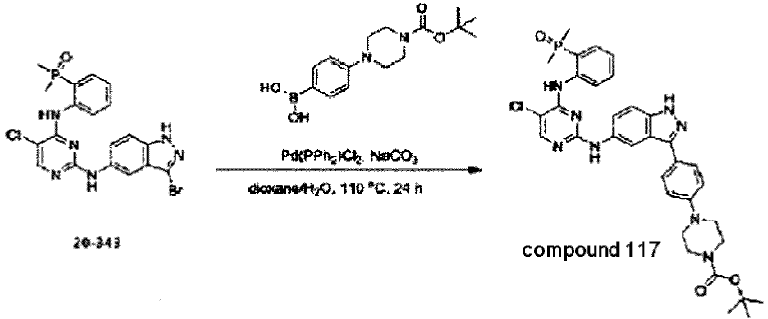

To a solution of 20-343 (50.0 mg, 0.102 mmol) in dioxane/water 9:1 (5 mL) were added 4-(4-Boc-piperazino)phenyl boronic acid (37.4 mg, 0.122 mmol) and then, bis(triphenylphosphine)palladium dichloride (14.3 mg, 0.0204 mmol) and sodium carbonate (65.8 mg, 0.202 mmol) . The reaction mixture was heated at 110°C for 24 hours. TLC indicated the formation of a new spot. The solvent was evaporated in a vacuum. The residue was subjected to purification by MPLC using 10 % MeOH:MC as an eluent to afford Compound 117 as a gray solid (37.4 mg, 0.056 mmol, 55 %).

LC/MS: 673.33[M+H]⁺

¹H NMR (300 MHz, DMSO-d6) δ 12.93 (s, 1H), 11.25 (s, 1H), 9.39 (s, 1H), 8.72-8.53 (m, 1H), 8.28-8.15 (m, 2H), 7.78 (d, J = 8.5 Hz, 2H), 7.68-7.59 (m, 1H), 7.58-7.51 (m, 1H), 7.51-7.46 (m, 1H), 7.18-6.96 (m, 4H), 3.54-3.44 (m, 4H), 3.22-3.12 (m, 4H), 1.81 (s, 3H), 1.76 (s, 3H), 1.44 (s, 9H).

### Compound 118. (2-((5-chloro-2-((3-(3-hydroxyphenyl)-1H-indazol-5-yl)amino)pyrimidin-4-yl)amino)phenyl)dimethylphosphine oxide

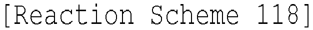

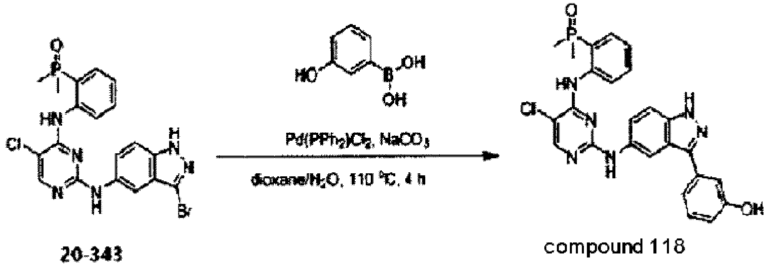

To a solution of 20-343 (50.0 mg, 0.102 mmol) in dioxane/water 9:1 (5 mL) were added (3-hydroxyphenyl)boronic acid (16.8 mg, 0.122 mmol) and then, bis(triphenylphosphine)palladium dichloride (14.3 mg, 0.0204 mmol) and sodium carbonate (65.8 mg, 0.202 mmol) . The reaction mixture was heated at 110°C for 24 hours. TLC indicated the formation of a new spot. The solvent was evaporated in a vacuum. The residue was subjected to purification by MPLC using 10 % MeOH:MC as an eluent to afford Compound 118 as a white solid (18.7 mg, 0.037 mmol, 37 %).

LCMS: 505.2 [M+H]⁺

¹H NMR (400 MHz, DMSO-d6) δ 13.07 (s, 1H), 11.21 (s, 1H), 9.48 (s, 1H), 8.23-8.17 (m, 2H), 8.09 (d, J = 1.2 Hz, 1H), 7.73-7.61 (m, 2H), 7.55-7.47 (m, 2H), 7.39-7.31 (m, 2H), 7.25 (t, J = 7.8 Hz, 1H), 7.22-7.16 (m, 1H), 7.06-6.97 (m, 1H), 6.75 (d, J = 8.5 Hz, 1H), 1.79 (s, 3H), 1.76 (s, 3H).

### Compound 119. (2-((5-Chloro-2-((3-(4-(piperazine-1-yl)phenyl)-1H-indazol-5-yl)amino)pyrimidin-4-yl)amino)phenyl)dimethylphosphine oxide

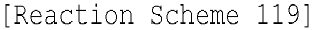

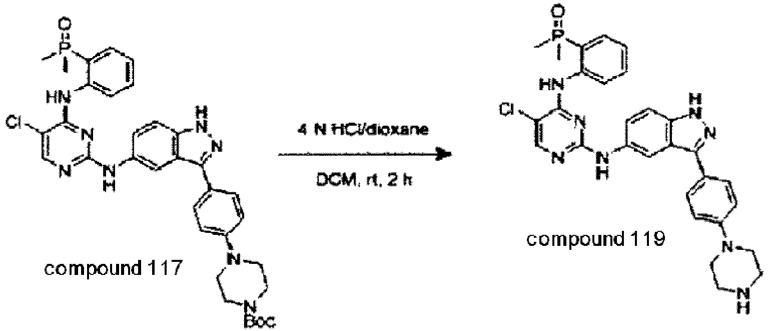

To a solution of Compound 117 (80.0 mg, 0.134 mmol) in 5 mL of DCM was added 4N HCl in dioxane (0.083 mL, 0.335 mmol) at room temperature before stirring for 2 hours. When the reaction was completed as monitored by TLC, the solvent was completely evaporated in a vacuum. The residue was dissolved in 15 mL of water and washed with DCM (15 mL×2). The aqueous layer was alkalinized with sodium bicarbonate, followed by extraction with DCM (20 mL×3). The organic layers thus obtained were pooled and dried over sodium sulfate and the solvent was evaporated in a vacuum to afford Compound 119 as a gray solid (56.0 mg, 0.112 mmol, 84 %).

¹H NMR (400 MHz, DMSO-d6) δ 12.92 (s, 1H), 11.23 (s, 1H), 9.39 (s, 1H), 8.61 (s, 1H), 8.28-8.17 (m, 2H), 7.75 (d, J = 8.6 Hz, 2H), 7.61 (d, J = 9.0 Hz, 1H), 7.56-7.46 (m, 2H), 7.21-7.07 (m, 1H), 7.05-6.94 (m, 3H), 3.16-3.02 (m, 4H), 2.91-2.79 (m, 4H), 1.80 (s, 3H), 1.77 (s, 3H).

### <EXAMPLE 2. Assay for Enzyme Inhibition and Anticancer Activity of Inventive Compounds>

Compounds 1 to 119 synthesized in Example 1 according to the present disclosure were assayed for inhibitory activity against MLK3 / LRRK2 enzymes and anticancer activity against HepG2 / HS746T cancer cells, and the results are summarized in Table 1, below.

Inhibitory activity against MLK3 and LRRK2 enzymes was evaluated in Eurofins. In brief, human recombinant MLK3 protein was mixed with the compounds in a reaction buffer (8 mM MOPS (pH 7.0), 0.2 mM EDTA, 0.33 mg/mL myelin basic protein, 5 mM DTT) to which a mixture of 10 mM magnesium acetate and gamma-33P-ATP was then added before incubation at room temperature for 120 minutes. Afterward, the reaction was terminated with 0.5 % phosphoric acid. Ten µL of the total mixture was spotted onto a P30 filtermat which was then washed four times for 4 minutes with 0.425 % phosphoric acid and once in methanol prior to scintillation counting. Values for the activity of MLK3 were expressed as % of the control. For the activity of LRRK2, human recombinant LRRK2 protein was mixed with the compounds in a reaction buffer (50 mM HEPES (pH 8.0), 0.2 mM EDTA, 0.01 % Brij-35, 1 % (w/v) BSA, 5 mM DTT, 250 uM substrate peptide RLGRDKYKTLRQIRQ) and then reacted with a mixture of 10 mM magnesium acetate and gamma-33P-ATP at room temperature for 40 minutes. Afterward, the reaction was terminated with 0.5 % phosphoric acid. Ten µL of the total mixture was spotted onto a P30 filtermat which was then washed four times for 4 minutes with 0.425 % phosphoric acid and once in methanol prior to scintillation counting. Values for the activity of LRRK2 were expressed as % of the control.

Assay for anticancer activity was conducted as follows. HepG2 and HS746T cancer cells were purchased from the Korean Cell Line Bank and cultured according to the instruction provided thereby. One hundred µL of a culture of the cells were seeded at a density of 4,000 cells/well into 96-well plates. After 24 hours, the cells were treated with the compounds for 72 hours. Then, cell viability was assayed using CCK-8 and expressed as % of the control.

**[TABLE 1]**

| **compound no**. | **kinase activity assay (%)** | | **Cell viability (% of Con. 10µM)** | | |
|---|---|---|---|---|---|
| | **MLK3** | **LRRK2** | | | |
| | IC₅₀ | IC₅₀ | HepG2 | HS746T | |
| compound 2 | + | + | NT | | |
| compound 3 | + | + | | | |
| compound 4 | + | ++ | | | |
| compound 5 | + + | + | | | |
| compound 6 | + | + | | | |
| compound 7 | + | + | 75 | 103 | |
| compound 9 | - | - | 110 | 104 | |
| compound 10 | - | - | 91 | 120 | |
| compound 11 | + + + | + + + | 28 | 27 | |
| compound 12 | - | - | 85 | 97 | |
| compound 14 | - | - | 108 | 110 | |
| compound 15 | - | - | 116 | 110 | |
| compound 16 | - | - | 82 | 64 | |
| compound 17 | + + + | + + + | 35 | 17 | |
| compound 18 | + + | + + + | 32 | 16 | |
| compound 19 | + + | + + | 34 | 25 | |
| compound 23 | + + | + + | 27 | 26 | |
| compound 27 | + | - | 81 | 74 | |
| compound 30 | - | - | 102 | 85 | |
| compound 37 | - | - | 100 | 90 | |
| compound 38 | - | + | 73 | 90 | |
| compound 41 | + + + | + + | 26 | 33 | |
| compound 42 | + + + | + + + | 48 | 30 | |
| compound 44 | + + + | + + + | 53 | 17 | |
| compound 46 | + + + | + + + | 26 | 13 | |
| compound 47 | + + + | + + + | 24 | 8 | |
| compound 48 | + + + | + + + | 37 | 9 | |
| compound 49 | + + + | + + + | 31 | 0.3 | |
| compound 50 | + + + | + + + | 20 | 2 | |
| compound 51 | + + + | + + + | 24 | 2 | |
| compound 52 | + + + | + + + | 21 | 1 | |
| compound 53 | + + + | + + + | 23 | 14 | |
| compound 54 | + + + | + + + | 17 | 4 | |
| compound 55 | + + + | + + + | 19 | 2 | |
| compound 58 | - | + | 83 | 85 | |
| compound 62 | + + + | + + + | 26 | 13 | |
| compound 64 | + + + | + + + | 23 | 29 | |
| compound 65 | + + + | + + + | 20 | 23 | |
| compound 66 | + + + | + + + | 31 | 22 | |
| compound 67 | + + + | + + + | 33 | 25 | |
| compound 68 | + + | + + + | 43 | 64 | |
| compound 69 | + + + | + + + | 29 | 23 | |
| compound 70 | + + | + + + | 30 | 25 | |
| compound 71 | + + | + + + | 63 | 83 | |
| compound 72 | + + | + + + | 35 | 9 | |
| compound 73 | + + + | + + + | 19 | 0.5 | |
| compound 75 | + + | + + + | 28 | 21 | |
| compound 78 | + + | + + + | NT | 17 | |
| compound 79 | + + | + + + | | 19 | |
| compound 81 | + + | + + + | | 13 | |
| compound 82 | + + | + + | | 18 | |
| compound 83 | + + + | + + + | | 11 | |
| compound 84 | + + + | + + + | | 11 | |
| compound 85 | + + + | + + + | | 6 | |
| compound 91 | + + + | + + + | | 5.8 | |
| compound 92 | + + + | + + + | | 14.1 | |
| compound 93 | + + + | + + + | 42 | 18 | |
| compound 94 | + + + | + + + | 30 | 15 | |
| compound 95 | + + + | + + + | 39 | 19 | |
| compound 96 | + + + | + + + | 15 | 10 | |
| compound 97 | + + + | + + + | 21 | 11 | |
| compound 98 | + + + | + + + | 27 | 10 | |
| compound 99 | + + + | + + + | 26 | 9 | |
| compound 101 | + + + | + + | 56 | 13 | |
| compound 104 | + + + | + + + | 30 | 11 | |
| compound 105 | + + + | + + | 55 | 23 | |
| compound 106 | + + + | + + | 75 | 17 | |
| compound 107 | + + + | + + + | 63 | 11 | |
| compound 108 | + + + | + + | 67 | 8 | |
| compound 109 | + + + | + + | 53 | 25 | |
| compound 110 | + + + | + + | 24 | 12 | |
| compound 111 | + + + | + + | | 7 | 7 |
| compound 112 | + + + | + + | | 6 | 7 |
| compound 113 | + + + | + + | | 16 | 26 |
| compound 114 | + + + | + + | | 68 | 33 |
| compound 115 | + + + | + + + | | 50 | 10 |
| compound 116 | + + + | + + | | 32 | 19 |
| compound 117 | + + + | + + | | 68 | 20 |
| compound 118 | + + + | + + | | 43 | 5 |
| - IC₅₀(Inhibitory concentration 50%): | | | + + + (0,001~0,01 µM ), | | |
| | | | + + (0,01~0.1 µM ), | | |
| | | | + (0.1~1 µM ), | | |
| | | | - (> 1 µM ) | | |
| - NT: not tested | | | | | |

Referring to Table 1, when used at a density of 0.001-1 µM, the pyridine compounds of the present disclosure each exhibited inhibitory activity against both MLK3 and LRRK2 and excellent anticancer activity against HepG2 / HS746T cancer cells.

Therefore, the compounds of the present disclosure are understood to have advantageous applications in preventing or treating diseases associated with activity of MLK3 and LRRK2, including cancers, virus infectious diseases, Parkinson's disease, non-alcoholic steatohepatitis, and tuberculosis.

### <FORMULATION EXAMPLE 1. Preparation of Powder>

Compound 10 of the present disclosure (5-chloro-N-(2-(isopropylsulfonyl)phenyl)-2-(1H-pyrazol-4-yl)pyrimidine-4-amine) 2 g, and lactose 1 g were mixed and loaded into an airtight sac to give a powder.

### <FORMULATION EXAMPLE 2. Preparation of Tablet>

Compound 10 of the present disclosure (5-chloro-N-(2-(isopropylsulfonyl)phenyl)-2-(1H-pyrazol-4-yl)pyrimidine-4-amine) 100 mg, microcrystalline cellulose 100 mg, lactose hydrate 60 mg, low-substituted hydroxypropyl cellulose 20 mg, and magnesium stearate 2 mg were mixed and compressed into a tablet according to a typical tableting method.

### <FORMULATION EXAMPLE 3. Preparation of Capsule>

Compound 47 of the present disclosure (2-((5-chloro-2-((1,2,3,4-tetrahydroisoquinolin-7-yl)amino)pyrimidin-4-yl)amino)phenyl)dimethylphosphine oxide 100 mg, microcrystalline cellulose 100 mg, lactose hydrate 60 mg, low-substituted hydroxypropyl cellulose 20 mg, and magnesium stearate 2 mg were mixed and loaded into a gelatin capsule according to a typical method to give a capsule.

### <FORMULATION EXAMPLE 4. Preparation of Pill>

Compound 47 of the present disclosure (2-((5-chloro-2-((1,2,3,4-tetrahydroisoquinolin-7-yl)amino)pyrimidin-4-yl)amino)phenyl)dimethylphosphine oxide 90 mg, glutinous rice starch 5mg and purified water 5 mg were mixed together with a small amount of anti-hygroscopic additives including dextrin, maltodextrin, corn starch, and microcrystalline cellulose (MCC) and the mixture was prepared into a pill 100 mg according to a typical method.

### <FORMULATION EXAMPLE 5. Preparation of Injection>

Compound 47 of the present disclosure (2-((5-chloro-2-((1,2,3,4-tetrahydroisoquinolin-7-yl)amino)pyrimidin-4-yl)amino)phenyl)dimethylphosphine oxide 10 mg was mixed together a suitable amount of sterile distilled water for injection, and a suitable amount of a pH adjuster and the mixture was put into an ample (2 ml) according to a conventional method.

## Claims

1. A compound, represented by the following Chemical Formula 1, an optical isomer thereof, or a pharmaceutically acceptable salt thereof: 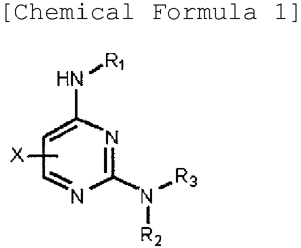 wherein,
R₁ is at least one substituent selected from the group consisting of a substituted or unsubstituted C₄-C₁₂ aryl, a substituted or unsubstituted C₄-C₁₂ heterocycloalkyl, a substituted or unsubstituted C₄-C₁₂ heterobicycloalkyl, a substituted or unsubstituted C₄-C₁₂ cycloalkyl, and a substituted or unsubstituted C₄-C₁₂ heterocycloaryl;
wherein the substituted aryl, heterocycloalkyl, cycloalkyl, or heteroaryl has as a substituent at least one selected from the group consisting of a hydrogen atom, 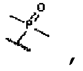 and 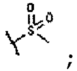
X is at least one selected from the group consisting of a hydrogen atom, halogen, hydroxy, a C₁-C₆ alkyl, and a C₁-C₆ alkoxy;
R₂ is at least one substituent selected from the group consisting of a hydrogen atom, a C₁-C₄ alkyl, and acetyl;
R₃ is one substituent selected from the group consisting of a substituted or unsubstituted C₄-C₁₂ aryl, a substituted or unsubstituted C₄-C₁₂ heterobicycloalkyl, and a substituted or unsubstituted C₄-C₁₂ heterobicycloaryl,
wherein the substituted aryl, heterocycloalkyl, or heteroaryl has as a substituent at least one selected from the group consisting of a hydrogen atom, halogen, cyano, aldehyde, CF₃, SH, trifluoroketone, a C₁-C₆ alkyl, a C₁-C₆ alkoxy, a substituted or unsubstituted C₄-C₁₂ cycloalkyl, a substituted or unsubstituted C₄-C₁₂ heterocycloalkyl, a substituted or unsubstituted C₄-C₁₂ aryl, and a substituted or unsubstituted C₄-C₁₂ heteroaryl, and
wherein the substituted cycloalkyl, heterocycloalkyl, aryl, or heteroaryl has as a substituent at least one selected from the group consisting of a hydrogen atom, halogen, NH₂, hydroxy, CF₃, NO₂, methanesulfonate, Boc(tert-butoxycarbonyl), methylketone, aldehyde, a C₁-C₆ hydroxyalkyl, a C₁-C₆ alkyloxy, piperazine, and a C₁-C₆ alkyl.

2. A compound represented by any one of the following Chemical Formulas 2 to 9, an optical isomer thereof, or pharmaceutically acceptable salt thereof: 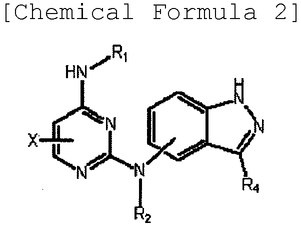 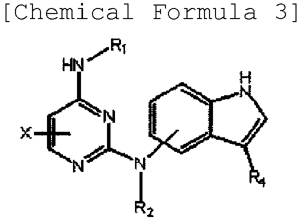 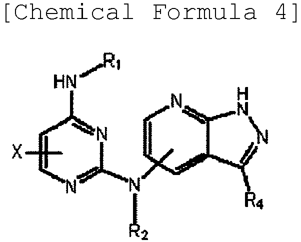 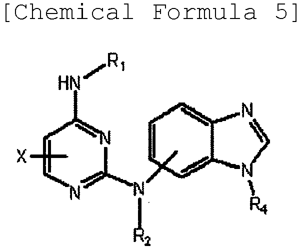 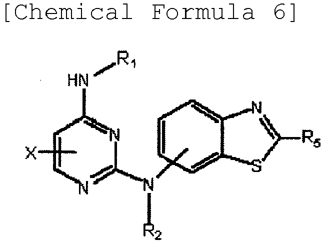 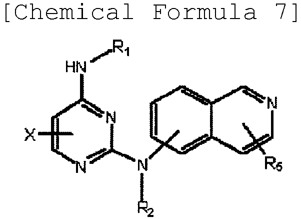 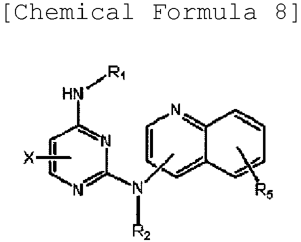 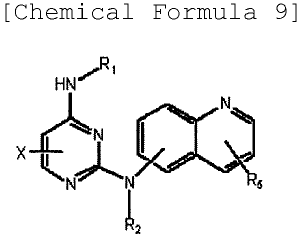 wherein,
R₁ is at least one substituent selected from substituted or unsubstituted C₄-C₁₂ aryls;
wherein the substituted aryl has as a substituent at least one selected from the group consisting of a hydrogen atom, 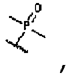 and 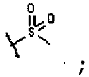
X is at least one selected from the group consisting of a hydrogen atom, halogen, hydroxy, a C₁-C₆ alkyl, and a C₁-C₆ alkoxy;
R₂ is at least one selected from the group consisting of a hydrogen atom, a C₁-C₄ alkyl, and acetyl;
R₄ is at least one selected from the group consisting of a hydrogen atom, halogen, cyano, aldehyde, CF₃, SH, trifluoroketone, C₁-C₆ alkyl, C₁-C₆ alkoxy, a substituted or unsubstituted C₄-C₁₂ cycloalkyl, a substituted or unsubstituted C₄-C₁₂ heterocycloalkyl, a substituted or unsubstituted C₄-C₁₂ aryl, and a substituted or unsubstituted C₄-C₁₂ heteroaryl,
wherein the substituted cycloalkyl, heterocycloalkyl, aryl, or heteroaryl has as a substituent at least one selected from the group consisting of a hydrogen atom, halogen, NH₂, hydroxy, CF₃, NO₂, methanesulfonate, Boc(tert-butoxycarbonyl), methylketone, aldehyde, a C₁-C₆ hydroxyalkyl, a C₁-C₆ alkyloxy, piperazine, and a C₁-C₆ alkyl;
R₅ is selected from a hydrogen atom, halogen, a C₁-C₄ alkyl, a C₁-C₄ alkoxy, hydroxy, a thio C₁-C₄ alkyl, and amino.

3. A compound represented by the following Chemical Formula 10, an optical isomer thereof, or a pharmaceutically acceptable salt thereof: 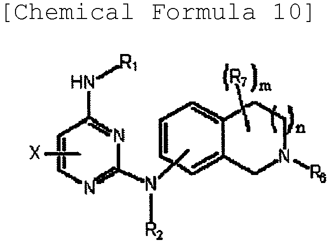 wherein,
R₁ is at least one substituent selected from the group consisting of substituted or unsubstituted C₄-C₁₂ aryls;
wherein the substituted aryl has as a substituent at least one selected from the group consisting of a hydrogen atom, 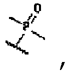 and 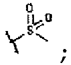
X is at least one substituent selected from the group consisting of a hydrogen atom, halogen, hydroxy, a C₁-C₆ alkyl, and a C₁-C₆ alkoxy;
R₂ is at least one substituent selected from the group consisting of a hydrogen atom, a C₁-C₄ alkyl, and acetyl;
R₆ is at least one substituent selected from the group consisting of a hydrogen atom, a C₁-C₆ alkyl, a halo C₁-C₆ alkylketone, trifluoroketone, acetyl, and Boc(tert-butoxycarbonyl);
R₇ is at least one substituent selected from the group consisting of a hydrogen atom, a C₁-C₆ alkyl, halogen, and hydroxy;
n is an integer of 0 or 1; and
m is an integer of 0, 1, or 2.

4. The compound of any one of claims 1 to 3, an optical isomer thereof, and a pharmaceutically acceptable salt thereof, wherein the compound is selected from the group consisting of:
5-chloro-N²-(3,5-dimethoxyphenyl)-N⁴-(2-(isopropylsulfonyl)phenyl)pyrimidine-2,4-diamine (Compound 1);
5-chloro-N²-(3,5-dichlorophenyl)-N⁴-(2-(isopropylsulfonyl)phenyl)pyrimidine-2,4-diamine (Compound 2);
5-chloro-N²-(3,5-dimethylphenyl)-N⁴-(2-(isopropylsulfonyl)phenyl) pyrimidine-2,4-diamine (Compound 3);
N²-(4-bromo-3,5-difluorophenyl)-5-chloro-N⁴-(2-(isopropylsulfonyl)phenyl)pyrimidine-2,4-diamine (Compound 4);
N²-(3,5-bis(trifluoromethyl)phenyl)-5-chloro-N⁴-(2-(isopropylsulfonyl)phenyl)pyrimidine-2,4-diamine (Compound 5);
5-chloro-N²-(3,5-difluorophenyl)-N⁴-(2-(isopropylsulfonyl)phenyl)pyrimidine-2,4-diamine (Compound 6);
(2-((5-chloro-2-((5,5,8,8-tetramethyl-5,6,7,8-tetrahydronaphthalen-2-yl)amino)pyrimidin-4-yl)amino)phenyl)dimethylphosphine oxide (Compound 7);
(2-((5-chloro-2-(quinolin-5-ylamino)pyrimidin-4-yl)amino)phenyl)dimethylphosphine oxide (Compound 8);
(2-((2-(5-amino-3,4-dihydroisoquinolin-2(1H)-yl)-5-chloropyrimidin-4-yl)amino)phenyl)dimethylphosphine oxide (Compound 9);
(2-((5-chloro-2-((2-methylpyridin-4-yl)amino)pyrimidin-4-yl)amino)phenyl)dimethylphosphine oxide (Compound 10);
(2-((2-(benzo[d] thiazol-6-ylamino)-5-chloropyrimidin-4-yl)amino)phenyl)dimethylphosphine oxide (Compound 11);
(2-((5-chloro-2-((4-fluorobenzyl)amino)pyrimidin-4-yl)amino)phenyl)dimethylphosphine oxide (Compound 12);
(2-((5-chloro-2-(cyclopentylamino)pyrimidin-4-yl)amino)phenyl)dimethylphosphine oxide (Compound 13);
(2-((2-(benzylamino)-5-chloropyrimidin-4-yl)amino)phenyl)dimethylphosphine oxide (Compound 14);
(2-((5-chloro-2-(pyridin-4-ylamino)pyrimidin-4-yl)amino)phenyl)dimethylphosphine oxide (Compound 15);
(2-((5-chloro-2-(cyclohexylamino)pyrimidin-4-yl)amino)phenyl)dimethylphosphine oxide (Compound 16);
(2-((5-chloro-2-((2-methylbenzo[d] thiazol-6-yl)amino)pyrimidin-4-yl)amino)phenyl)dimethylphosphine oxide (Compound 17);
(2-((5-chloro-2-(quinolin-6-ylamino)pyrimidin-4-yl)amino)phenyl)dimethylphosphine oxide (Compound 18);
(2-((5-chloro-2-(quinolin-3-ylamino)pyrimidin-4-yl)amino)phenyl)dimethylphosphine oxide (Compound 19);
(2-((5-chloro-2-(quinolin-8-ylamino)pyrimidin-4-yl)amino)phenyl)dimethylphosphine oxide (Compound 20);
(2-((5-chloro-2-(isoquinolin-5-ylamino)pyrimidin-4-yl)amino)phenyl)dimethylphosphine oxide (Compound 21);
(2-((5-chloro-2-((2,3-dihydro-1H-inden-2-yl)amino)pyrimidin-4-yl)amino)phenyl)dimethylphosphine oxide (Compound 22);
(2-((5-chloro-2-((2-mercaptobenzo[d] thiazol-6-yl)amino)pyrimidin-4-yl)amino)phenyl)dimethylphosphine oxide (Compound 23);
(2-((5-chloro-2-isopropoxypyrimidin-4-yl)amino)phenyl)dimethylphosphine oxide (Compound 24);
(2-((5-chloro-2-((6-ethoxybenzo[d] thiazol-2-yl)amino)pyrimidin-4-yl)amino)phenyl)dimethylphosphine oxide (Compound 25);
(2-((5-chloro-2-((4-methoxybenzo[d] thiazol-2-yl)amino)pyrimidin-4-yl)amino)phenyl)dimethylphosphine oxide (Compound 26);
(2-((5-chloro-2-((4-methylbenzo[d] thiazol-2-yl)amino)pyrimidin-4-yl)amino)phenyl)dimethylphosphine oxide (Compound 27);
(2-((5-chloro-2-((6-nitrobenzo[d] thiazol-2-yl)amino)pyrimidin-4-yl)amino)phenyl)dimethylphosphine oxide (Compound 28);
(2-((5-chloro-2-((6-chlorobenzo[d] thiazol-2-yl)amino)pyrimidin-4-yl)amino)phenyl)dimethylphosphine oxide (Compound 29);
(((5-chloropyrimidin-2,4-diyl)bis(azanediyl))bis(2,1-phenylene))bis(dimethylphosphine oxide) (Compound 30);
(2-((5-chloro-2-((6-(methylsulfonyl)benzo[d] thiazol-2-yl)amino)pyrimidin-4-yl)amino)phenyl)dimethylphosphine oxide (Compound 31);
(2-((5-chloro-2-((6-(trifluoromethyl)benzo[d] thiazol-2-yl)amino)pyrimidin-4-yl)amino)phenyl)dimethylphosphine oxide (Compound 32);
(2-((2-(benzo[d] thiazol-2-ylamino)-5-chloropyrimidin-4-yl)amino)phenyl)dimethylphosphine oxide (Compound 33);
(2-((5-chloro-2-((6-fluorobenzo[d] thiazol-2-yl)amino)pyrimidin-4-yl)amino)phenyl)dimethylphosphine oxide (Compound 34);
(2-((5-chloro-2-((6-methylbenzo[d] thiazol-2-yl)amino)pyrimidin-4-yl)amino)phenyl)dimethylphosphine oxide (Compound 35);
(2-((5-chloro-2-((6-methoxybenzo[d] thiazol-2-yl)amino)pyrimidin-4-yl)amino)phenyl)dimethylphosphine oxide (Compound 36);
5-fluoro-N², N²-dimethyl-N⁴-(1-(methylsulfonyl)piperidin-4-yl)pyrimidine-2,4-diamine (Compound 37);
5-fluoro-N²-(8-fluoroquinolin-4-yl)-N⁴-(1-(methylsulfonyl)piperidin-4-yl)pyrimidine-2,4-diamine (Compound 38);
(2-((5-chloro-2-((3,4-dimethylisoxazol-5-yl)amino)pyrimidin-4-yl)amino)phenyl)dimethylphosphine oxide (Compound 39);
(2-((5-chloro-2-((3,4-dimethylisoxazol-5-yl)amino)pyrimidin-4-yl)amino)phenyl)dimethylphosphine oxide hydrochloride (Compound 40);
7-((5-chloro-4-((2-(dimethylphosphoryl)phenyl)amino)pyrimidin-2-yl)amino)-3,4-dihydroisoquinolin-1(2H)-one (Compound 41);
1-(7-((5-chloro-4-((2-(dimethylphosphoryl)phenyl)amino)pyrimidin-2-yl)amino)-1,1-dimethyl-3,4-dihydroisoquinolin-2(1H)-yl)-2,2,2-trifluoroethan-1-one (Compound 42);
1-(7-((5-chloro-4-((2-(dimethylphosphoryl)phenyl)amino)pyrimidin-2-yl)(ethyl)amino)-1,1-dimethyl-3,4-dihydroisoquinolin-2(1H)-yl)-2,2,2-trifluoroethan-1-one (Compound 43);
(2-((5-chloro-2-((1,1-dimethyl-1,2,3,4-tetrahydroisoquinolin-7-yl)amino)pyrimidin-4-yl)amino)phenyl)dimethylphosphine oxide (Compound 44);
(2-((5-chloro-2-((2-(5-chloro-4-((2-(dimethylphosphoryl)phenyl)amino)pyrimidin-2-yl)-1,2,3,4-tetrahydroisoquinolin-5-yl)amino)pyrimidin-4-yl)amino)phenyl)dimethylphosphine oxide (Compound 45);
1-(7-((5-chloro-4-((2-(dimethylphosphoryl)phenyl)amino)pyrimidin-2-yl)amino)-3,4-dihydroisoquinolin-2(1H)-yl)-2,2,2-trifluoroethan-1-one (Compound 46);
(2-((5-chloro-2-((1,2,3,4-tetrahydroisoquinolin-7-yl)amino)pyrimidin-4-yl)amino)phenyl)dimethylphosphine oxide (Compound 47);
1-(6-((5-chloro-4-((2-(dimethylphosphoryl)phenyl)amino)pyrimidin-2-yl)amino)-3,4-dihydroisoquinolin-2(1H)-yl)-2,2,2-trifluoroethan-1-one (Compound 48);
(2-((5-chloro-2-((1,2,3,4-tetrahydroisoquinolin-6-yl)amino)pyrimidin-4-yl)amino)phenyl)dimethylphosphine oxide (Compound 49);
1-(7-((5-chloro-4-((2-(dimethylphosphoryl)phenyl)amino)pyrimidin-2-yl)amino)-4,4-dimethyl-3,4-dihydroisoquinolin-2(1H)-yl)-2,2,2-trifluoroethan-1-one (Compound 50);
(2-((5-chloro-2-((1-methyl-1,2,3,4-tetrahydroisoquinolin-7-yl)amino)pyrimidin-4-yl)amino)phenyl)dimethylphosphine oxide (Compound 51);
(2-((5-chloro-2-((4,4-dimethyl-1,2,3,4-tetrahydroisoquinolin-7-yl)amino)pyrimidin-4-yl)amino)phenyl)dimethylphosphine oxide (Compound 52);
1-(7-((5-chloro-4-((2-(dimethylphosphoryl)phenyl)amino)pyrimidin-2-yl)amino)-1-isopropyl-3,4-dihydroisoquinolin-2(1H)-yl)-2,2,2-trifluoroethan-1-one (Compound 53);
1-(7-((5-chloro-4-((2-(dimethylphosphoryl)phenyl)amino)pyrimidin-2-yl)amino)-1-methyl-3,4-dihydroisoquinolin-2(1H)-yl)-2,2,2-trifluoroethan-1-one (Compound 54);
(2-((5-chloro-2-((1-isopropyl-1,2,3,4-tetrahydroisoquinolin-7-yl)amino)pyrimidin-4-yl)amino)phenyl)dimethylphosphine oxide (Compound 55);
2,2,2-trifluoro-1-(7-((5-fluoro-4-((1-(methylsulfonyl)piperidin-4-yl)amino)pyrimidin-2-yl)amino)-3,4-dihydroisoquinolin-2(1H)-yl)ethan-1-one (Compound 56);
5-fluoro-N⁴-(1-(methylsulfonyl)piperidin-4-yl)-N²-(1,2,3,4-tetrahydroisoquinolin-7-yl)pyrimidine-2,4-diamine (Compound 57);
2,2,2-trifluoro-1-(7-((5-fluoro-4-((1-(methylsulfonyl)piperidin-4-yl)amino)pyrimidin-2-yl)amino)-1-isopropyl-3,4-dihydroisoquinolin-2(1H)-yl)ethan-1-one (Compound 58);
2,2,2-trifluoro-1-(7-((5-fluoro-4-((1-(methylsulfonyl)piperidin-4-yl)amino)pyrimidin-2-yl)amino)-1-methyl-3,4-dihydroisoquinolin-2(1H)-yl)ethan-1-one (Compound 59);
5-fluoro-N²-(1-isopropyl-1,2,3,4-tetrahydroisoquinolin-7-yl)-N⁴-(1-(methylsulfonyl)piperidin-4-yl)pyrimidine-2,4-diamine (Compound 60);
5-fluoro-N²-(1-methyl-1,2,3,4-tetrahydroisoquinolin-7-yl)-N⁴-(1-(methylsulfonyl)piperidin-4-yl)pyrimidine-2,4-diamine (Compound 61);
1-(7-((5-chloro-4-((2-(dimethylphosphoryl)phenyl)amino)pyrimidin-2-yl)amino)-6-methoxy-3,4-dihydroisoquinolin-2(1H)-yl)-2,2,2-trifluoroethan-1-one (Compound 62);
(2-((5-chloro-2-((6-methoxy-1,2,3,4-tetrahydroisoquinolin-7-yl)amino)pyrimidin-4-yl)amino)phenyl)dimethylphosphine oxide (Compound 63);
(2-((2-((1H-indazol-6-yl)amino)-5-chloropyrimidin-4-yl)amino)phenyl)dimethylphosphine oxide (Compound 64);
(2-((2-((1H-indol-5-yl)amino)-5-chloropyrimidin-4-yl)amino)phenyl)dimethylphosphine oxide (Compound 65);
(2-((2-((1H-indazol-5-yl)amino)-5-chloropyrimidin-4-yl)amino)phenyl)dimethylphosphine oxide (Compound 66);
(2-((2-((3-bromo-1H-indazol-5-yl)amino)-5-chloropyrimidin-4-yl)amino)phenyl)dimethylphosphine oxide (Compound 67);
(2-((2-((1H-indol-4-yl)amino)-5-chloropyrimidin-4-yl)amino)phenyl)dimethylphosphine oxide (Compound 68);
(2-((5-chloro-2-((2-methyl-1H-indol-5-yl)amino)pyrimidin-4-yl)amino)phenyl)dimethylphosphine oxide (Compound 69);
7-((5-chloro-4-((2-(dimethylphosphoryl)phenyl)amino)pyrimidin-2-yl)amino)-4-methyl-1H-indole-3-carbonitrile (Compound 70);
(2-((2-((1H-indol-7-yl)amino)-5-chloropyrimidin-4-yl)amino)phenyl)dimethylphosphine oxide (Compound 71);
1-(5-((5-chloro-4-((2-(dimethylphosphoryl)phenyl)amino)pyrimidin-2-yl)amino)isoindolin-2-yl)-2,2,2-trifluoroethan-1-one (Compound 72);
(2-((5-chloro-2-(isoindolin-5-ylamino)pyrimidin-4-yl)amino)phenyl)dimethylphosphine oxide (Compound 73);
(2-((5-chloro-2-(pyrrolo[2,1-f] [1,2,4] triazin-4-ylamino)pyrimidin-4-yl)amino)phenyl)dimethylphosphine oxide (Compound 74);
(2-((5-chloro-2-((2-(trifluoromethyl)-1H-benzo[d] imidazol-5-yl)amino)pyrimidin-4-yl)amino)phenyl)dimethylphosphine oxide (Compound 75);
(2-((5-chloro-2-((2-mercapto-1H-benzo[d] imidazol-5-yl)amino)pyrimidin-4-yl)amino)phenyl)dimethylphosphine oxide (Compound 76);
(2-((5-chloro-2-(imidazo[1,2-a] pyridin-8-ylamino)pyrimidin-4-yl)amino)phenyl)dimethylphosphine oxide (Compound 77);
(2-((5-chloro-2-((1-methyl-1H-pyrazolo[3,4-b] pyridin-5-yl)amino)pyrimidin-4-yl)amino)phenyl)dimethylphosphine oxide (Compound 78);
(2-((2-((1H-pyrrolo[2,3-b] pyridin-5-yl)amino)-5-chloropyrimidin-4-yl)amino)phenyl)dimethylphosphine oxide (Compound 79);
(2-((5-chloro-2-((1-isopropyl-1H-pyrazolo[4,3-c] pyridin-6-yl)amino)pyrimidin-4-yl)amino)phenyl)dimethylphosphine oxide (Compound 80);
(2-((2-((3-bromo-1H-pyrazolo[3,4-b] pyridin-5-yl)amino)-5-chloropyrimidin-4-yl)amino)phenyl)dimethylphosphine oxide (Compound 81);
(2-((2-((1H-pyrazolo[3,4-b] pyridin-5-yl)amino)-5-chloropyrimidin-4-yl)amino)phenyl)dimethylphosphine oxide (Compound 82);
(2-((5-chloro-2-((3-(2-fluoropyridin-3-yl)-1H-indazol-5-yl)amino)pyrimidin-4-yl)amino)phenyl)dimethylphosphine oxide (Compound 83);
(2-((2-((3-(1H-pyrazol-4-yl)-1H-indazol-5-yl)amino)-5-chloropyrimidin-4-yl)amino)phenyl)dimethylphosphine oxide (Compound 84);
tert-butyl 4-(5-((5-chloro-4-((2-(dimethylphosphoryl)phenyl)amino)pyrimidin-2-yl)amino)-1H-indazol-3-yl)-3,6-dihydropyridine-1(2H)-carboxylate (Compound 85);
5-fluoro-N²-(1H-indazol-5-yl)-N⁴-(1-(methylsulfonyl)piperidin-4-yl)pyrimidine-2,4-diamine (Compound 86);
5-fluoro-N²-(1H-indazol-6-yl)-N⁴-(1-(methylsulfonyl)piperidin-4-yl)pyrimidine-2,4-diamine (Compound 87);
N²-(3-bromo-1H-indazol-5-yl)-5-fluoro-N (methylsulfonyl)piperidin-4-yl)pyrimidine-2,4-diamine (Compound 88);
5-fluoro-N²-(1H-indol-5-yl)-N (methylsulfonyl)piperidin-4-yl)pyrimidine-2,4-diamine (Compound 89);
5-fluoro-N²-(2-methyl-1H-indol-5-yl)-N⁴-(1-(methylsulfonyl)piperidin-4-yl)pyrimidine-2,4-diamine (Compound 90);
2-((5-chloro-2-((3-(1,2,3,6-tetrahydropyridin-4-yl)-1H-indazol-5-yl)amino)pyrimidin-4-yl)amino)phenyl)dimethylphosphine oxide (Compound 91);
5-(5-((5-chloro-4-((2-(dimethylphosphoryl)phenyl)amino)pyrimidin-2-yl)amino)-1H-indazol-3-yl)furan-2-carbaldehyde (Compound 92);
(2-((5-chloro-2-((3-(1-isopropyl-1H-pyrazol-4-yl)-1H-indazol-5-yl)amino)pyrimidin-4-yl)amino)phenyl)dimethylphosphine oxide (Compound 93);
(2-((5-chloro-2-((3-(1-methyl-1H-pyrazol-4-yl)-1H-indazol-5-yl)amino)pyrimidin-4-yl)amino)phenyl)dimethylphosphine oxide (Compound 94);
(2-((5-chloro-2-((3-(1-propyl-1H-pyrazol-4-yl)-1H-indazol-5-yl)amino)pyrimidin-4-yl)amino)phenyl)dimethylphosphine oxide (Compound 95);
(2-((5-chloro-2-((3-phenyl-1H-indazol-5-yl)amino)pyrimidin-4-yl)amino)phenyl)dimethylphosphine oxide (Compound 96);
(2-((5-chloro-2-((3-(3-nitrophenyl)-1H-indazol-5-yl)amino)pyrimidin-4-yl)amino)phenyl)dimethylphosphine oxide (Compound 97);
(2-((5-chloro-2-((3-(2-fluoropyridin-3-yl)-1H-pyrazolo[3,4-b] pyridin-5-yl)amino)pyrimidin-4-yl)amino)phenyl)dimethylphosphine oxide (Compound 98);
1-(4-(5-((5-chloro-4-((2-(dimethylphosphoryl)phenyl)amino)pyrimidin-2-yl)amino)-1H-indazol-3-yl)-3,6-dihydropyrimidin-1(2H)-yl)ethan-1-one (Compound 99);
(2-((2-((3-(3-aminophenyl)-1H-indazol-5-yl)amino)-5-chloropyrimidin-4-yl)amino)phenyl)dimethylphosphine oxide (Compound 100);
(2-((5-chloro-2-((3-(5-(hydroxymethyl)furan-2-yl)-1H-indazol-5-yl)amino)pyrimidin-4-yl)amino)phenyl)dimethylphosphine oxide (Compound 101);
tert-butyl4-(5-((4-((2-(dimethylphosphoryl)phenyl)amino)pyrimidin-2-yl)amino)-1H-indazol-3-yl)piperidine-1-carboxylate (Compound 102);
dimethyl(2-((2-((3-(piperidin-4-yl)-1H-indazol-5-yl)amino)pyrimidin-4-yl)amino)phenyl)phosphine oxide hydrochloride (Compound 103);
(2-((5-chloro-2-((3-(pyridin-4-yl)-1H-indazol-5-yl)amino)pyrimidin-4-yl)amino)phenyl)dimethylphosphine oxide (Compound 104);
4-(5-((5-chloro-4-((2-(dimethylphosphoryl)phenyl)amino)pyrimidin-2-yl)amino)-1H-indazol-3-yl)benzonitrile (Compound 105);
1-(4-(5-((5-chloro-4-((2-(dimethylphosphoryl)phenyl)amino)pyrimidin-2-yl)amino)-1H-indazol-3-yl)phenyl)ethan-1-one (Compound 106);
(2-((5-chloro-2-((3-(3-chloro-4-fluorophenyl)-1H-indazol-5-yl)amino)pyrimidin-4-yl)amino)phenyl)dimethylphosphine oxide (Compound 107);
(2-((5-chloro-2-((3-(4-hydroxyphenyl)-1H-indazol-5-yl)amino)pyrimidin-4-yl)amino)phenyl)dimethylphosphine oxide (Compound 108);
(2-((5-chloro-2-((3-(3-fluoro-4-methoxyphenyl)-1H-indazol-5-yl)amino)pyridin-4-yl)amino)phenyl)dimethylphosphine oxide (Compound 109);
(2-((5-chloro-2-((3-(4-(trifluoromethoxy)phenyl)-1H-indazol-5-yl)amino)pyrimidin-4-yl)amino)phenyl)dimethylphosphine oxide (Compound 110);
(2-((5-chloro-2-((3-(4-chloro-3-(trifluoromethyl)phenyl)-1H-indazol-5-yl)amino)pyrimidin-4 yl)amino)phenyl)dimethylphosphine oxide (Compound 111);
(2-((5-chloro-2-((3-(3-chloro-4-methylphenyl)-1H-indazol-5-yl)amino)pyrimidin-4-yl)amino)phenyl)dimethylphosphine oxide (Compound 112);
(2-((5-chloro-2-((3-(3-chloro-5-methoxyphenyl)-1H-indazol-5-yl)amino)pyrimidin-4-yl)amino)phenyl)dimethylphosphine oxide (Compound 113);
(2-((5-chloro-2-((3-(4-(methylsulfonyl)phenyl)-1H-indazol-5-yl)amino)pyrimidin-4-yl)amino)phenyl)dimethylphosphine oxide (Compound 114);
(2-((5-chloro-2-((3-(3-fluoro-5-isopropoxyphenyl)-1H-indazol-5-yl)amino)pyrimidin-4-yl)amino)phenyl)dimethylphosphine oxide (Compound 115);
3-(5-((5-chloro-4-((2-(dimethylphosphoryl)phenyl)amino)pyrimidin-2-yl)amino)-1H-indazol-3-yl)benzonitrile (Compound 116);
tert-butyl 4-(4-(5-((5-chloro-4-((2-(dimethylphosphoryl)phenyl)amino)pyrimidin-2-yl)amino)-1H-indazol-3-yl)phenyl)piperazine-1-carboxylate (Compound 117);
(2-((5-chloro-2-((3-(3-hydroxyphenyl)-1H-indazol-5-yl)amino)pyrimidin-4-yl)amino)phenyl)dimethylphosphine oxide (Compound 118); and
(2-((5-chloro-2-((3-(4-(piperazine-1-yl)phenyl)-1H-indazol-5-yl)amino)pyrimidin-4-yl)amino)phenyl)dimethylphosphine oxide (Compound 119).

5. A pharmaceutical composition comprising the compound of any one of claims 1 to 3, the optical isomer thereof, or the pharmaceutically acceptable salt thereof as an active ingredient for prevention or treatment of cancer, a virus infectious disease, Parkinson's disease, non-alcoholic steatohepatitis, and tuberculosis.

6. The pharmaceutical composition of claim 5, wherein the cancer is selected from the group consisting of lung cancer, liver cancer, stomach cancer, colorectal cancer, bladder cancer, prostate cancer, breast cancer, ovarian cancer, cervical cancer, thyroid cancer, melanoma, blood cancer, colon cancer, non-small cell lung cancer, pancreatic cancer, skin cell, head and neck cancer, small bowel cancer, rectal cancer, endometrial cancer, vaginal cancer, testis cancer, esophageal cancer, bile duct cancer, lymph gland cancer, gall bladder cancer, endocrine gland cancer, adrenal cancer, lymphoma, multiple myeloma, thymoma, mesothelioma, kidney cancer, brain cancer, tumors of central nervous system, brainstem glioma, and pituitary adenoma.

7. The pharmaceutical composition of claim 5, wherein the virus infectious disease is caused by at least one virus selected from the group consisting of ZIKA virus, acquired human immunodeficiency virus (HIV), influenza virus, influenza A virus subtype H1N1, avian influenza virus, rhinovirus, adenovirus, coronavirus, parainfluenza virus, respiratory syncytial virus, herpesvirus (HSV), rotavirus, and hepatitis virus.
